(19) Europäisches Patentamt
European Patent Office
Office européen des brevets

(11) Numéro de publication: **0 294 669 B1**

(12) # FASCICULE DE BREVET EUROPEEN

(45) Date de publication de fascicule du brevet: **17.07.91**

(51) Int. Cl.[5]: **C07C 217/84**, A61K 7/13

(21) Numéro de dépôt: **88108494.1**

(22) Date de dépôt: **27.05.88**

(54) **Nouvelles métaphénylènediamines,leur procédé de préparation, composés intermédiaires et utilisation de ces métaphenylènediamines en tant que coupleurs pour la teinture d'oxydation des fibres kératiniques et en particulier des cheveux humains.**

(30) Priorité: **29.05.87 LU 86905**

(43) Date de publication de la demande:
**14.12.88 Bulletin 88/50**

(45) Mention de la délivrance du brevet:
**17.07.91 Bulletin 91/29**

(84) Etats contractants désignés:
**AT BE CH DE ES FR GB GR IT LI NL SE**

(56) Documents cités:
**EP-A- 0 075 242**

**CHEMICAL ABSTRACTS, vol. 27, no. 4, 20 février 1933, page 970, Columbus, Ohio, US; W. BAKER et al.:"Derivatives of 1,2,3,4-tetrahydroxybenzene"**

(73) Titulaire: **L'OREAL**
**14, Rue Royale**
**F-75008 Paris(FR)**

(72) Inventeur: **Junino, Alex**
**16 Rue du Docteur Bergonié**
**F-93180 Livry-Gargan(FR)**
Inventeur: **Vandenbossche, Jean-Jacques**
**6 Rue Léon Richet**
**F-93600 Aulnay-sous-Bois(FR)**
Inventeur: **Borowiak, Hervé**
**84, 8ème Avenue**
**F-93290 Tremblay-Les-Gonesse(FR)**
Inventeur: **Lang, Gérard**
**44 Avenue Lacour**
**F-95210 Saint-Gratien(FR)**

(74) Mandataire: **Casalonga, Axel et al**
**BUREAU D.A. CASALONGA - JOSSE Morassistrasse 8**
**W-8000 München 5(DE)**

**EP 0 294 669 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention a pour objet de nouvelles métaphénylènediamines, leur procédé de préparation, des composés intermédiaires, des compositions tinctoriales pour fibres kératiniques et en particulier pour cheveux humains, contenant ces métaphénylènediamines à titre de coupleurs associés à des précurseurs de colorants d'oxydation, ainsi qu'un procédé de teinture utilisant lesdites compositions.

On sait qu'il est courant d'utiliser pour la teinture des fibres kératiniques telles que les cheveux humains ou les fourrures, des compositions tinctoriales contenant des précurseurs de colorants d'oxydation et en particulier des paraphénylène diamines, des ortho-ou para-aminophénols que l'on désigne généralement sous le terme de bases d'oxydation.

On sait également que pour faire varier les nuances obtenues avec ces bases d'oxydation, on utilise des modificateurs de coloration ou coupleurs, et en particulier des métaphénylènediamines, des métaaminophénols et des métadiphénols.

Dans les milieux alcalins oxydants utilisés habituellement en teinture d'oxydation, les paraphénylènediamines et para-aminophénols donnent naissance en présence de coupleurs tels que les métaphénylènediamines à des indamines ou à des indoanilines colorées.

Les indamines formées à partir de métaphénylènediamines et de para-phénylènediamines en milieu alcalin oxydant, et plus particulièrement en présence d'eau oxygénée, confèrent aux fibres kératiniques des colorations bleues très puissantes. Les indoanilines formées à partir de métaphénylènediamines et de para-aminophénols en milieu alcalin oxydant confèrent aux fibres kératiniques des colorations rouges plus ou moins pourpres. Selon les bases d'oxydation auxquelles elles sont associées, les métaphénylènediamines peuvent donc donner des colorations rouges ou bleues qui sont deux couleurs fondamentales en teinture capillaire, indispensables pour obtenir non seulement des noirs et des gris mais aussi des châtains cuivrés ou cendrés. On voit ainsi le rôle extrêmement important joué par les métaphénylènediamines en teinture capillaire d'oxydation.

Il est important par ailleurs que les bases d'oxydation et les coupleurs, qui sont utilisés dans les compositions de teinture par oxydation, confèrent aux cheveux des colorations stables à la lumière, au lavage, aux intempéries, à la transpiration et qui soient peu ou non sélectives. Il est nécessaire également que ces composés bénéficient d'une bonne innocuité.

On connaît déjà de nombreux coupleurs du type des métaphénylènediamines substituées sur le noyau aromatique. Cependant, un grand nombre d'entre eux ne répondent pas aux exigences souhaitées.

La demanderesse vient de découvrir de nouvelles métaphénylènediamines qui allient une très bonne innocuité avec les qualités tinctoriales d'un bon coupleur et qui peuvent donc être utilisées avantageusement comme coupleurs en association avec des précurseurs de colorants d'oxydation, notamment de type para, dans des compositions de teinture d'oxydation pour fibres kératiniques.

Associées à la plupart des paraphénylènediamines en milieu alcalin oxydant, les métaphénylènediamines selon l'invention confèrent aux cheveux des colorations bleues puissantes plus ou moins riches en vert ou des pourpres plus ou moins rouges.

Lorsqu'elles sont associées aux para-aminophénols en milieu alcalin oxydant, les métaphénylènediamines selon l'invention confèrent aux cheveux des colorations rouges de bonne stabilité.

La présente invention a donc pour objet les métaphénylènediamines répondant à la formule (I) ci-dessous ou leurs sels d'addition avec un acide :

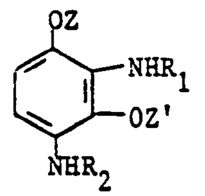

(I)

formule dans laquelle :

$R_1$ et $R_2$ représentent indépendamment l'un de l'autre un atome d'hydrogène, un radical alkyle ayant 1 à 4 atomes de carbone ou un radical mono- ou polyhydroxyalkyle ayant 2 ou 3 atomes de carbone;

Z et Z′ représentent indépendamment l'un de l'autre un radical alkyle ayant de 1 à 4 atomes de carbone ou hydroxyalkyle ayant de 2 à 4 atomes de carbone, sous réserve que lorsque $R_1$ et $R_2$ désignent simultanément un atome d'hydrogène, Z et Z′ ne désignent pas simultanément le radical méthyle.

La présente invention a également pour objet un procédé de préparation des composés de formule (I)

ainsi que les composés intermédiaires de formule (XI) suivante :

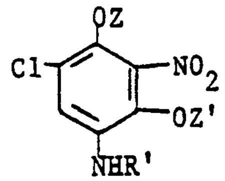

(XI)

dans laquelle Z et Z′ désignent un radical alkyle ayant 1 à 4 atomes de carbone ou hydroxyalkyle ayant 2 à 4 atomes de carbone et R′ désigne un atome d'hydrogène, un radical alkyle ayant de 1 à 4 atomes de carbone, un radical mono- ou polyhydroxyalkyle ayant 2 ou 3 atomes de carbone, acétyle ou β-chloréthoxycarbonyle.

La présente invention vise aussi l'utilisation à titre de coupleurs des composés de formule (I) ou de leurs sels d'addition avec un acide en association avec des précurseurs de colorants d'oxydation pour la teinture des fibres kératiniques et en particulier des cheveux humains.

Un autre objet de la présente invention est donc une composition de teinture capillaire comprenant, à titre de coupleur, au moins un composé de formule (I) ou l'un de ses sels d'acides, en association avec au moins un précurseur de colorant d'oxydation de type para, dans un support aqueux cosmétiquement acceptable.

La présente invention vise aussi le procédé de teinture des cheveux utilisant la composition tinctoriale ci-dessus.

Les composés de formule (I) sont préparés à partir des 3,5-dinitrochlorobenzènes 2,4-disubstitués de formule (II) ci-après :

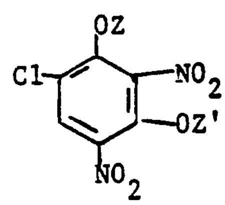

(II)

dans laquelle Z et Z′ ont les significations indiquées ci-dessus.

Selon la signification des radicaux $R_1$ et $R_2$ dans la formule (I) des composés finals, ces composés sont préparés selon les schémas réactionnels suivants :

1°) Procédé de préparation des composés (I) dans lesquels $R_1 = R_2 = H$ (composés IA)

Ce procédé consiste à réduire et déshalogéner, consécutivement ou simultanément, les 3,5-dinitrochlorobenzènes 2,4-disubstitués de formule (II)

a) Réduction du composé (II) suivie d'une déshalogénation

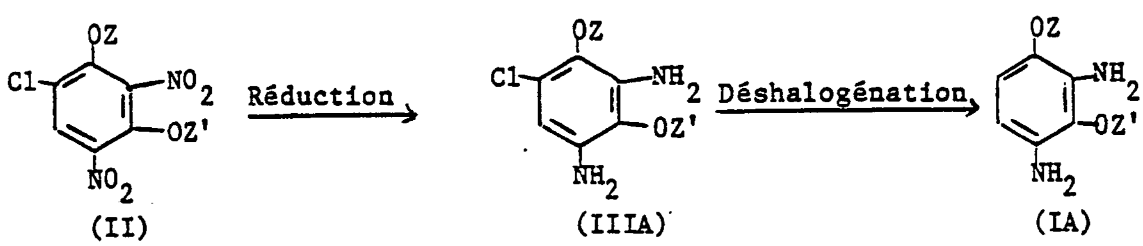


(II) (IIIA) (IA)

Z et Z′ désignent un radical alkyle en $C_1$-$C_4$ ou hydroxyalkyle en $C_2$-$C_4$ mais non simultanément un radical méthyle.

Le 3,5-dinitrochlorobenzène 2,4-disubstitué de formule (II) est réduit par le fer en présence d'acide acétique, à une température comprise entre 50 et 100 °C. On obtient ainsi un 3,5-diaminochlorobenzène 2,4-disubstitué de formule ($III_A$) qui est alors soumis à une réaction de déshalogénation. Cette réaction s'effectue en présence de palladium sur charbon, d'acétate d'ammonium et de formiate de triéthylamine dans un milieu solvant constitué par de l'eau, un alcool inférieur ou un mélange hydroalcoolique, à une température comprise entre 50 et 100 °C, selon Org. Chem., vol. 42, n° 22, 1977, p. 3491.

b) Réduction et déshalogénation simultanées du composé (II)

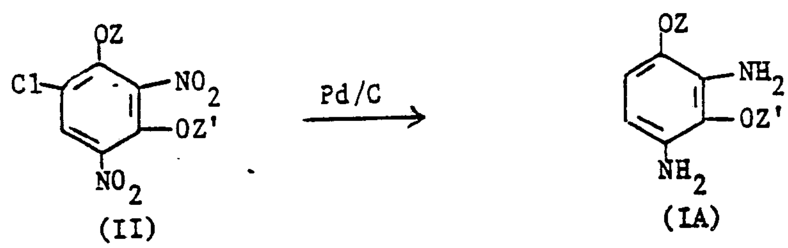


(II) (IA)

Z et Z' désignent un radical alkyle en $C_1$-$C_4$ ou hydroxyalkyle en $C_2$-$C_4$ mais ne désignent pas simultanément un radical méthyle.

Cette réaction, qui permet d'obtenir directement le 2,4-diaminobenzène 1,3-disubstitué de formule ($I_A$), s'effectue sous pression d'hydrogène en présence de palladium sur charbon comme indiqué dans l'article "Catalytic hydrogenation", Organic Synthesis, RYLANDER, Academic Press Inc. L'addition d'acétate d'ammonium ou de triéthylamine favorise la réaction. Celle-ci s'effectue dans un solvant constitué par de l'eau, un alcool inférieur ou un mélange hydroalcoolique, à une température comprise entre 50 °C et 200 °C.

2°) Procédé de préparation des composés (I) dans lesquels $R_1 = R_2 \neq H$

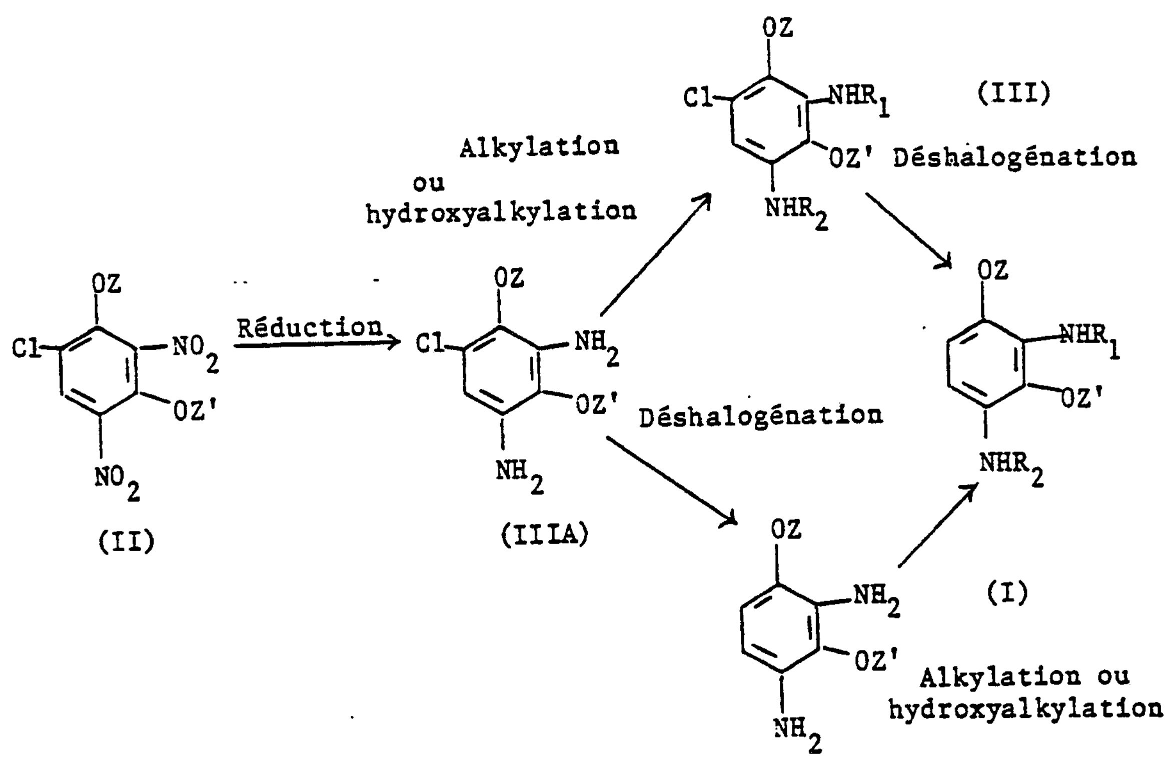


(III) (II) (IIIA) (I) ($I_A$)

Dans les formules ci-dessus, $R_1$, $R_2$, Z et Z' ont les significations indiquées ci-dessus, et Z et Z' peuvent désigner simultanément un radical méthyle.

Le 3,5-dinitrochlorobenzène 2,4-disubstitué de formule (II) est réduit par le fer en présence d'acide acétique à une température comprise entre 50 et 100 °C. On obtient le 3,5-diaminochlorobenzène 2,4-disubstitué de formule ($III_A$).

Selon une première voie réactionnelle, le composé ($III_A$) est déshalogéné comme indiqué ci-dessus en a) pour la préparation du composé ($I_A$). Les fonctions amines du 2,4-diamino-benzène 1,3-disubstitué de formule ($I_A$) sont ensuite alkylées ou hydroxyalkylées selon les procédés classiques d'alkylation ou d'hydroxyalkylation des amines aromatiques.

Le composé ($I_A$) peut aussi être préparé par réduction et déshalogénation simultanées selon le procédé 1°) b) décrit ci-dessus.

Selon une variante du procédé, on peut d'abord procéder à l'alkylation ou l'hydroxyalkylation des fonctions amines du 3,5-diaminochlorobenzène 2,4-disubstitué de formule ($III_A$) pour obtenir le composé de formule (III) qui subit ensuite une déshalogénation selon le même procédé que celui indiqué ci-dessus pour conduire au 2,4-diamino-benzène 1,3-disubstitué final de formule (I).

3°) Procédé de préparation des composés (I) dans lesquels $R_1$ = H et $R_2 \neq$ H (composés IB)

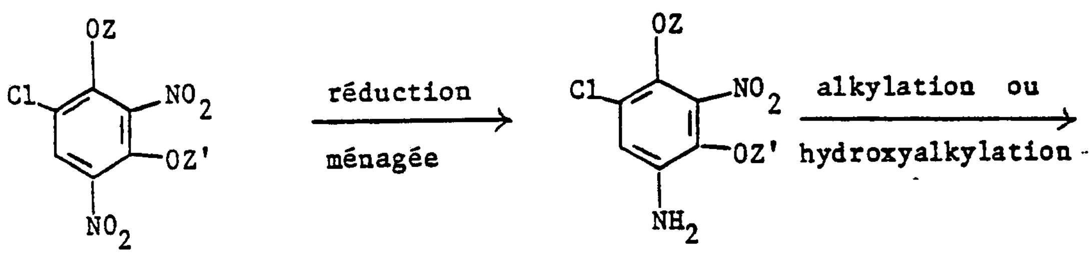


(II) (IV)

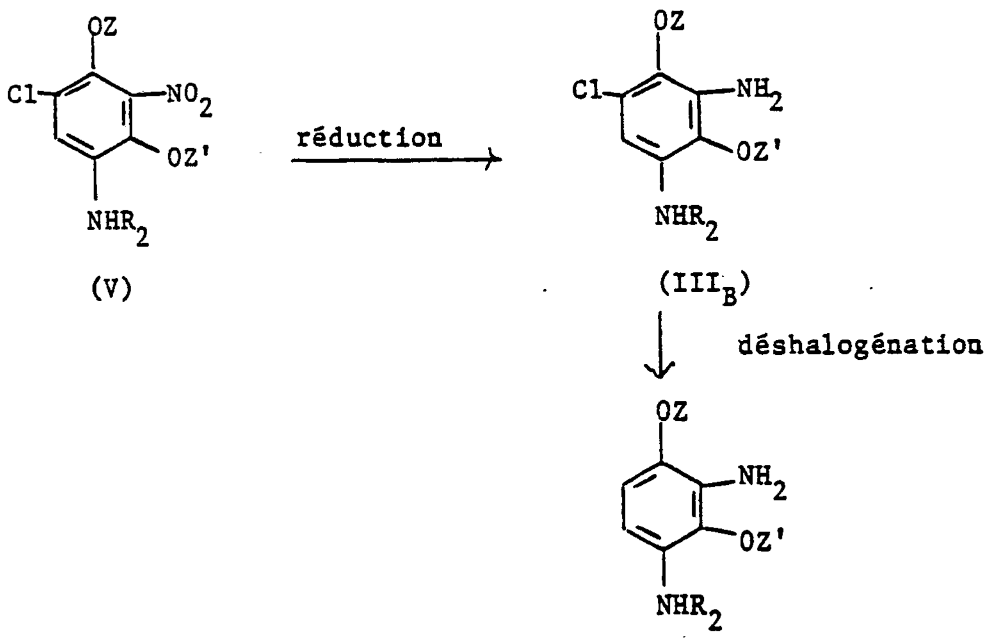


(V) ($III_B$)

($I_B$)

Dans les formules ci-dessus, Z, Z' et $R_2$ ont les significations indiquées ci-dessus, Z et Z' pouvant désigner simultanément le radical méthyle.

Le 3,5-dinitrochlorobenzène 2,4-disubstitué de formule (II) est soumis dans une première étape à une réduction ménagée qui s'effectue par transfert d'hydrogène en présence d'un catalyseur tel que le palladium sur charbon et de cyclohexène utilisé comme donneur d'hydrogène.

Dans une seconde étape, le 5-amino-3-nitrochlorobenzène 2,4-disubstitué de formule (IV) est soumis à une alkylation ou une hydroxyalkylation du groupement amino pour conduire au composé (V) qui est réduit dans une troisième étape par le fer en présence d'acide acétique à une température comprise entre 20 et 100° C. On obtient ainsi le composé de formule ($III_B$) qui est soumis dans une quatrième étape à une réaction de déshalogénation comme indiqué ci-dessus pour la préparation des composés (IA).

4°) Procédé de préparation des composés (I) dans lesquels $R_1 \neq H$ et $R_2 = H$ (composés IC)

a) Premier procédé

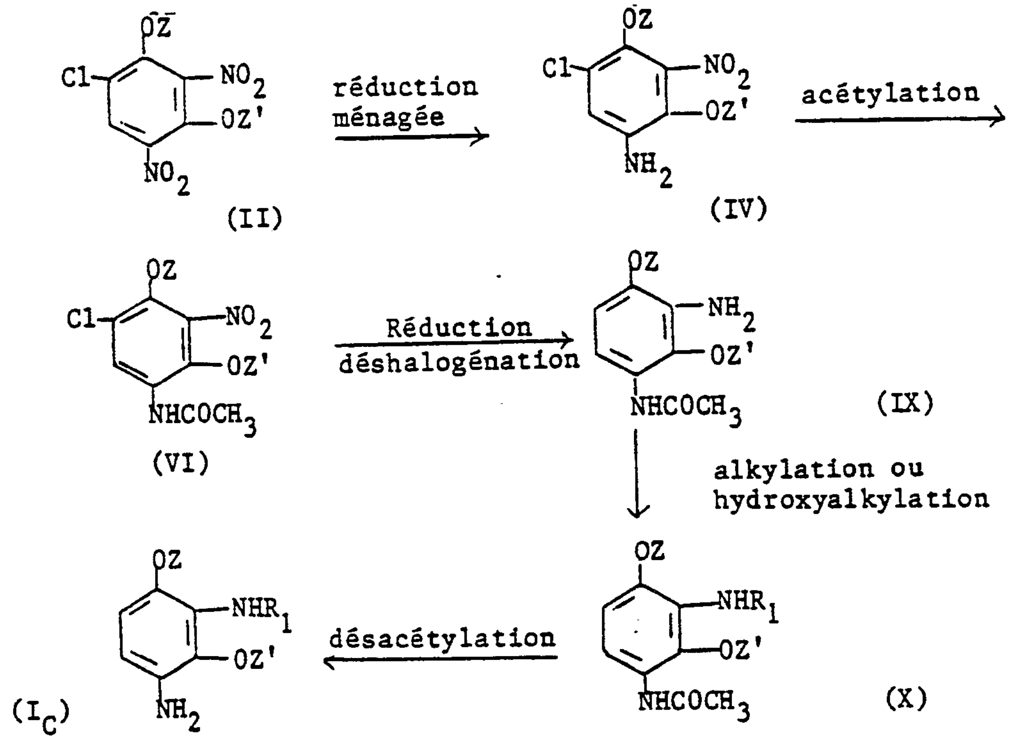


Dans les formules ci-dessus, Z, Z' et $R_1$ ont les significations indiquées ci-dessus, Z et Z' pouvant désigner simultanément le radical méthyle.

Le 5-amino-3-nitrochlorobenzène 2,4-disubstitué de formule (IV) obtenu par réduction ménagée du composé de formule (II) comme indiqué dans le procédé de préparation des composés (IB), est acétylé par l'anhydride acétique à une température comprise entre 20° et 100° C pour conduire au composé de formule (VI).

Le composé (VI) est ensuite réduit et déshalogéné simultanément comme indiqué dans le procédé de préparation b) des composés (IA) sous pression d'hydrogène en utilisant le palladium sur charbon, de préférence en présence d'acétate d'ammonium ou de triéthylamine.

Le composé (IX) ainsi obtenu est soumis à une alkylation ou hydroxyalkylation pour conduire au composé (X) qui, après désacétylation, donne le composé ($I_C$).

b) Deuxième Procédé

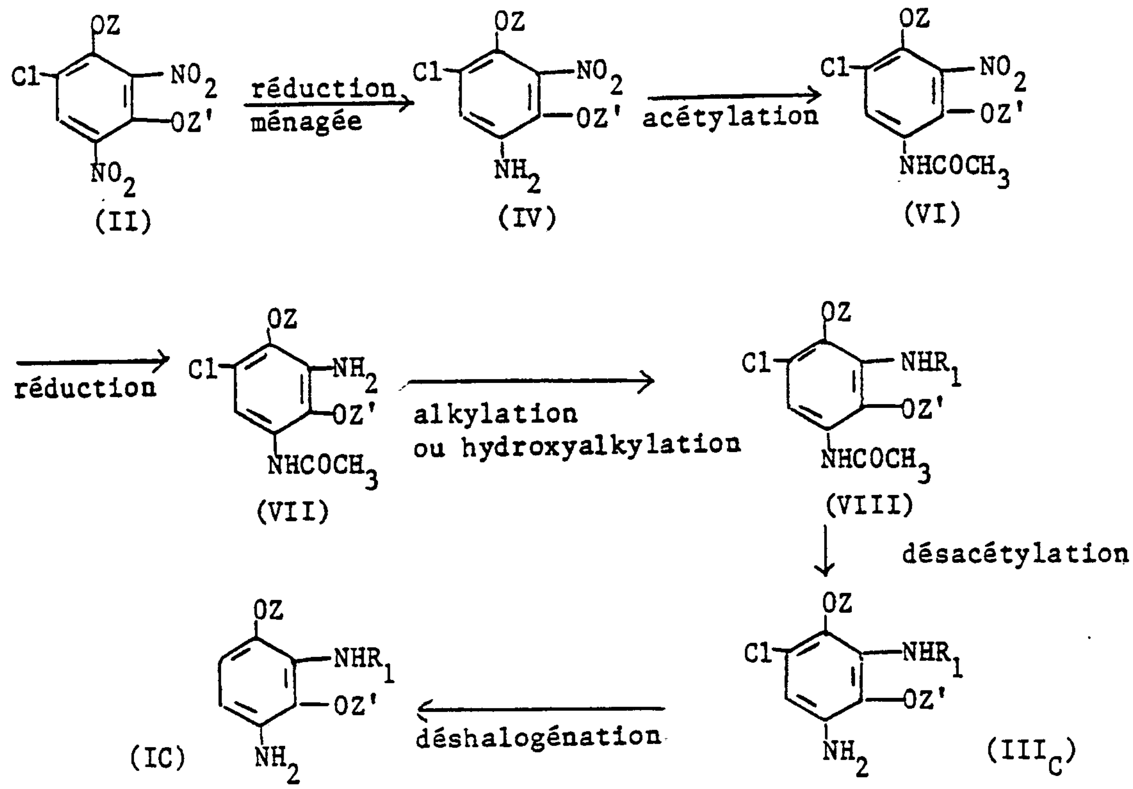


(II) (IV) (VI) (VII) (VIII) (IC) ($III_C$)

Z, Z' et $R_1$ ayant les significations indiquées ci-dessus, Z et Z' pouvant désigner simultanément le radical méthyle.

Le composé (VI) obtenu dans le premier procédé par réduction ménagée du composé (II) et acétylation du composé (IV) subit une réduction par le fer en présence d'acide acétique à une température comprise entre 50 et 100° C pour conduire au composé (VII).

Le composé (VII) est alkylé ou hydroxyalkylé pour donner le composé (VIII).

Par désacétylation du composé (VIII), on obtient le composé ($III_C$) qui est déshalogéné comme décrit dans le procédé de préparation des composés (IA) en présence de palladium sur charbon, d'acétate d'ammonium et de formiate de triéthylamine dans un milieu solvant constitué par de l'eau, un alcool inférieur ou un mélange hydroalcoolique à une température comprise entre 50° et 100° C.

5°) Procédé de préparation des composés (I) dans lesquels $R_1 \neq R_2 \neq H$

Ces composés dans lesquels $R_1$ et $R_2$ ne désignent pas un atome d'hydrogène et ont des significations différentes, Z et Z' pouvant donc désigner simultanément le radical méthyle, peuvent être préparés par alkylation ou hydroxyalkylation des fonctions amines primaires des composés (IB) ou (IC) selon des méthodes classiques.

Les étapes d'alkylation et d'hydroxyalkylation impliquées dans les différents procédés ci-dessus sont des réactions classiques déjà connues.

Pour l'alkylation, on peut utiliser des halogénures d'alkyle ou des sulfates de dialkyle.

Pour l'hydroxyalkylation, la méthode préférée consiste à faire réagir le chloroformiate de β-chloroéthyle sur le composé portant la fonction amine, à transformer le carbamate obtenu en oxazolidone qui est ensuite hydrolysée pour conduire au dérivé hydroxyéthylé. Ce procédé est décrit dans la demande de brevet français n° 2 571 364. Le carbamate intermédiaire de β-chloroéthyle peut aussi être soumis directement à l'action d'une base minérale forte, telle que la soude ou la potasse, pour donner le composé (I) dans lequel $R_1$ ou $R_2$ est un radical β-hydroxyéthyle.

Les composés de départ de formule (II), à savoir les 3,5-dinitrochlorobenzènes 2,4-disubstitués, peuvent être obtenus selon l'un des trois procédés suivants :

a) Premier procédé

Ce procédé est décrit dans "Recueil T. Chimiques, Pays Bas, R40, p. 451-471. Il consiste à nitrer le 1,2,4-trichlorobenzène par l'acide nitrique fumant en présence éventuellement d'acide sulfurique pour obtenir le 1,2,4-trichloro-3,5-dinitrobenzène dont les atomes de chlore en positions 2 et 4 sont ensuite substitués par les radicaux OZ et OZ′ par réaction avec l'alcoolate alcalin correspondant.

Ce procédé peut être schématisé de la façon suivante :

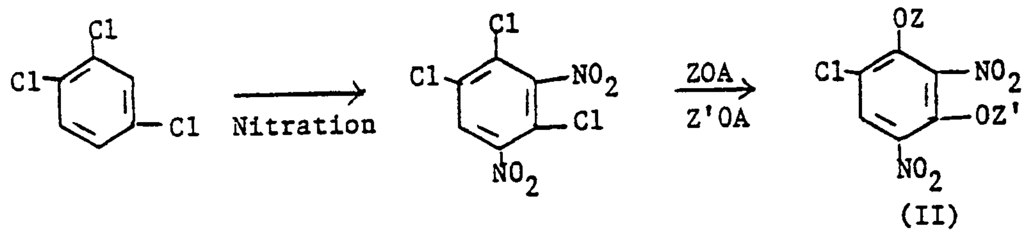


(II)

A étant un métal alcalin

b) Deuxième procédé

Il consiste à nitrer le 2,4-dialcoxychlorobenzène ou le 2,4-di(hydroxyalcoxy)chlorobenzène par l'acide nitrique fumant en présence éventuellement d'acide sulfurique. On obtient en une seule étape respectivement le 2,4-dialcoxy-3,5-dinitrochlorobenzène ou le 2,4-di(hydroxyalcoxy)3,5-dinitrochlorobenzène.

Ce procédé peut être schématisé de la façon suivante :

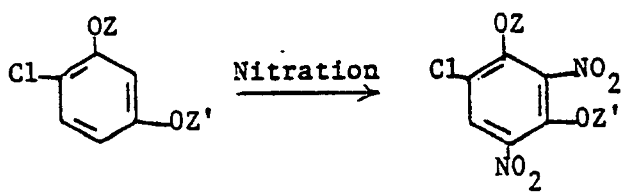


(II)

c) Troisième procédé

Il consiste à alkyler ou hydroxyalkyler le 3,6-dichlorophénol ou le 3,4-dichlorophénol, puis à nitrer le composé obtenu et enfin à substituer un atome de chlore par un radical alcoxy ou hyroxyalcoxy par action d'un alcoolate alcalin ZOA ou Z′OA.

Ce procédé est surtout intéressant dans le cas où Z et Z′ sont différents.

Ce procédé peut être résumé par les deux schémas ci-après :

α)

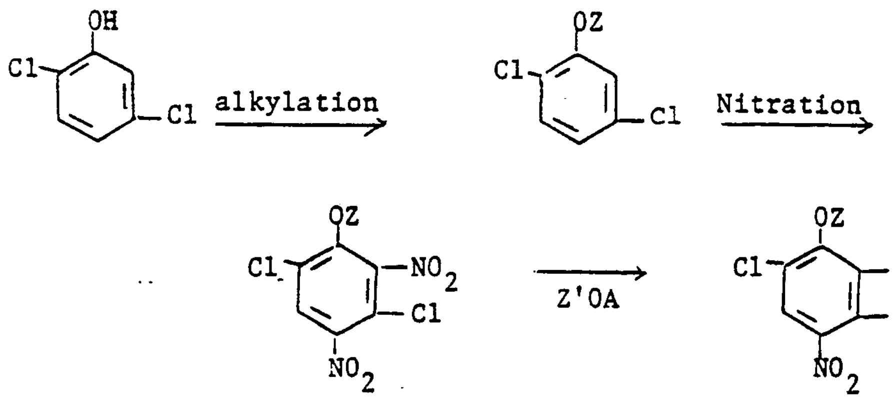


(II)

β)

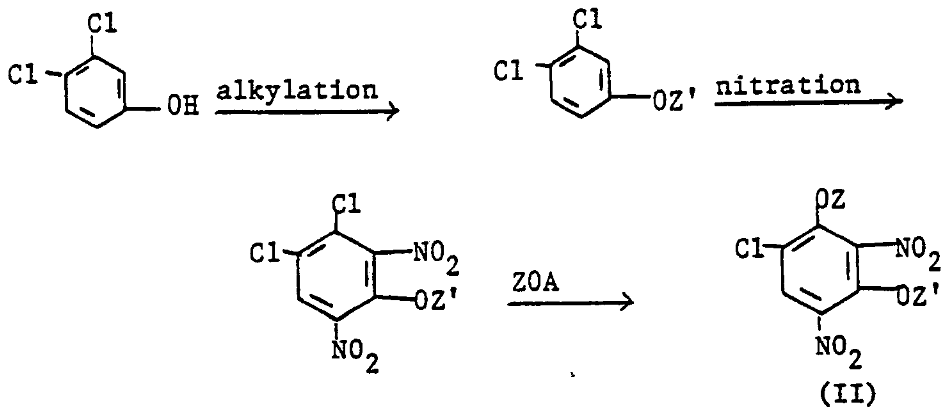


(II)

Des composés de formule (I) particulièrement préférés selon l'invention sont le 4-(β-hydroxyéthyl)-amino-2-amino-1,3-diméthoxybenzène, le 4-amino-2-(β-hydroxyéthyl)amino-1,3-diméthoxybenzène, le 4-méthylamino-2-amino-1,3-diméthoxybenzène, le 2,4-di(β-hydroxyéthyl)amino-1,3-diméthoxybenzène, le 2,4-diamino-1,3-diéthoxybenzène et le 2,4-diamino-1,3-di(γ-hydroxypropoxy)benzène, ainsi que leurs sels d'addition avec un acide et en particulier un acide minéral tel que l'acide chlorhydrique, bromhydrique ou sulfurique.

Des composés intermédiaires de formule (XI) particulièrement préférés selon l'invention sont le 5-amino-2,4-diméthoxy-3-nitrochlorobenzène, le 5-acétamido-2,4-diméthoxy-3-nitrochlorobenzène, le 5-méthylamino-2,4-diméthoxy-3-nitrochlorobenzène et le 5-(β-chloréthoxy carbonyl)amino-2,4-diméthoxy-3-nitrochlorobenzène.

Les compositions de teinture capillaire conformes à l'invention comprennent, à titre de coupleur, au moins un composé de formule (I) ou l'un de ses sels d'acide, en association avec au moins un précurseur de colorant d'oxydation de type para, dans un support aqueux cosmétiquement acceptable.

Le précurseur de colorant d'oxydation de type para est choisi parmi les dérivés benzéniques ou hétérocycliques comme par exemple la pyridine sur lesquels sont fixés en position para deux groupements amino ou un groupement amino et un groupement hydroxy. Ces précurseurs de colorants d'oxydation peuvent être présents dans les compositions tinctoriales sous forme de bases libres ou sous forme de sels d'addition d'acides.

Les précurseurs de colorants d'oxydation particulièrement préférés utilisables conformément à l'invention, sont choisis parmi les paraphénylènediamines répondant à la formule générale (XII) suivant :

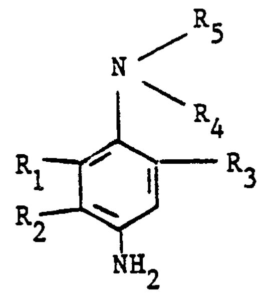

(XII)

ou les sels correspondants, formule dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ peuvent former conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène.

Parmi les composés de formule (XII), on peut citer la p-phénylènediamine, la p-toluylènediamine, la méthoxyparaphénylènediamine, la chloroparaphénylènediamine, la 2,6-diméthyl-p-phénylènediamine, la 2,5-diméthyl-paraphénylènediamine, la 2-méthyl-5-méthoxy-paraphénylènediamine, la 2,6-diméthyl-5-méthoxy-paraphénylènediamine, la N,N-diméthylparaphénylènediamine, la 3-méthyl-4-amino-N,N-diéthylaniline, la N,N-di(β-hydroxyéthyl)paraphénylènediamine, la 3-méthyl-4-amino-N,N-di-(β-hydroxyéthyl)aniline, la 3-chloro-4-amino-N,N-di-(β-hydroxyéthyl)aniline, la 4-amino-N,N-(éthyl, carbamylméthyl) aniline, la 3-méthyl-4-amino-N,N-(éthyl, carbamylméthyl) aniline, la 4-amino-N,N-(éthyl, β-pipéridinoéthyl)aniline, la 3-méthyl-4-amino-N,N-(éthyl,β-pipéridinoéthyl)aniline, la 4-amino-N,N-(éthyl, β-morpholinoéthyl)aniline, la 3-méthyl-4-amino-N,N-(éthyl, β-morpholinoéthyl)aniline, la 4-amino-N,N-(éthyl, β-acétylaminoéthyl)aniline, la 4-amino-N, β-méthoxyéthylaniline, la 3-méthyl-4-amino-N,N-(éthyl, β-acétylaminoéthyl)aniline, la 4-amino-N,N-(éthyl,β-mésylaminoéthyl)aniline, la 3-méthyl-4-amino-N,N-(éthyl, β-mésylaminoéthyl)aniline, la 4-amino-N,N-(éthyl, β-sulfoéthyl)aniline, la 3-méthyl-4-amino-N,N-(éthyl, β-sulfoéthyl)aniline, la N-[(4′-amino)phényl]morpholine, la N-[(4′-amino)phényl] pipéridine, la 2,3-diméthyl-p-phénylènediamine, l'isopropyl-p-phénylènediamine. Ces précurseurs de colorants par oxydation de type para peuvent être introduits dans la composition tinctoriale sous forme de base libre ou sous forme de sels, tels que sous forme de chlorhydrate, de bromhydrate ou de sulfate.

Le composé (I) ou ses sels peut également être utilisé avec des p-aminophénols pour donner des nuances particulièrement stables à la lumière, aux intempéries et au lavage après développement en présence d'oxydant. Parmi les para-aminophénols, on peut citer le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

Le composé (I) ou ses sels peut également être utilisé avec des précurseurs de colorants d'oxydation para hétérocycliques parmi lesquels on peut citer la 2,5-diaminopyridine, la 2-hydroxy-5-aminopyridine, la tétraaminopyrimidine.

Les compositions tinctoriales selon l'invention peuvent également contenir des précurseurs de colorants d'oxydation de type ortho tels que les orthoaminophénols, orthophénylènediamines, orthodiphénols. On peut citer par exemple, le 1-amino-2-hydroxybenzène, le 6-méthyl-1-hydroxy 2-aminobenzène, le 4-méthyl-1-amino-2-hyroxybenzène.

Les compositions tinctoriales conformes à l'invention contenant le composé (I) ou ses sels peuvent contenir éventuellement d'autres coupleurs connus en eux-mêmes tels que les métadiphénols, les métaaminophénols, les métaphénylènediamines, les métaacylaminophénols, les métauréidophénols, les métacarbalcoxyaminophénols, 1′α-naphtol, les coupleurs possédant un groupe méthylène actif tels que les composés β-cétoniques et les pyrazolones.

On peut citer en particulier, à titre d'exemple, le 2,4-dihydroxyphénoxyéthanol,le 2,4-dihydroxyanisole, le métaaminophénol, la résorcine, le monométhyléther de résorcine, le 2-méthyl-5-aminophénol, le 2-méthyl-5-N-(β-hydroxyéthyl)aminophénol, le 2-méthyl-5-N-(β-mésylamino-éthyl)aminophénol, le 2,6-diméthyl-3-aminophénol, la 6-hydroxybenzomorpholine, le 2,4-diaminophénoxyéthanol, la 6-aminobenzomorpholine, le 2-[N-($\beta$-hydroxyéthyl)amino]-4-aminophénoxyéthanol, le 2-amino-4-N-($\beta$-hydroxyéthyl)-aminoanisole, le (2,4-diamino)phényl-$\beta$,$\gamma$-dihydroxypropyléther, la 2,4-diaminophénoxyéthylamine, le 3,4-méthylènedioxyphénol, la 3,4-méthylènedioxyaniline et leurs sels.

On peut rajouter à ces compositions, comme cela est bien connu en vue de nuancer ou enrichir en reflets les colorations apportées par les précurseurs de colorants d'oxydation, des colorants directs tels que des colorants azoïques, anthraquinoniques ou les dérivés nitrés de la série benzénique.

L'ensemble des composés para et des coupleurs utilisés dans les compositions tinctoriales conformes à l'invention représente de préférence de 0,1 à 7% en poids total de ladite composition. La concentration en composé (I) peut varier entre 0,05 et 3,5% du poids total de la composition.

Le support aqueux cosmétiquement acceptable a un pH qui peut varier entre 8 et 11, et il est de préférence compris entre 9 et 11.

Il est ajusté à la valeur désirée à l'aide d'un agent alcalinisant tels que l'ammoniaque, les carbonates alcalins, les alcanolamines comme la mono-, la di- ou la triéthanolamine.

Les compositions tinctoriales conformes à l'invention contiennent également dans leur forme de réalisation préférée des agents tensio-actifs anioniques, cationiques, non-ioniques amphotères ou leurs mélanges. Parmi ces agents tensio-actifs, on peut citer plus particulièrement les alkylbenzènesulfonates, les alkylnaphtalènesulfonates, les sulfates, les éther-sulfates et sulfonates d'alcools gras, les sels d'ammonium quaternaire tels que le bromure de triméthylcétylammonium, le bromure de cétylpyridinium, les éthanolamides d'acides gras éventuellement oxyéthylénés, les acides, les alcools et les amines polyoxyéthylénés, les alcools polyglycérolés, les alkylphénols polyoxyéthylénés ou polyglycérolés ainsi que les alkylsulfates polyoxyéthylénés. Les agents tensio-actifs sont présents dans les compositions conformes à l'invention dans des proportions comprises entre 0,5 et 40% en poids, et de préférence entre 4 et 30% en poids par rapport au poids total de la composition.

Ces compositions peuvent également contenir des solvants organiques pour solubiliser les composés qui ne seraient pas suffisamment solubles dans l'eau. Parmi ces solvants, on peut mentionner à titre d'exemple, les alcanols inférieurs en $C_1$-$C_4$ tels que l'éthanol et l'isopropanol; le glycérol; les glycols ou éthers de glycol comme le 2-butoxyéthanol, l'éthylèneglycol, le propylèneglycol, le monoéthyléther et le monométhyléther du diéthylèneglycol, ainsi que les produits analogues et leurs mélanges. Les solvants sont présents de préférence dans une proportion comprise entre 1 et 40% en poids, et en particulier entre 5 et 30% en poids par rapport au poids total de la composition.

Les agents épaississants que l'on peut ajouter dans les compositions conformes à l'invention sont pris notamment dans le groupe formé par l'alginate de sodium, la gomme arabique, les dérivés de la cellulose tels que la méthylcellulose, l'hydroxyéthylcellulose, l'hydroxypropylcellulose, l'hydroxyméthylcellulose, la carboxyméthylcellulose, les polymères d'acide acrylique, la gomme de Xanthane. On peut également utiliser des agents épaississants minéraux tels que la bentonite. Ces agents épaississants sont présents de préférence en des proportions comprises entre 0,1 et 5% en poids, et en particulier entre 0,5 et 3% en poids par rapport au poids total de la composition.

Les compositions peuvent contenir des agents anti-oxydants choisis en particulier parmi le sulfite de sodium, l'acide thioglycolique, le bisulfite de sodium, l'acide ascorbique et l'hydroquinone. Ces agents anti-oxydants sont présents dans la composition dans des proportions comprises entre 0,05 et 1,5% en poids par rapport au poids total de la composition.

D'autres adjuvants utilisables conformément à l'invention sont par exemple des agents de pénétration, des agents séquestrants, des tampons et des parfums.

Les compositions tinctoriales conformes à l'invention peuvent se présenter sous des formes diverses telles que sous forme de liquides, de crèmes, de gels ou sous toute autre forme appropriée pour réaliser une teinture de fibres kératiniques et notamment des cheveux humains. Elles peuvent également être conditionnées en flacons aérosols en présence d'un agent propulseur.

Les compositions tinctoriales conformes à l'invention, contenant un précurseur de colorant par oxydation de type para et le composé (I) ou l'un de ses sels, sont utilisées dans un procédé de teinture capillaire mettant en oeuvre la révélation par un oxydant.

Conformément à ce procédé, on mélange au moment de l'emploi la composition tinctoriale décrite ci-dessus avec une solution oxydante en une quantité suffisante, puis on applique le mélange obtenu sur les cheveux.

La solution oxydante contient des agents d'oxydation tels que l'eau oxygénée, le peroxyde d'urée ou les persels tels que le persulfate d'ammonium. On utilise de préférence une solution d'eau oxygénée à 20 volumes.

Le mélange obtenu est appliqué sur les cheveux et on laisse poser pendant 10 à 40 minutes, de

préférence 15 à 30 minutes, après quoi on rince les cheveux, on les lave au shampooing, on les rince à nouveau et on les sèche.

Un autre procédé de mise en oeuvre du composé (I) conforme à l'invention consiste à teindre les cheveux suivant un procédé en plusieurs temps, selon lequel, dans un premier temps, on applique le précurseur de colorant d'oxydation para au moyen d'une composition sus-définie et dans un second temps, on applique le composé (I). L'agent oxydant est présent dans la composition appliquée dans le second temps ou bien appliqué sur les cheveux eux-mêmes dans un troisième temps, les conditions de pose et de séchage et de lavage étant identiques.

Les exemples ci-après servent à mieux illustrer l'invention mais ne sont en aucun cas limitatifs de sa portée.

Exemple de préparation n° 1

Préparation du dichlorhydrate de 4-(β-hydroxyéthyl)amino-2-amino-1,3-diméthoxybenzène (composé IB)

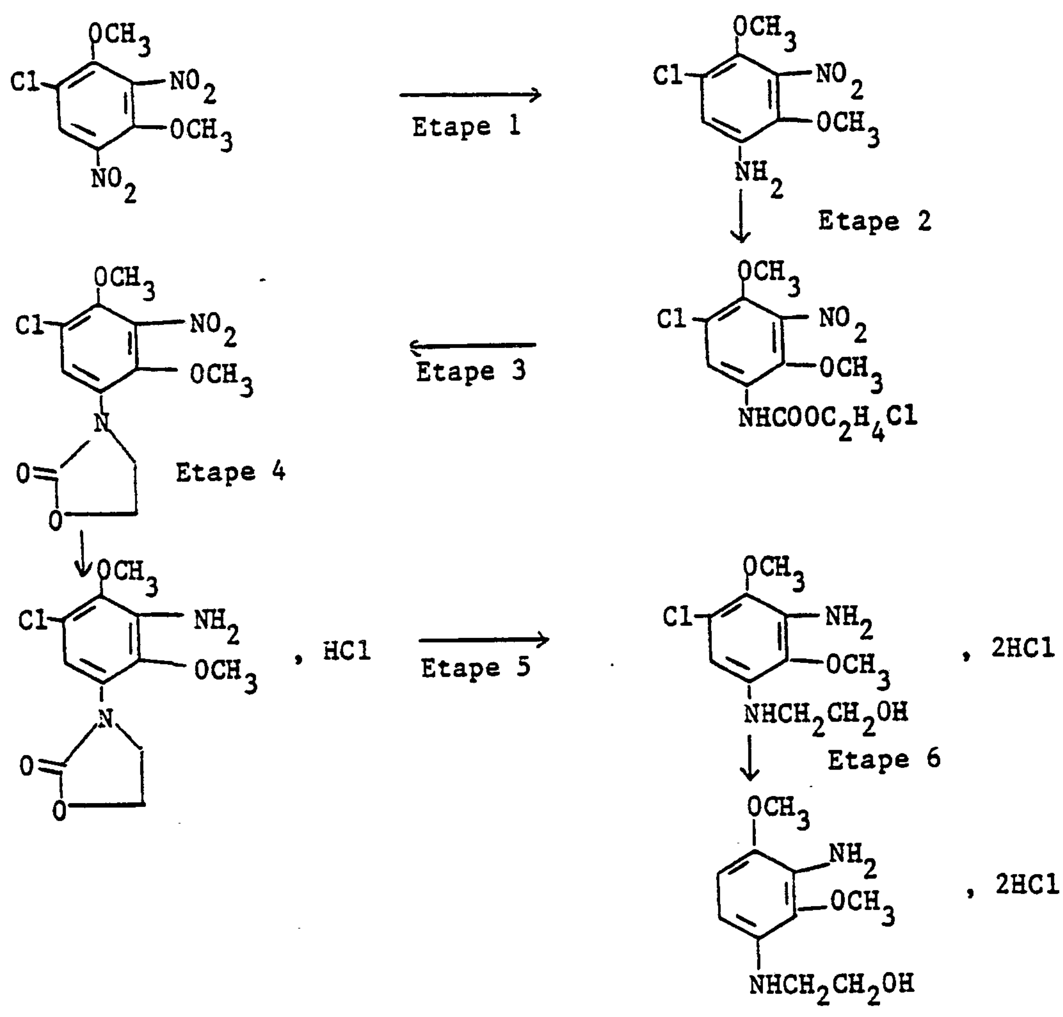


Etape 1

Préparation du 5-amino-2,4-diméthoxy-3-nitrochlorobenzène

On porte au reflux pendant 1 heure le mélange constitué de 0,2 mole (52,5 g) de 3,5-dinitro-2,4-diméthoxychlorobenzène et de 15,9 g de palladium sur charbon (10%) dans 260 ml d'éthanol absolu additionnés de 110 ml de cyclohexène.

Le catalyseur est éliminé par filtration. Par évaporation à sec sous vide, on obtient une huile qui cristallise par ajout d'eau glacée. Après essorage et séchage, le produit obtenu est recristallisé dans un mélange de benzène et de cyclohexane. Il fond à 68° C.

L'analyse élémentaire du produit obtenu est la suivante :

| | Calculé pour $C_8H_9N_2O_4Cl$ | Trouvé |
|---|---|---|
| C% | 41,28 | 41,10 |
| H% | 3,87 | 3,90 |
| N% | 12,04 | 11,98 |
| O% | 27,52 | 27,68 |
| Cl% | 15,27 | 14,99 |

Etape 2

Préparation du 5-(β-chloréthoxycarbonyl)amino-2,4-diméthoxy-3-nitrochlorobenzène

On dissout 0,05 mole (11,7 g) de 5-amino-2,4-diméthoxy-3-nitrochlorobenzène dans 50 ml de dioxane. On ajoute 5 g de carbonate de calcium, puis on élève la température aux environs de 90° C. On introduit alors sous agitation 0,05 mole (7,2 g) de chloroformiate de β-chloréthyle. L'addition terminée, on maintient l'agitation 30 minutes supplémentaires à 90° C. Les sels minéraux présents dans le milieu réactionnel sont éliminés par filtration à chaud. Après addition d'eau glacée au filtrat, le produit attendu cristallise. Le produit obtenu est essoré, lavé à l'eau. Après séchage, il est recristallisé de l'éthanol. Il fond à 93° C.

L'analyse élémentaire du produit obtenu est la suivante :

| | Calculé pour $C_{11}H_{12}Cl_2N_2O_6$ | Trouvé |
|---|---|---|
| C% | 38,94 | 38,78 |
| H% | 3,54 | 3,53 |
| N% | 8,26 | 8,35 |
| O% | 28,32 | 28,40 |
| Cl% | 20,94 | 21,03 |

Etape 3

Préparation de la N-[(3'-chloro-4',6'-diméthoxy-5'-nitro)phényl] oxazolidine-1,3 one-2

On chauffe à 70° C 0,03 mole (10,2 g) de carbamate de β-chloréthyle obtenu selon le mode opératoire décrit à l'étape 2, dans 50 ml de méthanol. On ajoute rapidement 0,03 mole de méthylate de sodium en solution à 30% dans le méthanol. Le chauffage est maintenu 15 minutes supplémentaires après la fin de l'addition. Les sels minéraux sont éliminés par filtration. Du filtrat refroidi et dilué à l'eau glacée on isole par filtration le produit attendu qui a cristallisé. Après séchage, il est recristallisé de l'éthanol. Il fond à 133° C.

L'analyse élémentaire du produit obtenu est la suivante :

| | Calculé pour $C_{11}H_{11}ClN_2O_6$ | Trouvé |
|---|---|---|
| C% | 43,64 | 43,81 |
| H% | 3,64 | 3,64 |
| N% | 9,26 | 9,37 |
| O% | 31,74 | 31,62 |
| Cl% | 11,74 | 11,74 |

Etape 4

Préparation du chlorhydrate de la N-[(3'-chloro-4',6'-diméthoxy-5'-amino)phényl]oxazolidine-1,3 one-2

A 60 ml d'eau additionnés de 3 ml d'acide acétique et préalablement chauffés au bain-marie bouillant, on ajoute 12 g de fer en poudre réduit à l'hydrogène et peu à peu, sous agitation, 0,02 mole (6,05 g) de l'oxazolidine-1,3 one-2 obtenue selon le mode opératoire décrit à l'étape précédente. Les additions terminées, on maintient le chauffage pendant 15 minutes supplémentaires. Les boues ferriques sont éliminées du milieu réactionnel par filtration à chaud. Le milieu réactionnel débarrassé des boues ferriques est extrait à l'acétate d'éthyle. La phase acétate d'éthyle est lavée à l'eau et séchée sur sulfate de sodium. Par ajout d'une solution 7N d'acide chlorhydrique dans l'éthanol absolu on précipite le produit attendu.

L'analyse élémentaire du produit obtenu est la suivante :

| | Calculé pour $C_{11}H_{14}N_2O_4Cl_2$ | Trouvé |
|---|---|---|
| C% | 42,72 | 42,59 |
| H% | 4,53 | 4,64 |
| N% | 9,06 | 8,96 |
| O% | 20,71 | 20,60 |
| Cl% | 22,98 | 22,87 |

Etape 5

Préparation du dichlorhydrate de 5-(β-hydroxyéthyl)amino-3-amino-2,4-diméthoxychlorobenzène

On ajoute 0,03 mole (9,3 g) de chlorhydrate de la N-/(3′-chloro-4′,6′-diméthoxy-5′-amino)-phényl/oxazolidine-1,3 one-2 obtenue selon le mode opératoire décrit dans l'étape précédente à 10 ml d'eau additionnés de 10 ml d'éthanol. A cette solution on ajoute 18 ml de soude 10 N. On chauffe 30 minutes à 80 °C. Après refroidissement et décantation, la phase supérieure est diluée à l'acétate d'éthyle. Après lavage à l'eau puis séchage sur sulfate de sodium, on ajoute 14 ml d'une solution d'acide chlorhydrique 7N dans l'éthanol absolu. Le produit attendu précipite. Il est recristallisé d'une solution hydroalcoolique d'acide chlorhydrique.

L'analyse élémentaire du produit obtenu est la suivante :

| Analyse | Calculé pour $C_{10}H_{17}N_2O_3Cl_3$ | Trouvé |
|---|---|---|
| C% | 37,56 | 37,44 |
| H% | 5,32 | 5,24 |
| N% | 8,76 | 8,83 |
| O% | 15,02 | 15,21 |
| Cl% | 33,33 | 33,07 |

Etape 6

Préparation du dichlorhydrate de 4-(β-hydroxyéthyl)amino-2-amino-1,3-diméthoxybenzène

On chauffe sous agitation, au reflux, le mélange constitué par 37,2 g d'acétate d'ammonium, 33 g de palladium à 10% sur charbon, 0,12 mole (38,5 g) de dichlorhydrate de 5-(β-hydroxyéthyl)amino-3-amino-2,4-diméthoxychlorobenzène obtenu selon le mode opératoire décrit à l'étape 5 dans 240 ml d'éthanol additionnés de 24 ml d'eau. On ajoute 36,4 ml de triéthylamine, puis goutte à goutte 15,5 g d'acide formique. Le chauffage est maintenu 30 minutes après la fin de l'addition. Le catalyseur est éliminé par filtration à chaud. L'extrait sec obtenu par évaporation sous vide du filtrat est dissous dans l'acétate d'éthyle. A l'acétate d'éthyle séché sur sulfate de sodium, on ajoute 52 ml d'une solution 7N d'acide chlorhydrique dans l'éthanol absolu pour précipiter le produit attendu. Il est recristallisé à chaud d'une solution hydroalcoolique d'acide chlorhydrique.

L'analyse du produit obtenu est la suivante :

| | Calculé pour $C_{10}H_{18}N_2O_3Cl_2, 1/_4H_2O$ | Trouvé |
|---|---|---|
| C% | 41,45 | 41,32 |
| H% | 6,39 | 6,33 |
| N% | 9,67 | 9,58 |
| O% | 17,96 | 18,06 |
| Cl% | 24,52 | 24,45 |

Exemple de préparation n° 2

Préparation du dichlorhydrate de 4-amino-2-(β-hydroxyéthyl)amino-1,3-diméthoxybenzène (composé IC)

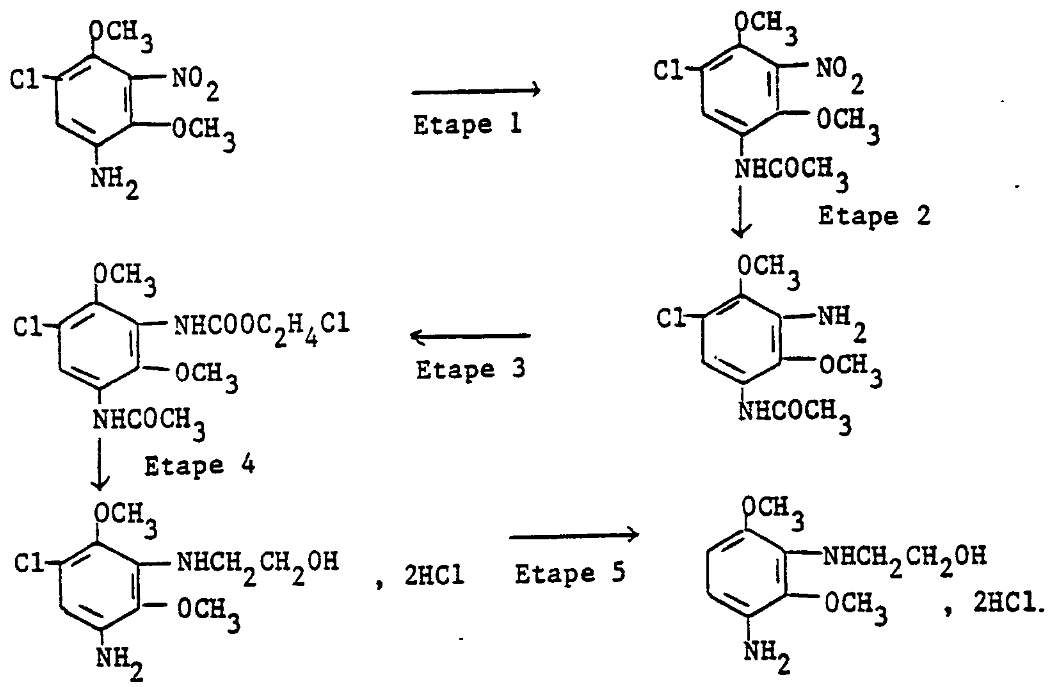


Etape 1

Préparation du 5-acétamido-2,4-diméthoxy-3-nitrochlorobenzène

On chauffe à 70° C 330 ml d'anhydride acétique additionnés de quelques gouttes d'acide sulfurique. On ajoute sous agitation 0,7 mole (163 g) de 5-amino-2,4-diméthoxy-3-nitrochlorobenzène préparé selon le mode opératoire décrit dans l'exemple 1 (étape 1).

Par dilution du milieu réactionnel par de l'eau glacée, le produit attendu précipite. Recristallisé de l'éthanol à 96°, il fond à 115° C.

L'analyse élémentaire du produit obtenu est la suivante :

| | Calculé pour $C_{10}H_{11}N_2O_5Cl$ | Trouvé |
|---|---|---|
| C% | 43,72 | 43,67 |
| H% | 4,01 | 4,07 |
| N% | 10,20 | 10,17 |
| O% | 29,14 | 29,04 |
| Cl% | 12,93 | 12,98 |

Etape 2
Préparation du 5-acétamido-2,4-diméthoxy-3-aminochlorobenzène

A 760 ml d'eau additionnés de 13 ml d'acide acétique et préalablement chauffés à 80 °C on ajoute 253 g de fer en poudre réduit à l'hydrogène et peu à peu, sous agitation, 0,46 mole (126,5 g) de 5-acétamido-2,4-diméthoxy-3-nitrochlorobenzène préparé à l'étape précédente. Les additions terminées, on maintient le milieu réactionnel au bain-marie bouillant pendant 15 minutes. Après refroidissement, le milieu réactionnel est centrifugé. Les boues ferriques séparées de la phase liquide sont extraites à l'acétate d'éthyle. La phase acétate d'éthyle lavée à l'eau puis séchée sur sulfate de sodium est diluée par 100 ml d'une solution 7N d'acide chlorhydrique dans l'éthanol absolu. Le produit attendu, qui a précipité sous forme de chlorhydrate, est essoré. Il est dissous dans le minimum d'eau. Après neutralisation, le produit attendu précipite. Recristallisé de l'alcool il fond à 113 °C.

L'analyse élémentaire du produit obtenu est la suivante :

| Analyse | Calculé pour $C_{10}H_{13}ClN_2O_3$ | Trouvé |
|---|---|---|
| C% | 49,08 | 49,12 |
| H% | 5,32 | 5,31 |
| N% | 11,45 | 11,50 |
| O% | 19,63 | 19,74 |
| Cl% | 14,52 | 14,49 |

Etape 3
Préparation du 5-acétamido-2,4-diméthoxy-3-(β-chloréthoxycarbonyl)-aminochlorobenzène

A 70 ml de dioxane on ajoute 0,05 mole (14 g) de chlorhydrate de 5-acétamido-2,4-diméthoxy-3-aminochlorobenzène obtenu selon l'étape précédente, puis 5,5 ml de soude 10N. On élève la température au voisinage de 90 °C puis on ajoute 5 g de carbonate de calcium. On introduit alors sous agitation 7,55 g de chloroformiate de β-chloréthyle. L'addition terminée, on maintient le chauffage 30 minutes à 90 °C. Le milieu réactionnel est dilué par un mélange glace/eau après refroidissement. Le produit attendu précipite après acidification du milieu réactionnel. Recristallisé de l'éthanol il fond à 148 °C.

L'analyse élémentaire du produit obtenu est la suivante :

| | Calculé pour $C_{13}H_{16}Cl_2N_2O_5$ | Trouvé |
|---|---|---|
| C% | 44,44 | 44,46 |
| H% | 4,56 | 4,54 |
| N% | 7,98 | 7,95 |
| O% | 22,79 | 22,66 |
| Cl% | 20,23 | 20,29 |

Etape 4
Préparation du dichlorhydrate de 5-amino-3-(β-hydroxyéthyl)amino-2,4-diméthoxychlorobenzène

On porte au reflux le mélange constitué de 0,25 mole (88 g) de carbamate de β-chloréthyle obtenu à l'étape précédente et 250 ml de soude 10N dans 10 ml d'eau additionnés de 15 ml d'éthanol. Après 1 heure de chauffage, le milieu réactionnel refroidi et neutralisé est extrait à l'acétate d'éthyle. Les phases acétate d'éthyle rassemblées sont évaporées sous vide après avoir été lavées et séchées sur sulfate de sodium. L'extrait sec obtenu est dissous dans de l'éther isopropylique. Le produit attendu précipite après ajout d'une solution 7N d'acide chlorhydrique dans l'éthanol absolu. Il est recristallisé d'un mélange hydroalcoolique contenant de l'acide chlorhydrique.

L'analyse élémentaire du produit est la suivante :

| | Calculé pour $C_{10}H_{17}N_2O_3Cl_3$ | Trouvé |
|---|---|---|
| C% | 37,56 | 37,59 |
| H% | 5,32 | 5,36 |
| N% | 8,76 | 8,68 |
| O% | 15,02 | 15,24 |
| Cl% | 33,33 | 33,21 |

Etape 5
Préparation du dichlorhydrate de 4-amino-2-(β-hydroxyéthyl)amino-1,3-diméthoxybenzène

A 60 ml d'éthanol additionnés de 6 ml d'eau contenant 9,3 g d'acétate d'ammonium, 4,8 g de palladium à 10% sur charbon et 0,03 mole (9,6 g) du composé préparé à l'étape précédente, préalablement chauffés à 80°C, on ajoute 9,1 g de triéthylamine puis lentement, sous agitation 3,9 g d'acide formique. Les additions terminées, on maintient le milieu réactionnel 15 minutes à 80°C. Le milieu réactionnel après refroidissement est filtré afin d'éliminer le catalyseur. L:e filtrat évaporé à sec sous vide est dilué à l'acétate d'éthyle. A cette solution préalablement séchée sur sulfate de sodium, on ajoute 10 ml d'une solution 7N d'acide chlorhydrique dans l'éthanol absolu pour précipiter le produit attendu. Il est purifié par dissolution dans le minimum d'eau chaude et précipitation par ajout d'une solution 7N d'acide chlorhydrique dans l'éthanol absolu.

L'analyse élémentaire du produit obtenu est la suivante :

| | Calculé pour $C_{10}H_{18}N_2O_3Cl_2$ | Trouvé |
|---|---|---|
| C% | 42,11 | 41,93 |
| H% | 6,32 | 6,38 |
| N% | 9,82 | 9,79 |
| O% | 16,84 | 17,02 |
| Cl% | 24,91 | 24,77 |

Exemple de préparation n°3
Préparation du dichlorhydrate de 2-méthylamino-4-amino-1,3-diméthoxybenzène (Composé IB)

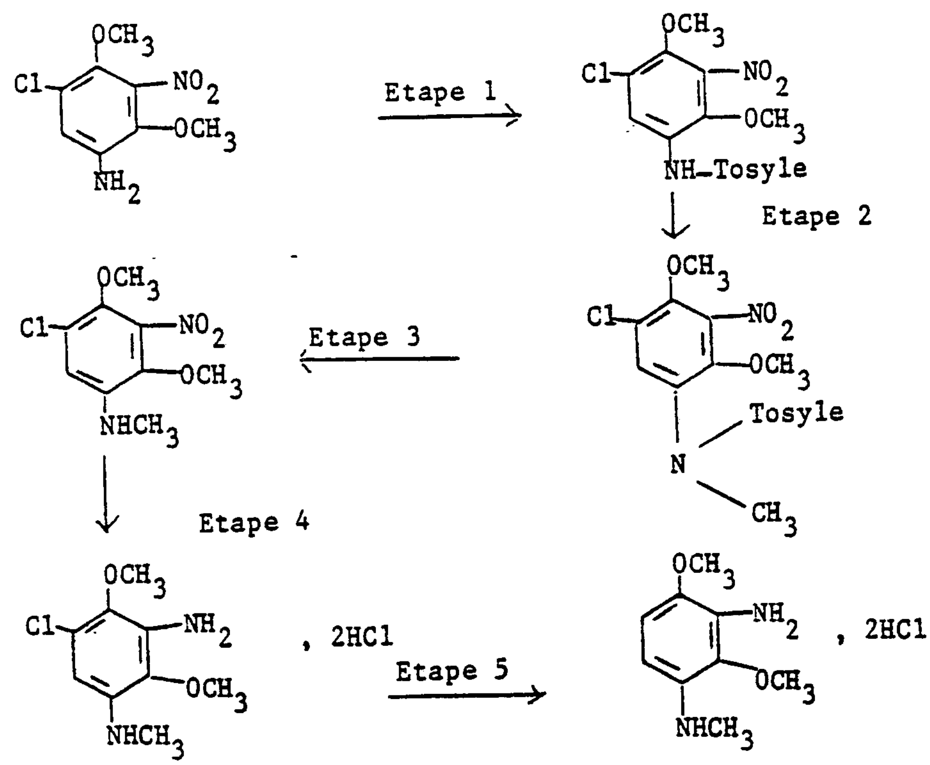


Etape 1
Préparation du 5-N-tosylamino-2,4-diméthoxy-3-nitrochlorobenzène

Ou ajoute peu à peu, à 40°C, 0,11 mole (21 g) de p-toluènesulfochlorure à une solution de 0,1 mole (23,35 g) de 5-amino-2,4-diméthoxy-3-nitrochlorobenzène préparé à l'étape 1 de l'exemple 1. On poursuit l'agitation 15 minutes à 40°C après la fin de l'addition.

Le mélange réactionnel est dilué par l'eau glacée. Par acidification par l'acide chlorhydrique concentré, le produit attendu précipite. Après essorage, lavage à l'eau puis à l'alcool, le produit est séché. Il est recristallisé de l'éthanol.

L'analyse du produit obtenu donne les résultats suivants :

| Analyse | Calculé pour $C_{15}H_{15}N_2O_6SCl$ | Trouvé |
|---|---|---|
| C% | 46,57 | 46,49 |
| H% | 3,88 | 3,90 |
| N% | 7,24 | 7,38 |
| O% | 24,84 | 24,56 |
| S% | 8,28 | 8,17 |
| Cl% | 9,18 | 9,35 |

Etape 2
Préparation du 5-N,N-tosyl, méthylamino-2,4-diméthoxy-3-nitro-chlorobenzène

On ajoute à 30°-35°C 0,022 mole (2,1 ml) de sulfate de méthyle à une solution de 0,02 mole (7,7 g) de 5-N-tosylamino-2,4-diméthoxy-3-nitro-chlorobenzène préparé à l'étape précédente dans 25 ml d'une solution normale de soude. On poursuit l'agitation 15 minutes après la fin de l'addition. Le produit attendu précipite par dilution du milieu réactionnel avec de l'eau glacée. Après essorage et lavage à l'eau, le produit obtenu est recristallisé de l'alcool.

L'analyse du produit obtenu donne les résultats suivants :

| Analyse | Calculé pour $C_{16}H_{17}N_2O_6SCl$ | Trouvé |
|---|---|---|
| C% | 47,94 | 47,96 |
| H% | 4,24 | 4,28 |
| N% | 6,99 | 7,00 |
| O% | 23,97 | 23,94 |
| S% | 7,99 | 7,84 |
| Cl% | 8,86 | 8,91 |

Etape 3

Préparation du 5-méthylamino-2,4-diméthoxy-3-nitrochlorobenzène

On ajoute peu à peu 0,025 mole (10 g) du composé préparé selon l'étape précédente à 20 ml d'acide sulfurique concentré, la température étant maintenue à 20° C; 15 minutes après la fin de l'addition, le milieu réactionnel est dilué par un mélange glace/eau. Le produit attendu qui a précipité, est essoré, lavé à l'eau, séché sous vide en présence de $P_2O_5$. Il est recristallisé du cyclohexane; il fond à 57° C.

L'analyse du produit obtenu donne les résultats suivants :

| Analyse | Calculé pour $C_9H_{11}N_2O_4Cl$ | Trouvé |
|---|---|---|
| C% | 43,81 | 43,77 |
| H% | 4,46 | 4,55 |
| N% | 11,36 | 11,35 |
| O% | 25,96 | 26,12 |
| Cl% | 14,40 | 14,36 |

Etape 4

Préparation du dichlorhydrate de 5-méthylamino-3-amino-2,4-diméthoxychlorobenzène

A 200 ml d'eau additionnés de 2,5 ml d'acide acétique et préalablement chauffés au bain-marie bouillant, on ajoute 50 g de fer en poudre réduit à l'hydrogène et peu à peu, sous agitation, 0,1 mole (24,65 g) de 5-méthylamino-2,4-diméthoxy-3-nitrochlorobenzène obtenu selon le mode opératoire de l'étape 3. Les additions terminées, on maintient le chauffage 15 minutes supplémentaires. Le milieu réactionnel est centrifugé, le produit attendu est extrait des boues ferriques par l'acétate d'éthyle. Les phases acétate d'éthyle sont lavées à l'eau, puis séchées sur sulfate de sodium anhydre. Par ajout de 43 ml d'une solution 7N d'acide chlorhydrique dans l'éthanol, on précipite le produit attendu. Après essorage du précipité, lavage à l'éthanol, puis séchage, le produit obtenu est recristallisé d'une solution hydroalcoolique d'acide chlorhydrique.

L'analyse du produit obtenu donne les résultats suivants :

| Analyse | Calculé pour $C_9H_{15}N_2O_2Cl_3$ | Trouvé |
|---|---|---|
| C% | 37,31 | 37,21 |
| H% | 5,18 | 5,23 |
| N% | 9,67 | 9,54 |
| O% | 11,05 | 11,10 |
| Cl% | 36,79 | 36,86 |

Etape 5

Préparation du dichlorhydrate de 4-méthylamino-2-amino-1,3-diméthoxybenzène

On chauffe à 70° C sous agitation le mélange constitué par 8,5 g d'acétate d'ammonium, 7,5 g de palladium à 10% sur charbon, 0,05 mole (15 g) de dichlorhydrate de 5-méthylamino-3-amino-2,4-diméthoxychlorobenzène dans 14 ml d'eau additionnés de 90 ml d'éthanol et 15,2 g de triéthylènediamine, puis on ajoute goutte à goutte 6,44 g d'acide formique. Le chauffage est maintenu 1 heure après la fin de l'addition. Le catalyseur est éliminé par filtration. L'extrait sec obtenu par évaporation sous vide du filtrat est dissous dans l'acétate d'éthyle. A l'acétate d'éthyle séché sur sulfate de sodium, on ajoute une solution 7N d'acide chlorhydrique dans l'éthanol absolu pour précipiter le produit attendu. Il est recristallisé à chaud d'une solution hydroalcoolique d'acide chlorhydrique.

L'analyse du produit obtenu est la suivante :

| Analyse | Calculé pour $C_9H_{16}N_2O_2Cl_2$ | Trouvé |
|---|---|---|
| C% | 42,35 | 42,36 |
| H% | 6,27 | 6,35 |
| N% | 10,98 | 10,88 |
| O% | 12,55 | 12,70 |
| Cl% | 27,84 | 27,81 |

Exemple de préparation n° 4

Préparation du dichlorhydrate de 2,4-di(β-hydroxyéthyl)amino-1,3-diméthoxybenzène

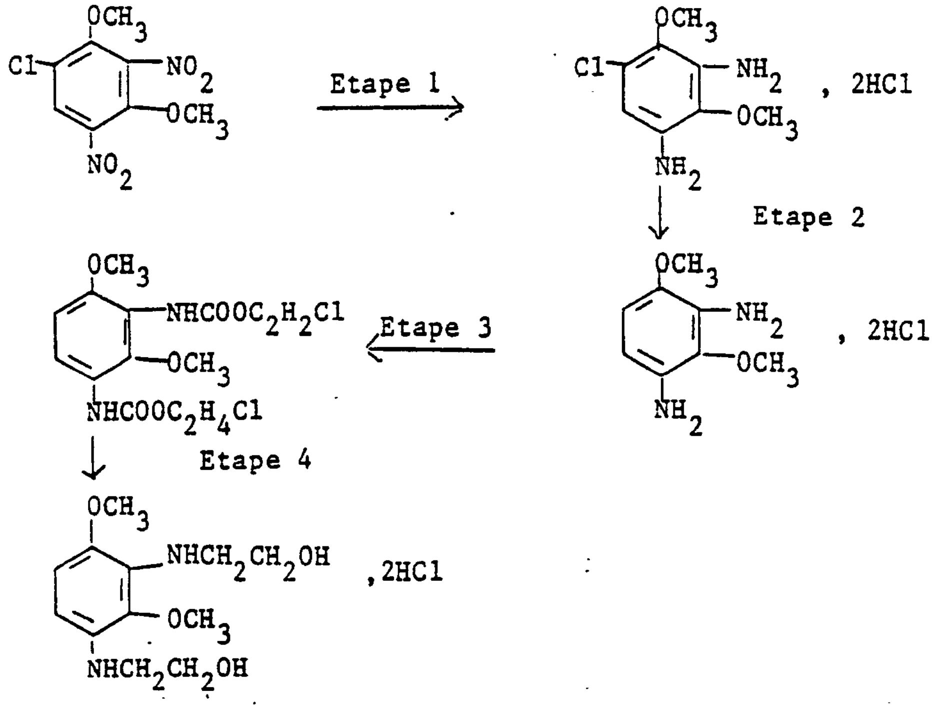


Etape 1
Préparation du dichlorhydrate de 2,4-diméthoxy-3,5-diaminochlorobenzène

A 270 ml d'eau additionnés de 27 ml d'acide acétique préalablement chauffés à 80° C, on ajoute 100 g de fer en poudre réduit à l'hydrogène et peu à peu, sous agitation, 0,25 mole (66 g) de 2,4-diméthoxy-3,5-dinitrochlorobenzène. Les additions terminées, on maintient le milieu réactionnel au bain-marie bouillant pendant 30 minutes supplémentaires. Après refroidissement, le milieu réactionnel est centrifugé. Les boues ferriques qui contiennent le produit attendu sont reprises sous brassage par de l'acétone. Après filtration des boues ferriques puis lavage à l'acétone, le produit attendu précipite du filtrat acétonique par ajout d'une solution d'acide chlorhydrique dans l'éthanol. Après essorage et lavage, le produit attendu est recristallisé à chaud d'un mélange d'acide chlorhydrique et d'eau.

L'analyse élémentaire du produit obtenu donne les résultats suivants :

| Analyse | Calculé pour $C_8H_{13}N_2Cl_3O_2$ | Trouvé |
|---|---|---|
| C% | 34,85 | 34,85 |
| H% | 4,72 | 4,82 |
| N% | 10,16 | 10,03 |
| O% | 11,62 | 11,80 |
| Cl% | 38,66 | 38,46 |

Etape 2
Préparation du dichlorhydrate de 2,4-diamino-1,3-diméthoxybenzène

On chauffe à 75° C sous agitation le mélange constitué par 77 g d'acétate d'ammonium, 42 g de palladium à 10% sur charbon, 0,25 mole (69 g) de dichlorhydrate de 2,4-diméthoxy-3,5-diaminochlorobenzène dans 420 ml d'éthanol additionés de 50 ml d'eau. On ajoute 75 g de triéthylamine puis, goutte à goutte, 31 g d'acide formique. Après 30 minutes de chauffage supplémentaire, le milieu réactionnel est filtré chaud. Le filtrat est évaporé à sec. Par ajout d'acétate d'éthyle, on précipite les sels minéraux que l'on

élimine par essorage. On ajoute au filtrat séché sur sulfate de sodium 10 ml d'une solution alcoolique d'acide chlorhydrique 7N. Le produit attendu précipite, il est recristallisé d'un mélange d'eau et d'une solution d'acide chlorhydrique dans l'éthanol.

L'analyse élémentaire du produit obtenu donne les résultats suivants :

| Analyse | Calculé pour $C_8H_{14}N_2O_2Cl_2$ | Trouvé |
|---|---|---|
| C% | 39,83 | 39,75 |
| H% | 5,81 | 5,79 |
| N% | 11,62 | 11,60 |
| O% | 13,28 | 13,54 |
| Cl% | 29,46 | 29,29 |

Etape 3

Préparation du 2,4-di(β-chloréthoxycarbonyl)amino-1,3-diméthoxybenzène

A 90 ml de dioxane, on ajoute 0,05 mole (12,05 g) de dichlorhydrate de 2,4-diamino-1,3-diméthoxybenzène puis 10 ml de soude 10N. On élève la température au voisinage de 85° -90° C puis on ajoute 10 g de carbonate de calcium. On introduit alors sous agitation 15 g de chloroformiate de β-chloréthyle. L'addition terminée, on maintient le chauffage à 90° C pendant 15 minutes. Le milieu réactionnel est dilué par 500 g d'eau glacée. Par acidification du milieu réactionnel, le produit attendu précipite lentement. Après essorage, lavage à l'eau puis séchage sous vide en présence de $P_2O_5$, il peut être recristallisé du benzène. Il fond à 105° C.

L'analyse du produit obtenu donne les résultats suivants :

| Analyse | Calculé pour $C_{14}H_{18}N_2O_6Cl_2$ | Trouvé |
|---|---|---|
| C% | 44,09 | 43,99 |
| H% | 4,72 | 4,77 |
| N% | 7,35 | 7,31 |
| O% | 25,20 | 25,25 |
| Cl% | 18,60 | 18,72 |

Etape 4

Préparation du dichlorhydrate de 2,4-di(β-hydroxyéthyl)amino-1,3-diméthoxybenzène

On chauffe 1 heure au reflux le mélange constitué de 0,22 mole (83 g) de 2,4-di(β-chloréthoxycarbonyl)amino-1,3-diméthoxybenzène préparé selon le mode opératoire décrit à l'étape 3 et de 200 ml de soude 10N dans 200 ml d'alcool additionnés de 100 ml d'eau.

L'alcool est chassé sous pression réduite. Après neutralisation des eaux, le produit est extrait à l'acétate d'éthyle. Le produit attendu est obtenu par ajout d'une solution d'acide chlorhydrique dans l'éthanol absolu aux phases acétate d'éthyle préalablement séchées sur sulfate de sodium. Après essorage, puis séchage sous vide en présence de $P_2O_5$, il est recristallisé d'une solution hydroalcoolique d'acide chlorhydrique.

L'analyse du produit obtenu donne les résultats suivants :

| Analyse | Calculé pour $C_{12}H_{22}N_2O_4Cl_2$ | Trouvé |
|---|---|---|
| C% | 43,77 | 43,39 |
| H% | 6,69 | 6,76 |
| N% | 8,51 | 8,44 |
| O% | 19,45 | 19,92 |
| Cl% | 21,58 | 21,39 |

Exemple de préparation n° 5 (Procédé I)
Préparation du dichlorydrate de 2,4-diamino-1,3-diéthoxybenzène

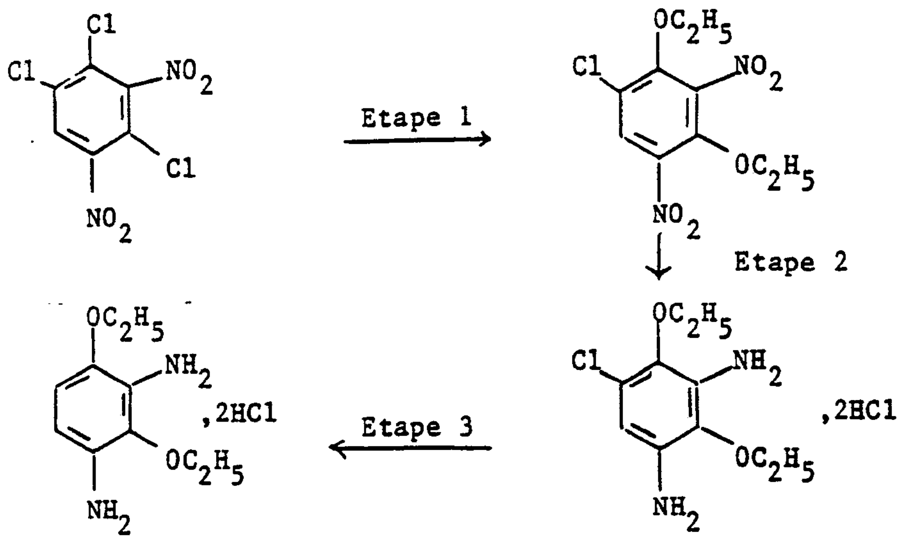


Etape 1
Préparation du 3,5-dinitro-2,4-diéthoxychlorobenzène

On porte à 75°C 0,1 mole (27,15 g) de 3,5-dinitro-1,2,4-trichlorobenzène dans 110 ml d'éthanol absolu. On ajoute 0,2 mole d'éthylate de sodium en solution à 15% dans l'éthanol absolu. Le mélange réactionnel est chauffé 30 minutes à 75°C après la fin de la coulée, puis dilué par 300 g d'un mélange glace/eau. Le produit attendu précipite. Après séchage sous vide et recristallisation de l'éther isopropylique, il fond à 78°C.

L'analyse élémentaire du produit obtenu est la suivante :

| Analyse | Calculé pour $C_{20}H_{11}N_2O_6Cl$ | Trouvé |
|---|---|---|
| C% | 41,31 | 41,22 |
| H% | 3,79 | 3,81 |
| N% | 9,64 | 9,65 |
| O% | 33,05 | 32,88 |
| Cl% | 12,22 | 12,07 |

Etape 2
Préparation du dichlorhydrate de 2,4-diéthoxy-3,5-diaminochlorobenzène

A 450 ml d'eau additionnés de 8,5 ml d'acide acétique préalablement chauffé à 80 °C, on ajoute 170 g de fer en poudre réduit à l'hydrogène et peu à peu, sous agitation, 0,3 mole (87g) de 3,5-dinitro-2,4-diéthoxychlorobenzène. Les additions terminées, on maintient le milieu réactionnel au bain-marie bouillant pendant 30 minutes supplémentaires. Après refroidissement, le milieu réactionnel est centrifugé. Les boues ferriques sont réempâtées dans l'acétate d'éthyle, et les eaux mères sont extraites à l'acétate d'éthyle. Les phases acétate d'éthyle réunies sont lavées à l'eau puis séchées sur $Na_2SO_4$. Par ajout d'une solution d'acide chlorhydrique dans l'éthanol, le produit attendu précipite. Après essorage, il est lavé à l'acétone. Il est recristallisé d'une solution hydro-alcoolique d'acide chlorhydrique.

L'analyse du produit obtenu donne les résultats suivants :

| Analyse | Calculé pour $C_{10}H_{17}N_2O_2Cl_3$ | Trouvé |
|---|---|---|
| C% | 39,54 | 39,67 |
| H% | 5,60 | 5,64 |
| N% | 9,23 | 9,26 |
| O% | 10,54 | 10,48 |
| Cl% | 35,09 | 35,26 |

Etape 3

Préparation du dichlorhydrate de 2,4-diamino-1,3-diéthoxybenzène

On chauffe à 70 °C sous agitation le mélange constitué par 7,7 g d'acétate d'ammonium, 3,8 g de palladium à 10% sur charbon, 0,025 mole (7,6 g) de dichlorhydrate de 2,4-diéthoxy-3,5-diaminochlorobenzènedans 42 ml d'éthanol additionnés de 5 ml d'eau. On ajoute 7,5 g de triéthylamine, puis, goutte à goutte, 3,1 g d'acide formique. Après 20 minutes de chauffage supplémentaires à 80 °C, le milieu réactionnel est filtré à chaud. Le filtrat est évaporé à sec. Par ajout d'acétate d'éthyle, on précipite les sels minéraux que l'on élimine par filtration. On ajoute au filtrat séché sur sulfate de sodium 10 ml d'une solution alcoolique d'acide chlorhydrique 7N. Le produit attendu précipite; il est recristallisé d'une solution d'acide chlorhydrique 6N.

L'analyse du produit obtenu donne les résultats suivants :

| Analyse | Calculé pour $C_{10}H_{18}N_2O_2Cl_2$ | Trouvé |
|---|---|---|
| C% | 44,61 | 44,70 |
| H% | 6,69 | 6,75 |
| N% | 10,41 | 10,37 |
| O% | 11,90 | 12,00 |
| Cl% | 26,39 | 26,29 |

Exemple de préparation n° 6 (Procédé II)

Préparation du dichlorhydrate de 2,4-diamino-1,3-diéthoxybenzène (directement à partir du 2,4-diéthoxy-3,5-dinitrochlorobenzène)

On chauffe à 80 °C pendant 1 heure sous une pression de 20 kg d'hydrogène le mélange constitué par 0,1 mole (29 g) de 2,4-diéthoxy-3,5-dinitrochlorobenzène, 15,4 g d'acétate d'ammonium et 5,2 g de palladium à 10% sur charbon dans 100 ml d'éthanol additionnés de 15 ml d'eau.

Le milieu réactionnel est filtré à chaud afin d'éliminer le catalyseur. Le filtrat est évaporé à sec sous pression réduite. On ajoute de l'acétate d'éthyle afin de précipiter les sels minéraux qu'on élimine par essorage. Après séchage sur sulfate de sodium, on précipite le produit attendu par ajout d'une solution 7N d'acide chlorhydrique dans l'éthanol absolu. Le produit ainsi obtenu est identique au produit préparé dans l'exemple de préparation n° 5.

Exemple de préparation n° 7

Préparation du dichlorhydrate de 2,4-diamino-1,3-di-(γ-hydroxypropoxy)benzène

Etape 1

Préparation du 3,5-dinitro-2,4-di-(γ-hydroxypropoxy)chlorobenzène

On porte à 85° C 1 mole (271,5 g) de 3,5-dinitro-1,2,4-trichlorobenzène dans 700 ml de 1,3-propanediol. On ajoute en 30 minutes 2 moles de potasse en poudre dissoute dans 280 ml de 1,3-propanediol. Le mélange réactionnel est chauffé 1 heure à 85° C après la fin de l'addition. Après refroidissement, le produit attendu est filtré, lavé à l'eau puis dissous dans 1,5 litre d'acétate d'éthyle qui, après lavage à l'eau, est séché sur sulfate de sodium. Par concentration à sec, on obtient un précipité qui, recristallisé de l'éther isopropylique, fond à 90° C.

L'analyse du produit obtenu donne les résultats suivants :

| Analyse | Calculé pour $C_{12}H_{15}N_2O_8Cl$ | Trouvé |
|---|---|---|
| C% | 41,08 | 41,12 |
| H% | 4,28 | 4,33 |
| N% | 7,99 | 8,04 |
| O% | 36,52 | 36,45 |
| Cl% | 10,13 | 10,09 |

Etape 2

Préparation du dichlorhydrate de 2,4-di-(γ-hydroxypropoxy)-3,5-diamino chlorobenzène

A 700 ml d'eau additionnée de 10 ml d'acide acétique préalablement chauffé à 95° C, on ajoute 250 g de fer en poudre réduit à l'hydrogène et peu à peu, sous agitation, 0,34 mole (119 g) de 3,5-dinitro-2,4-di-(γ-hydroxypropoxy)chlorobenzène. Les additions terminées, on maintient le milieu réactionnel 10 minutes supplémentaires à 95° C. Après refroidissement, on ajoute 1 litre d'acétate d'éthyle. Le milieu réactionnel est filtré afin d'éliminer les boues ferriques. La phase acétate d'éthyle, séparée de la phase aqueuse, est, après lavage à l'eau puis séchage par du sulfate de sodium, évaporée à sec. L'extrait sec ainsi obtenu est repris par une solution d'acide chlorhydrique dans l'éthanol absolu. Le produit attendu précipite. Après essorage puis séchage, l'analyse du produit obtenu donne les résultats suivants :

| Analyse | Calculé pour $C_{12}H_{21}N_2O_4Cl_3$ | Trouvé |
|---|---|---|
| C% | 39,62 | 39,49 |
| H% | 5,78 | 5,67 |
| N% | 7,70 | 7,56 |
| O% | 17,61 | 17,81 |
| Cl% | 29,30 | 29,26 |

Etape 3

Préparation du dichlorhydrate de 2,4-diamino-1,3-di-(γ-hydroxypropoxy)benzène

On ajoute 0,45 mole (63,6 ml) de triéthylamine au mélange constitué par 47 g d'acétate d'ammonium, 27 g de palladium à 10% sur charbon et 0,15 mole (55 g) de dichlorhydrate de 2,4-di-(γ-hydroxypropyl) 3,5-diamino-chlorobenzène dans 270 ml d'éthanol additionné de 27 ml d'eau. A ce milieu réactionnel porté au reflux, on ajoute peu à peu (forte exothermicité) 0,4 mole (18,4 g) d'acide formique en 15 minutes environ. Le milieu réactionnel est filtré à chaud pour éliminer le catalyseur puis concentré à sec. Par ajout

d'acétate d'éthyle, les sels minéraux précipitent; après les avoir filtrés, on ajoute une solution alcoolique 7N d'acide chlorhydrique : le produit attendu cristallise.

L'analyse du produit obtenu donne les résultats suivants :

| Analyse | Calculé pour $C_{12}H_{22}N_2O_4Cl_2$ | Trouvé. |
|---|---|---|
| C% | 43,77 | 43,82 |
| H% | 6,69 | 6,67 |
| N% | 8,51 | 8,40 |
| O% | 19,45 | 19,68 |
| Cl% | 21,58 | 21,48 |

Exemple de teinture 1

On prépare le mélange tinctorial suivant :

| | | |
|---|---|---|
| - Dichlorhydrate de 4-(β-hydroxyéthyl)amino-2-amino-1,3-diméthoxybenzène | | 0,71 g |
| - p-phénylènediamine | | 0,25 g |
| - CEMULSOL NP 4 - RHONE POULENC (nonylphénol oxyéthylèné à 4 moles O.E.) | | 12 g |
| - CEMULSOL NP 9 - RHONE POULENC (nonylphénol oxyéthylèné à 9 moles O.E.) | | 15 g |
| - Alcool oléique polyglycérolé à 2 moles de glycérol | | 1,5 g |
| - Alcool oléique polyglycérolé à 4 moles de glycérol | | 1,5 g |
| - Propylène glycol | | 6 g |
| - TRILON B (acide éthylènediamine tétracétique) | | 0,12 g |
| - Ammoniaque à 22°Bé | | 11 g |
| - Acide thioglycolique | | 0,6 g |
| - Eau | qsp | 100 g |
| - pH : 10,4 | | |

Au moment de l'emploi, on ajoute 100 g d'eau oxygénée à 20 volumes. Le mélange, appliqué 25 minutes à 35°C sur des cheveux naturellement blancs à 90%, leur confère après shampooing et rinçage une coloration pourpre gris foncé.

Exemple de teinture 2

On prépare le mélange tinctorial suivant :

| | | |
|---|---|---|
| - Dichlorhydrate de 4-(β-hydroxyéthyl)amino-2-amino-1,3-diméthoxybenzène | | 0,71 g |
| - Dichlorhydrate de p-toluylènediamine | | 0,49 g |
| - ALFOL C 16/18 - Société CONDEA (alcool cétylstéarylique) | | 19 g |
| - EUTANOL G - Société HENKEL (2-octyldodécanol) | | 4,5 g |
| - MERGITAL C.S. - Société HENKEL (alcool cétylstéarylique à 15 moles O.E.) | | 2,5 g |
| - Lauryl sulfate d'ammonium | | 10 g |
| - Polymère cationique présentant le motif récurrent suivant : 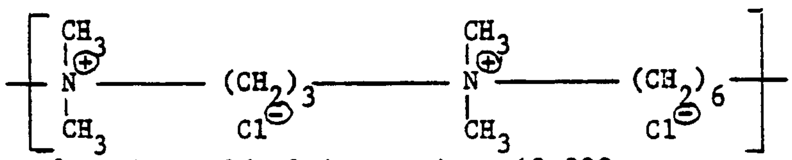 de poids moléculaire environ 10 000 | | 4 g |
| - Alcool benzylique | | 2 g |
| - Ammoniaque à 22°Bé | | 11 ml |
| - TRILON B (acide éthylène diamine tétracétique) | | 1 g |
| - Bisulfite de sodium à 35°Bé | | 1,2 g |
| - Eau | qsp | 100 g |
| - pH : 9,2 | | |

Au moment de l'emploi, on ajoute 100 g d'eau oxygénée à 20 volumes. Le mélange, appliqué 25 minutes à 35° C sur des cheveux décolorés, leur confère après shampooing et rinçage une coloration violet foncé.

Exemple de teinture 3

On prépare le mélange tinctorial suivant :

| | |
|---|---|
| - Dichlorhydrate de 4-amino-2-(β-hydroxyéthyl)-amino-1,3-diméthoxybenzène | 0,71 g |
| - p-phénylènediamine | 0,27 g |
| - Alcool oléique polyglycérolé à 2 moles de glycérol | 4,5 g |
| - Alcool oléique polyglycérolé à 4 moles de glycérol | 4,5 g |
| - ETHOMEEN O 12 - Société ARMOON HESS CHEMICAL Ltd (oléylamine oxyéthylénée à 12 moles d'O.E.) | 4,5 g |
| - COMPERLAN KD - Société HENKEL (diéthanolamide de coprah) | 9 g |
| - Propylène glycol | 4 g |
| - 2-butoxyéthanol | 8 g |
| - Ethanol à 96° | 6 g |
| - MASQUOL DTPA - Société PROTEX (sel pentasodique de l'acide diéthylène triamine pentacétique) | 2 g |
| - Hydroquinone | 0,15 g |
| - Solution de bisulfite de sodium à 35°Bé | 1,3 g |
| - Ammoniaque à 22°Bé | 10 g |
| - Eau qsp | 100 g |
| - pH : 10 | |

Au moment de l'emploi, on ajoute 100 g d'eau oxygénée à 20 volumes. Le mélange, appliqué 20 minutes à 35°C sur des cheveux naturellement blancs à 90%, leur confère après shampooing et rinçage une coloration bleu pourpre grisâtre.

Exemple de teinture 4

On prépare le mélange tinctorial suivant :

| | |
|---|---|
| - Dichlorhydrate de 4-méthylamino-2-amino-1,3-diméthoxybenzène | 0,64 g |
| - p-phénylènediamine | 0,27 g |
| - ALFOL C 16/18 - Société CONDEA (alcool cétylstéarylique) | 19 g |
| - EUTANOL G - Société HENKEL (2-octyldodécanol) | 4,5 g |
| - MERGITAL C.S. - Société HENKEL (alcool cétylstéarylique à 15 moles O.E.) | 2,5 g |
| - Lauryl sulfate d'ammonium | 10 g |
| - Polymère cationique présentant le motif récurrent suivant : 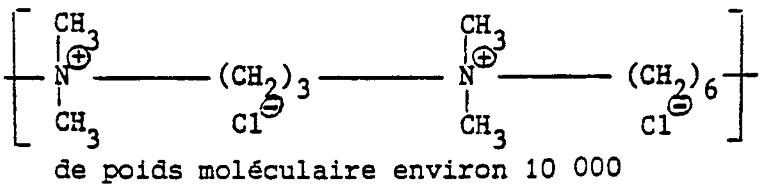 de poids moléculaire environ 10 000 | 4 g |
| - Alcool benzylique | 2 g |
| - Ammoniaque à 22°Bé | 11 g |
| - TRILON B (acide éthylène diamine tétracétique) | 1 g |
| - Bisulfite de sodium à 35°Bé | 1,2 g |
| - Eau qsp | 100 g |
| - pH : 9,3 | |

Au moment de l'emploi, on ajoute 100 g d'eau oxygénée à 20 volumes. Le mélange, appliqué 20 minutes à 35 °C sur des cheveux naturellement blancs à 90%, leur confère après shampooing et rinçage une coloration pourpre rouge foncé.

Exemple de teinture 5

On prépare le mélange tinctorial suivant :

| | | |
|---|---|---|
| - dichlorhydrate de 2,4-di(β-hydroxyéthyl)amino-1,3-diméthoxybenzène | | 0,82 g |
| - p-phénylènediamine | | 0,27 g |
| - CARBOPOL 934 - Société GOODRICH CHEMICALS | | 3 g |
| - Alcool à 96° | | 11 g |
| - 2-butoxyéthanol | | 5 g |
| - Bromure de triméthylcétyl ammonium | | 2 g |
| - TRILON B (acide éthylènediamine tétracétique) | | 0,2 g |
| - Ammoniaque à 22°Bé | | 10 g |
| - Bisulfite de sodium à 35°Bé | | 1 g |
| - Eau | qsp | 100 g |
| - pH : 9,5 | | |

Au moment de l'emploi, on ajoute 100 g d'eau oxygénée à 20 volumes. Le mélange, appliqué 20 minutes à 35° C sur des cheveux naturellement blancs à 90%, leur confère après shampooing et rinçage une coloration pourpre foncé légèrement gris.

Exemple de teinture 6

On prépare le mélange tinctorial suivant :

| | | |
|---|---|---|
| - Dichlorhydrate de 4-amino-2-(β-hydroxyéthyl)-amino-1,3-diméthoxybenzène | | 0,71 g |
| - p-aminophénol | | 0,27 g |
| - Alcool oléique polyglycérolé à 2 moles de glycérol | | 4,5 g |
| - Alcool oléique polyglycérolé à 4 moles de glycérol | | 4,5 g |
| - ETHOMEEN O 12 - Société ARMOON HESS CHEMICAL Ltd (oléylamine oxyéthylénée à 12 moles d'O.E.) | | 4,5 g |
| - COMPERLAN KD - Société HENKEL (diéthanolamide de coprah) | | 4 g |
| - Propylène glycol | | 4 g |
| - 2-butoxy-éthanol | | 8 g |
| - Ethanol à 96° | | 6 g |
| - MASQUOL DTPA - Société PROTEX (sel pentasodique de l'acide diéthylène triamine pentacétique) | | 2 g |
| - Hydroquinone | | 0,15 g |
| - Solution de bisulfite de sodium à 35°Bé | | 1,3 g |
| - Ammoniaque à 22°Bé | | 10 g |
| - Eau | qsp | 100 g |
| - pH :9,9 | | |

Au moment de l'emploi, on ajoute 100 g d'eau oxygénée à 20 volumes. Le mélange, appliqué 20 minutes à 35° C sur des cheveux décolorés, leur confère après shampooing et rinçage une coloration rouge grisâtre.

Exemple de teinture 7

On prépare le mélange tinctorial suivant :

| | |
|---|---|
| - Dichlorhydrate de 4-(β-hydroxyéthyl)amino-2-amino-1,3-diméthoxybenzène | 0,71 g |
| - p-aminophénol | 0,27 g |
| - Alcool oléique polyglycérolé à 2 moles de glycérol | 4,5 g |
| - Alcool oléique polyglycérolé à 4 moles de glycérol | 4,5 g |
| - ETHOMEEN O 12 - Société ARMOON HESS CHEMICAL Ltd (oléylamine oxyéthylénée à 12 moles d'O.E.) | 4,5 g |
| - COMPERLAN KD - Société HENKEL (diéthanolamide de coprah) | 9 g |
| - Propylène glycol | 4 g |
| - 2-butoxyéthanol | 8 g |
| - Ethanol à 96° | 6 g |
| - MASQUOL DTPA - Société PROTEX (sel pentasodique diéthylène triamine pentacétique) | 2 g |
| - Hydroquinone | 0,15 g |
| - Solution de bisulfite de sodium à 35°Bé | 1,3 g |
| - Ammoniaque à 22°Bé | 10 g |
| - Eau qsp | 100 g |
| - pH : 9,9 | |

Au moment de l'emploi, on ajoute 100 g d'eau oxygénée à 20 volumes. Le mélange, appliqué 20 minutes à 35° C sur des cheveux naturellement blancs à 90%, leur confère après shampooing et rinçage une coloration brun rouge clair légèrement gris.

Exemple de teinture 8

On prépare le mélange tinctorial suivant :

| | |
|---|---|
| - Dichlorhydrate de 4-(β-hydroxyéthyl)amino-2-amino-1,3-diméthoxybenzène | 0,12 g |
| - p-phénylènediamine | 0,08 g |
| - p-aminophénol | 0,16 g |
| - métaaminophénol | 0,13 g |
| - Résorcine | 0,13 g |
| - 2-méthyl-5-N(β-hydroxyéthyl)aminophénol | 0,1 g |
| - CARBOPOL 934 - Société GOODRICH CHEMICALS | 3 g |
| - Alcool à 96° | 11 g |
| - 2-butoxyéthanol | 5 g |
| - Bromure de triméthyl cétyl ammonium | 2 g |
| - TRILON B (acide éthylène diamine tétracétique) | 0,2 g |
| - Ammoniaque à 22°Bé | 10 g |
| - Bisulfite de sodium à 35°Bé | 1 g |
| - Eau qsp | 100 g |
| - pH : 9,5 | |

Au moment de l'emploi, on ajoute 100 g d'eau oxygénée à 20 volumes. Le mélange, appliqué 20 minutes à 35° C sur des cheveux naturellement blancs à 90%, leur confère après shampooing et rinçage une coloration brun rouge légèrement gris.

<u>Exemple de teinture 9</u>

On prépare le mélange tinctorial suivant :

| | |
|---|---|
| - Dichlorhydrate de 4-méthylamino-2-amino-1,3-diméthoxybenzène | 0,64 g |
| - Dichlorhydrate d'isopropyl-p-phénylènediamine | 0,56 g |
| - CARBOPOL 934 - Société GOODRICH CHEMICALS | 3 g |
| - Alcool à 96° | 11 g |
| - 2-butoxyéthanol | 5 g |
| - Bromure de triméthylcétyl ammonium | 2 g |
| - TRILON B (acide éthylène diamine tétracétique) | 0,2 g |
| - Bisulfite de sodium à 35°Bé | 1 g |
| - Ammoniaque à 22° Bé | 10 g |
| - Eau qsp | 100 g |
| - pH : 9,3 | |

Au moment de l'emploi, on ajoute 100 g d'eau oxygénée à 20 volumes. Le mélange, appliqué 25 minutes à 35° C sur des cheveux naturellement blancs à 90%, leur confère après shampooing et rinçage une coloration rouge myrtille.

Exemple de teinture 10

On prépare le mélange tinctorial suivant :

| | | |
|---|---|---|
| - Dichlorhydrate de 4-amino-2-(β-hydroxyéthyl)-amino-1,3-diméthoxybenzène | | 1,42 g |
| - 1,4-diamino-2,6-diméthylbenzène | | 1,05 g |
| - ALFOL 16/18 - Société CONDEA (alcool cétylstéarylique) | | 8 g |
| - CIRE DE LANETTE E - Société HENKEL (sulfate cétylstéarylique de sodium) | | 0,5 g |
| - CEMULSOL B - RHONE POULENC (huile de ricin éthoxylée) | | 1 g |
| - Diéthanolamide oléique | | 1,5 g |
| - MASQUOL DTPA - Société PROTEX (sel pentasodique de l'acide diéthylènetriamine pentacétique) | | 2,5 g |
| - Ammoniaque à 22°Bé | | 11 g |
| - Solution de bisulfite de sodium à 35° Bé | | 1 g |
| - Eau | qsp | 100 g |
| - pH : 10 | | |

Au moment de l'emploi, on ajoute 100 g d'eau oxygénée à 20 volumes. Le mélange, appliqué 25 minutes à 35°C sur des cheveux naturellement blancs à 90%, leur confère après shampooing et rinçage une coloration bleu marine.

Exemple de teinture 11

On prépare le mélange tinctorial suivant :

| | |
|---|---|
| - Dichlorhydrate de 2,4-diamino-1,3-di(γ-hydroxypropoxy)-benzène | 1 g |
| - Dichlorhydrate de 4-amino N,β-méthoxyéthylaniline | 0,71 g |
| - CEMULSOL NP 4 - RHONE POULENC (nonyl phénol oxyéthyléné à 4 moles O.E.) | 12 g |
| - CEMULSOL NP 9 - RHONE POULENC (nonyl phénol oxyéthyléné à 9 moles O.E.) | 15 g |
| - Alcool oléique polyglycérolé à 2 moles de glycérol | 1,5 g |
| - Alcool oléique polyglycérolé à 4 moles de glycérol | 1,5 g |
| - Propylène glycol | 6 g |
| - TRILON B (acide éthylène diamine tétracétique) | 0,12 g |
| - Ammoniaque 22°Bé | 11 g |
| - Eau qsp | 100 g |
| - pH 10 | |

Au moment de l'emploi, on ajoute 90 g d'eau oxygénée à 20 volumes. Le mélange, appliqué 25 minutes à 35°C sur des cheveux naturellement blancs à 90% leur confère, après shampooing et rinçage, une coloration bleu Tamise.

<u>Exemple de teinture 12</u>

On prépare le mélange tinctorial suivant :

| | |
|---|---|
| - Dichlorhydrate de 2,4-diamino-1,3-diéthoxybenzène | 0,75 g |
| - Dichlorhydrate de N,N-di-(β-hydroxyéthyl)paraphénylènediamine | 0,75 g |
| - ALFOL C 16/18 - Société CONDEA (alcool cétylstéarylique) | 8 g |
| - CIRE DE LANETTE E - Société HENKEL (sulfate cétylstéarylique de sodium) | 0,5 g |
| - CEMULSOL B - RHONE POULENC (huile de ricin éthoxylée) | 1 g |
| - Diéthanolamide oléique | 1,5 g |
| - MASQUOL DTPA - Société PROTEX (sel pentasodique de l'acide diéthylène triamine pentacétique) | 2,5 g |
| - Ammoniaque 22°Be | 11 g |
| - Eau qsp | 100 g |
| - pH 10,2 | |

Au moment de l'emploi, on ajoute 100 g d'eau oxygénée à 20 volumes. Le mélange, appliqué 10 minutes à 35°C sur des cheveux décolorées, leur confère, après shampooing et rinçage, une coloration bleu foncé.

## Revendications

## Revendications pour les Etats contractants suivants : AT, BE, CH, DE, FR,GB, GR, IT, LI, NL, SE

1. Composés de formule (I)

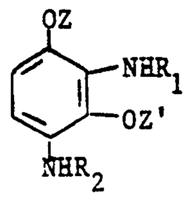

(I)

dans laquelle :
$R_1$ et $R_2$ représentent indépendamment l'un de l'autre un atome d'hydrogène, un radical alkyle ayant de 1 à 4 atomes de carbone ou un radical mono- ou polyhydroxyalkyle ayant 2 ou 3 atomes de carbone,
Z et Z′ représentent indépendamment l'un de l'autre un radical alkyle ayant de l à 4 atomes de carbone ou hydroxyalkyle ayant de 2 à 4 atomes de carbone, sous réserve que lorsque $R_1$ et $R_2$ désignent simultanément un atome d'hydrogène, Z et Z′ ne désignent pas simultanément le radical méthyle, et leurs sels d'addition avec un acide.

2. Composés selon la revendication 1, caractérisés par le fait qu'ils sont choisis parmi le 4-(β-hydroxyéthyl)amino-2-amino-1,3-diméthoxybenzène, le 4-amino-2-(β-hydroxyéthyl)amino-1,3-diméthoxybenzène,le 4-méthylamino-2-amino-1,3-diméthoxybenzène, le 2,4-di(β-hydroxyéthyl)amino-1,3-diméthoxybenzène, le 2,4-diamino-1,3-diéthoxybenzène, le 2,4-diamino-1,3-di(γ-hydroxypropoxy)-benzène et leurs sels d'addition avec un acide.

3. Utilisation à titre de coupleur d'un composé de formule (I) selon la revendication 1 ou 2 ou de l'un de ses sels d'addition avec un acide, en association avec des précurseurs de colorants d'oxydation, pour la teinture des fibres kératiniques, en particulier des cheveux humains.

4. Composition tinctoriale capillaire caractérisée par le fait qu'elle contient, dans un support aqueux cosmétiquement acceptable, au moins un composé de formule (I) selon la revendication 1 ou 2 ou l'un de ses sels d'acides, à titre de coupleur, en association avec au moins un précurseur de colorant d'oxydation de type para.

5. Composition tinctoriale selon la revendication 4, caractérisée par le fait qu'elle contient 0,05 à 3,5% en poids de composé (I) ou de l'un de ses sels d'addition avec un acide, sur la base du poids total de la composition.

6. Composition tinctoriale selon la revendication 4, caractérisée par le fait que le précurseur de colorant d'oxydation de type para est choisi parmi les paraphénylène diamines, les paraaminophénols, les composés para hétérocycliques ou leurs mélanges.

7. Composition tinctoriale selon la revendication 6, caractérisée par le fait que les paraphénylènediamines répondent à la formule :

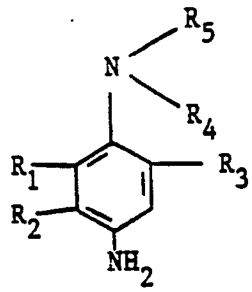

(XII)

dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ forment conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène, ou sont les sels des composés de formule (XII) ci-dessus.

**8.** Composition tinctoriale selon la revendication 7, caractérisée par le fait qu'elle contient au moins une paraphénylènediamine choisie parmi la p-phénylènediamine, la p-toluylènediamine, la méthoxyparaphénylènediamine, la chloroparaphénylènediamine, la 2,6-diméthyl-p-phénylènediamine, la 2,5-diméthylparaphénylènediamine, la 2-méthyl-5-méthoxy-paraphénylènediamine, la 2,6-diméthyl-5-méthoxy-paraphénylènediamine, la N,N-diméthylparaphénylènediamine, la 3-méthyl-4-amino-N,N-diéthylaniline, la N,N-di(β-hydroxyéthyl)paraphénylènediamine, la 3-méthyl-4-amino-N,N-di-(β-hydroxyéthyl)aniline, la 3-chloro-4-amino-N,N-di-(β-hydroxyéthyl)aniline, la 4-amino-N,N-(éthyl, carbamylméthyl)aniline, la 3-méthyl-4-amino-N,N-(éthyl, carbamylméthyl)aniline, la 4-amino-N,N-(éthyl, β-pipéridinoéthyl)aniline, la 3-méthyl-4-amino-N,N-(éthyl, β-pipéridinoéthyl)aniline, la 4-amino-N,N-(éthyl, β-morpholinoéthyl)aniline, la 3-méthyl-4-amino-N,N-(éthyl,β-morpholinoéthyl)aniline, la 4-amino-N,N-(éthyl, β-acétylaminoéthyl)-aniline, la 4-amino-N, β-méthoxyéthylaniline, la 3-méthyl-4-amino-N,N-(éthyl, β-acétylaminoéthyl) aniline, la 4-amino-N,N-(éthyl, β-mésylaminoéthyl)aniline, la 3-méthyl-4-amino-N,N-(éthyl, β-mésylaminoéthyl)aniline, la 4-amino- N,N-(éthyl, β-sulfoéthyl)aniline, la 3-méthyl-4-amino-N,N-(éthyl, β-sulfoéthyl)-aniline, la N-[(4′-amino)phényl]morpholine, la N-[(4′-amino)phényl] pipéridine, la 2,3-diméthyl-p-phénylènediamine, l'isopropyl-p-phénylènediamine, sous forme de base libre ou sous forme de sel cosmétiquement acceptable.

**9.** Composition tinctoriale selon la revendication 6, caractérisée par le fait qu'elle contient au moins un p-aminophénol choisi parmi le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

**10.** Composition tinctoriale selon la revendication 6, caractérisée par le fait que le précurseur de colorant d'oxydation de type para est un composé para hétérocyclique choisi parmi la 2,5-diamino- pyridine, la 2-hydroxy-5-amino-pyridine et la tétraaminopyrimidine.

**11.** Composition tinctoriale selon l'une quelconque des revendications 4 à 10, caractérisée par le fait qu'elle contient d'autres coupleurs choisis parmi les métadiphénols, les métaaminophénols, les métaphénylènediamines, les métaacylaminophénols, les métauréidophénols, les métacarbalcoxyaminophénols, l'α-naphtol, les composés β-cétoniques et les pyrazolones.

**12.** Composition tinctoriale selon l'une quelconque des revendications 4 à 11, caractérisée par le fait qu'elle contient 0,1 à 7% en poids d'un ou plusieurs précurseurs de colorants d'oxydation de type para et coupleurs sur la base du poids total de la composition.

**13.** Composition tinctoriale selon l'une quelconque des revendications 4 à 12, caractérisée par le fait qu'elle contient en outre des précurseurs de colorant de type ortho choisis parmi les orthoaminophénols, les orthophénylènediamines et les orthodiphénols.

**14.** Composition tinctoriale selon l'une quelconque des revendications 4 à 13, caractérisée par le fait qu'elle contient en outre des colorants directs choisis parmi les colorants azoïques, anthraquinoniques et les dérivés nitrés de la série benzénique.

**15.** Composition tinctoriale selon l'une quelconque des revendications 4 à 14, caractérisée par le fait qu'elle a un pH compris entre 8 et 11, et de préférence compris entre 9 et 11.

**16.** Composition tinctoriale selon l'une quelconque des revendications 4 à 15, caractérisée par le fait qu'elle contient 1 à 40% en poids d'un solvant organique choisi parmi les alcanols inférieurs, le glycérol, les glycols ou éthers de glycols, et leurs mélanges.

**17.** Composition tinctoriale selon l'une quelconque des revendications 4 à 16, caractérisée par le fait qu'elle contient en outre 0,5 à 40% en poids, d'au moins un agent tensio-actif anionique, cationique, non ionique, amphotère ou leurs mélanges.

**18.** Composition tinctoriale selon l'une quelconque des revendications 4 à 17, caractérisée par le fait qu'elle contient en outre des adjuvants cosmétiques choisis parmi les épaississants, les agents anti-oxydants, les agents de pénétration, les agents séquestrants, les tampons, les parfums, les agents alcalinisants, les propulseurs.

**19.** Procédé de teinture capillaire mettant en oeuvre la révélation par un oxydant, caractérisé par le fait qu'il consiste à mélanger au moment de l'emploi la composition tinctoriale selon l'une quelconque des revendications 4 à 18 avec une solution oxydante en une quantité suffisante, puis à appliquer le mélange obtenu sur les cheveux, à le laisser poser pendant 10 à 40 minutes, de préférence 15 à 30 minutes, puis à rincer les cheveux, à les laver au shampooing, à les rincer à l'eau et à les sécher.

**20.** Procédé de teinture capillaire mettant en oeuvre la révélation par un oxydant, caractérisé par le fait qu'il consiste dans un premier temps à appliquer sur les cheveux une composition tinctoriale contenant au moins un précurseur de colorant d'oxydation de type para tel que défini dans l'une quelconque des revendications 6 à 10 puis, dans un deuxième temps, à appliquer une composition tinctoriale contenant un composé de formule (I) ou l'un de ses sels d'addition avec un acide, l'agent oxydant étant présent dans la composition appliquée dans le deuxième temps ou bien appliqué sur les cheveux eux-mêmes dans un troisième temps, à laisser poser pendant 10 à 40 minutes, de préférence 15 à 30 minutes, puis à rincer les cheveux, à les laver au shampooing, à les rincer à nouveau et à les sécher.

**21.** Procédé de préparation d'un composé de formule (I) selon la revendication 1 dans lequel $R_1$ et $R_2$ désignent un atome d'hydrogène et Z et Z' ne désignent pas simultanément le radical méthyle, caractérisé par le fait qu'il consiste à procéder à la réduction et à la déshalogénation consécutives ou simultanées du 3,5-dinitrochlorobenzène 2,4-disubstitué de formule (II) suivante :

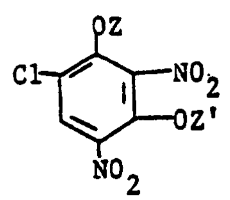

(II)

dans laquelle Z et Z' ont les significations indiquées dans la revendication 1.

**22.** Procédé selon la revendication 21, caractérisé par le fait qu'il consiste,

a) dans une première étape, à réduire le composé (II) par le fer en présence d'acide acétique à une température comprise entre 50 et 100 °C pour obtenir le 3,5-diaminochlorobenzène 2,4-disubstitué de formule (IIIA) :

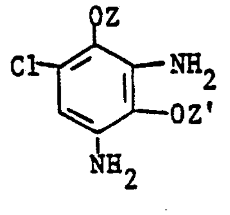

(IIIA)

dans laquelle Z et Z' ont les significations indiquées dans la revendication 1, puis

b) dans une seconde étape à déshalogéner le composé (IIIA) dans un milieu solvant constitué par l'eau, un alcool inférieur ou un mélange hydroalcoolique en présence de palladium sur charbon,

d'acétate d'ammonium et de formiate de triéthylamine à une température comprise entre 50° C et 100° C.

23. Procédé selon la revendication 21, caractérisé par le fait qu'il consiste à réduire et déshalogéner simultanément le composé (II) sous pression d'hydrogène en présence de palladium sur charbon dans un solvant choisi parmi l'eau, les alcools inférieurs ou les mélanges hydroalcooliques à une température comprise entre 50° C et 200° C, de préférence en présence d'acétate d'ammonium ou de triéthylamine.

24. Procédé de préparation d'un composé de formule (I) selon la revendication 1 dans laquelle $R_1$ et $R_2$ sont différents d'un atome d'hydrogène et ont des significations identiques à partir d'un 3,5-dinitrochlorobenzène 2,4-disubstitué, caractérisé par le fait qu'il consiste :

a) dans une première étape, à réduire le 3,5-dinitrochlorobenzène 2,4-disubstitué de formule (II) suivante :

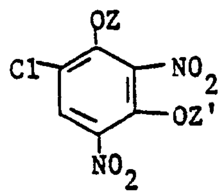

(II)

dans laquelle Z et Z′ ont les mêmes significations que dans la revendication 1 mais peuvent désigner simultanément le radical méthyle, par le fer en présence d'acide acétique à une température comprise entre 50 et 100° C, pour obtenir le 3,5-diaminochlorobenzène 2,4-disubstitué de formule (IIIA) suivante :

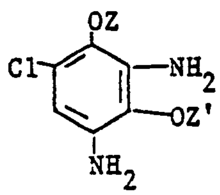

(IIIA)

Z et Z′ ayant les significations indiquées ci-dessus;

b) dans une seconde étape, à procéder à la déshalogénation du composé de formule (IIIA) en présence de palladium sur charbon, d'acétate d'ammonium et de formiate de triéthylamine, à une température comprise entre 50° C et 100° C, dans un milieu solvant constitué par de l'eau, un alcool inférieur ou un mélange hydroalcoolique pour obtenir le 2,4-diamino-benzène 1,3-disubstitué de formule (IA) suivante :

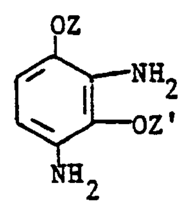

(IA)

Z et Z′ ayant les significations indiquées dans la revendication 1 et pouvant désigner simultanément un radical méthyle;

c) dans une troisième étape, à procéder à l'alkylation ou l'hydroxyalkylation du 2,4-diamino-benzène 1,3-disubstitué de formule (IA) selon les méthodes classiques utilisées pour les amines aromatiques; les seconde et troisième étapes pouvant être inversées.

25. Procédé de préparation d'un composé de formule (I) selon la revendication 1, dans laquelle $R_2$ est différent d'un atome d'hydrogène et $R_1$ = H à partir d'un 3,5-dinitrochlorobenzène 2,4-disubstitué de

formule (II) indiqué dans la revendication 25, caractérisé par le fait qu'il consiste :

a) dans une première étape, à procéder à une réduction ménagée du composé de formule (II) par transfert d'hydrogène en présence d'un catalyseur tel que le palladium sur charbon et de cyclohexène comme donneur d'hydrogène pour obtenir le 5-amino-3-nitrochlorobenzène 2,4-disubstitué de formule (IV) ci-après :

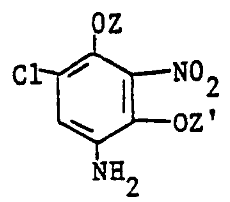

(IV)

dans laquelle Z et Z′ ont les significations indiquées dans la revendication 1 mais peuvent désigner simultanément le radical méthyle;

b) dans une seconde étape à soumettre le composé de formule (IV) à une alkylation ou une hydroxyalkylation du groupement amino pour obtenir le composé de formule (V) suivante :

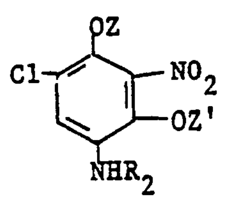

(V)

dans laquelle Z, Z′ et $R_2$ ont les signfications indiquées dans la revendication 1, Z et Z′ pouvant désigner simultanément le radical méthyle;

c) dans une troisième étape, à procéder à la réduction du composé de formule (V) par le fer en présence d'acide acétique, à une température comprise entre 20 et 100°C, pour obtenir le composé de formule ($III_B$) suivante :

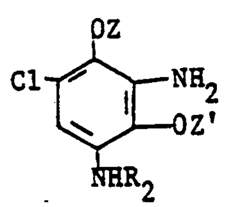

($III_B$)

dans laquelle Z, Z′ et $R_2$ ont les significations indiquées ci-dessus;

d) dans une quatrième étape, à procéder à la déshalogénation du composé de formule ($III_B$) selon le procédé indiqué dans la revendication 24 pour obtenir le composé de formule ($I_B$) suivante :

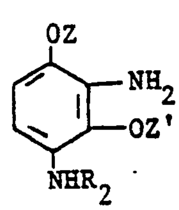

($I_B$)

dans laquelle Z, Z′ et $R_2$ ont les significations indiquées ci-dessus.

**26.** Procédé de préparation d'un composé de formule (I) selon la revendication 1 dans laquelle $R_2$ est un atome d'hydrogène et $R_1$ est différent d'un atome d'hydrogène à partir d'un 3,5-dinitrochlorobenzène 2,4-disubstitué de formule (II) indiqué dans la revendication 24, caractérisé par le fait qu'il consiste :

a) dans une première étape à procéder à la réduction ménagée du composé de formule (II) selon la méthode indiquée dans la revendication 25 pour obtenir le 5-amino-3-nitrochlorobenzène 2,4-disubstitué de formule (IV);
b) dans une seconde étape, à acétyler le composé de formule (IV) par l'anhydride acétique à une température comprise entre 20 et 100 °C pour obtenir le composé de formule (VI) suivante :

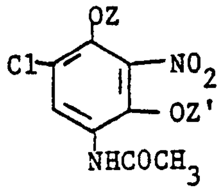

(VI)

dans laquelle Z et Z′ ont les significations indiquées dans la revendication 1 et peuvent désigner simultanément le radical méthyle;
c) dans une troisième étape, à procéder à la réduction et à la déshalogénation simultanées du composé de formule (VI) sous pression d'hydrogène en utilisant du palladium sur charbon en présence d'acétate d'ammonium ou de triéthylamine dans un solvant constitué par de l'eau, un alcool inférieur ou un mélange hydroalcoolique, à une température comprise entre 50 °C et 200 °C, pour obtenir le 4-acétamido-2-amino-benzène 1,3-disubstitué de formule (IX) suivante :

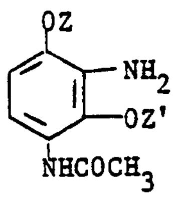

(IX) ;

d) dans une quatrième étape, à procéder à l'alkylation ou à l'hydroxyalkylation du groupement amino du composé de formule (IX) selon des méthodes classiques pour les amines aromatiques, pour obtenir le composé de formule (X) suivante :

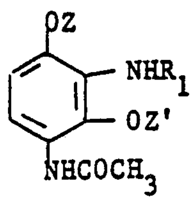

(X) ;

e) dans une cinquième étape, à désacétyler le composé de formule (X) pour obtenir le composé final de formule ($I_C$) suivante :

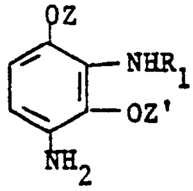

($I_C$)

dans laquelle $R_1$, Z et Z′ ont les significations indiquées dans la revendication 1, $R_1$ étant différent d'un atome d'hydrogène et Z et Z′ pouvant désigner simultanément le radical méthyle.

**27.** Procédé de préparation d'un composé de formule ($I_C$) selon la revendication 26, dans laquelle $R_2$ est un atome d'hydrogène et $R_1$ est différent d'un atome d'hydrogène, caractérisé par le fait que la troisième étape de réduction et déshalogénation simultanées du 5-acétamido-3-nitrochlorobenzène 2,4-

disubstitué de formule (VI) est remplacée par une étape de réduction simple par le fer en présence d'acide acétique à une température comprise entre 50 °C et 100 °C conduisant à la formation du 5-acétamido-3-aminochlorobenzène 2,4-disubstitué de formule (VII) suivante :

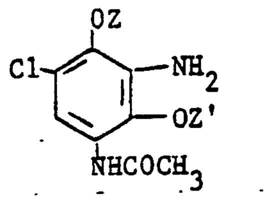

(VII)

,

dans laquelle Z et Z′ ont les significations indiquées dans la revendication 1 et pouvant désigner simultanément le radical méthyle, cette étape de réduction étant suivie d'une étape d'alkylation ou d'hydroxyalkylation du composé de formule (VII) pour obtenir le composé de formule (VIII) suivante :

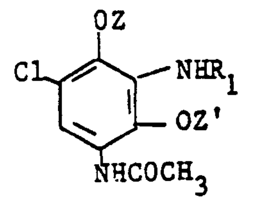

(VIII)

puis d'une étape de désacétylation du composé de formule (VIII) pour aboutir au composé de formule ($III_C$) suivante :

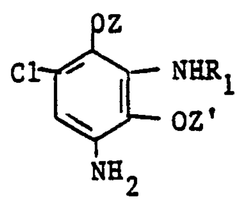

($III_C$)

qui est ensuite déshalogéné en présence de palladium sur charbon, d'acétate d'ammonium et de formiate de triéthylamine dans un solvant constitué par de l'eau, un alcool inférieur ou un mélange hydroalcoolique, à une température comprise entre 50 °C et 100 °C pour aboutir au composé de formule ($I_C$) indiquée dans la revendication 26 dans laquelle $R_1$, Z et Z′ ont les significations indiquées dans la revendication 1, Z et Z′ pouvant désigner simultanément le radical méthyle et $R_1$ étant différent d'un atome d'hydrogène.

**28.** Composé de formule :

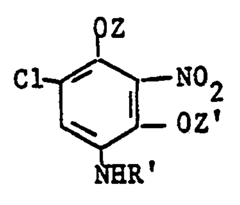

(XI)

dans laquelle R′ désigne un atome d'hydrogène, un radical alkyle ayant de 1 à 4 atomes de carbone, un radical mono- ou polyhydroxyalkyle ayant 2 ou 3 atomes de carbone, acétyle ou β-chloroéthoxycarbonyle et Z et Z′ représentent indépendamment l'un de l'autre un radical alkyle ayant de 1 à 4 atomes de carbone ou hydroxyalkyle ayant de 2 à 4 atomes de carbone.

**29.** Composé selon la revendication 28, caractérisé par le fait qu'il est choisi parmi le 5-amino-2,4-diméthoxy-3-nitrochlorobenzène, le 5-acétamido-2,4-diméthoxy-3-nitrochlorobenzène, le 5-méthylamino-2,4-diméthoxy-3-nitrochlorobenzène et le 5-($\beta$-chloréthoxycarbonyl)amino-2,4-diméthoxy-3-nitrochlorobenzène.

**Revendications pour l'Etat contractant suivant : ES**

1. Utilisation à titre de coupleur d'un composé de formule (I)

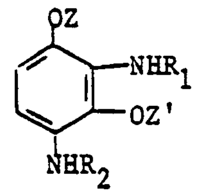

(I)

dans laquelle :
$R_1$ et $R_2$ représentent indépendamment l'un de l'autre un atome d'hydrogène, un radical alkyle ayant de 1 à 4 atomes de carbone ou un radical mono-ou polyhydroxyalkyle ayant 2 ou 3 atomes de carbone,
Z et Z' représentent indépendamment l'un de l'autre un radical alkyle ayant I à 4 atomes de carbone ou hydroxyalkyle ayant de 2 à 4 atomes de carbone, sous réserve que lorsque $R_1$ et $R_2$ désignent simultanément un atome d'hydrogène, Z et Z' ne désignent pas simultanément le radical méthyle,
ou de l'un de ses sels d'addition avec un acide, en association avec des précurseurs de colorants d'oxydation, pour la teinture des fibres kératiniques, en particulier des cheveux humains.

2. Composition tinctoriale capillaire caractérisée par le fait qu'elle contient, dans un support aqueux cosmétiquement acceptable, au moins un composé de formule (I) selon la revendication 1 ou l'un de ses sels d'acides, à titre de coupleur, en association avec au moins un précurseur de colorant d'oxydation de type para.

3. Composition tinctoriale selon la revendication 2, caractérisée par le fait qu'elle contient, à titre de coupleur, au moins un composé choisi parmi le 4-($\beta$-hydroxyéthyl)amino-2-amino-1,3-diméthoxybenzène, le 4-amino-2-($\beta$-hydroxyéthyl)amino-1,3-diméthoxybenzène,le 4-méthylamino-2-amino-1,3-diméthoxybenzène, le 2,4-di($\beta$- hydroxyéthyl)amino-1,3-diméthoxybenzène, le 2,4-diamino-1,3-diéthoxybenzène, le 2,4-diamino-1,3-di($\gamma$-hydroxypropoxy)benzène et leurs sels d'addition avec un acide.

4. Composition tinctoriale selon la revendication 2 ou 3, caractérisée par le fait qu'elle contient 0,05 à 3,5% en poids de composé (I) ou de l'un de ses sels d'addition avec un acide, sur la base du poids total de la composition.

5. Composition tinctoriale selon la revendication 2, caractérisée par le fait que le précurseur de colorant d'oxydation de type para est choisi parmi les paraphénylène diamines, les paraaminophénols, les composés para hétérocycliques ou leurs mélanges.

6. Composition tinctoriale selon la revendication 5, caractérisée par le fait que les paraphénylènediamines répondent à la formule :

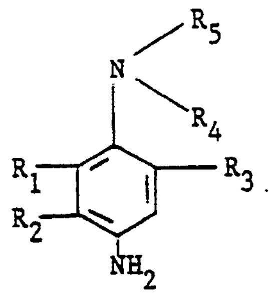

(XII)

dans laquelle $R_1$, $R_2$ et $R_3$ sont identiques ou différents et représentent un atome d'hydrogène ou d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridinoalkyle, morpholinoalkyle, les groupes alkyle ou alcoxy représentés par $R_4$ et $R_5$ ayant de 1 à 4 atomes de carbone, ou bien $R_4$ et $R_5$ forment conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène, ou sont les sels des composés de formule (XII) ci-dessus.

7. Composition tinctoriale selon la revendication 6, caractérisée par le fait qu'elle contient au moins une paraphénylènediamine choisie parmi la p-phénylènediamine, la p-toluylènediamine, la méthoxyparaphénylènediamine, la chloroparaphénylènediamine, la 2,6-diméthyl-p-phénylènediamine, la 2,5-diméthylparaphénylènediamine, la 2-méthyl-5-méthoxy-paraphénylènediamine, la 2,6-diméthyl-5-méthoxy-paraphénylènediamine, la N,N-diméthylparaphénylènediamine, la 3-méthyl-4-amino-N,N-diéthylaniline, la N,N-di(β-hydroxyéthyl)paraphénylènediamine, la 3-méthyl-4-amino-N,N-di-(β-hydroxyéthyl)aniline, la 3-chloro-4-amino-N,N-di-(β-hydroxyéthyl)aniline, la 4-amino-N,N-(éthyl, carbamylméthyl)aniline, la 3-méthyl-4-amino-N,N-(éthyl, carbamylméthyl)aniline, la 4-amino-N,N-(éthyl, β-pipéridinoéthyl)aniline, la 3-méthyl-4-amino-N,N-(éthyl, β-pipéridinoéthyl)aniline, la 4-amino-N,N-(éthyl, β-morpholinoéthyl)aniline, la 3-méthyl-4-amino-N,N-(éthyl, β-morpholinoéthyl)aniline, la 4-amino-N,N-(éthyl, β-acétylaminoéthyl)-aniline, la 4-amino-N, β-méthoxyéthylaniline, la 3-méthyl-4-amino-N,N-(éthyl, β-acétylaminoéthyl)aniline, la 4-amino- N,N-(éthyl, β-mésylaminoéthyl)aniline, la 3-méthyl-4-amino-N,N-(éthyl, β-mésylaminoéthyl)-aniline, la 4-amino-N,N-(éthyl, β-sulfoéthyl)aniline, la 3-méthyl-4-amino-N,N-(éthyl, β-sulfoéthyl)aniline, la N-[(4'-amino)phényl]morpholine, la N-[(4'-amino)phényl] pipéridine, la 2,3-diméthyl-p-phénylènediamine, l'isopropyl-p-phénylènediamine, sous forme de base libre ou sous forme de sel cosmétiquement acceptable.

8. Composition tinctoriale selon la revendication 5, caractérisée par le fait qu'elle contient au moins un p-aminophénol choisi parmi le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

9. Composition tinctoriale selon la revendication 5, caractérisée par le fait que le précurseur de colorant d'oxydation de type para est un composé para hétérocyclique choisi parmi la 2,5-diamino- pyridine, la 2-hydroxy-5-amino-pyridine et la tétraaminopyrimidine.

10. Composition tinctoriale selon l'une quelconque des revendications 2 à 9, caractérisée par le fait qu'elle contient d'autres coupleurs choisis parmi les métadiphénols, les métaaminophénols, les métaphénylènediamines, les métaacylaminophénols, les métauréidophénols, les métacarbalcoxyaminophénols, l'α-naphtol, les composés β-cétoniques et les pyrazolones.

11. Composition tinctoriale selon l'une quelconque des revendications 2 à 10, caractérisée par le fait qu'elle contient 0,1 à 7% en poids d'un ou plusieurs précurseurs de colorants d'oxydation de type para et coupleurs sur la base du poids total de la composition.

12. Composition tinctoriale selon l'une quelconque des revendications 2 à 11, caractérisée par le fait qu'elle contient en outre des précurseurs de colorant de type ortho choisis parmi les orthoaminophénols, les orthophénylènediamines et les orthodiphénols.

13. Composition tinctoriale selon l'une quelconque des revendications 2 à 12, caractérisée par le fait qu'elle contient en outre des colorants directs choisis parmi les colorants azoïques, anthraquinoniques et les dérivés nitrés de la série benzénique.

14. Composition tinctoriale selon l'une quelconque des revendications 2 à 13, caractérisée par le fait qu'elle a un pH compris entre 8 et 11, et de préférence compris entre 9 et 11.

15. Composition tinctoriale selon l'une quelconque des revendications 2 à 14, caractérisée par le fait qu'elle contient 1 à 40% en poids d'un solvant organique choisi parmi les alcanols inférieurs, le glycérol, les glycols ou éthers de glycols, et leurs mélanges.

16. Composition tinctoriale selon l'une quelconque des revendications 2 à 15, caractérisée par le fait qu'elle contient en outre 0,5 à 40% en poids, d'au moins un agent tensio-actif anionique, cationique, non ionique, amphotère ou leurs mélanges.

17. Composition tinctoriale selon l'une quelconque des revendications 2 à 16, caractérisée par le fait qu'elle contient en outre des adjuvants cosmétiques choisis parmi les épaississants, les agents anti-oxydants, les agents de pénétration, les agents séquestrants, les tampons, les parfums, les agents alcalinisants, les propulseurs.

18. Procédé de teinture capillaire mettant en oeuvre la révélation par un oxydant, caractérisé par le fait qu'il consiste à mélanger au moment de l'emploi la composition tinctoriale selon l'une quelconque des revendications 2 à 17 avec une solution oxydante en une quantité suffisante, puis à appliquer le mélange obtenu sur les cheveux, à le laisser poser pendant 10 à 40 minutes, de préférence 15 à 30 minutes, puis à rincer les cheveux, à les laver au shampooing, à les rincer à l'eau et à les sécher.

19. Procédé de teinture capillaire mettant en oeuvre la révélation par un oxydant, caractérisé par le fait qu'il consiste dans un premier temps à appliquer sur les cheveux une composition tinctoriale contenant au moins un précurseur de colorant d'oxydation de type para tel que défini dans l'une quelconque des revendications 5 à 9 puis, dans un deuxième temps, à appliquer une composition tinctoriale contenant un composé de formule (I) ou l'un de ses sels d'addition avec un acide, l'agent oxydant étant présent dans la composition appliquée dans le deuxième temps ou bien appliqué sur les cheveux eux-mêmes dans un troisième temps, à laisser poser pendant 10 à 40 minutes, de préférence 15 à 30 minutes, puis à rincer les cheveux, à les laver au shampooing, à les rincer à nouveau et à les sécher.

20. Procédé de préparation d'un composé de formule (I) selon la revendication 1 dans lequel $R_1$ et $R_2$ désignent un atome d'hydrogène et Z et Z′ ne désignent pas simultanément le radical méthyle, caractérisé par le fait qu'il consiste à procéder à la réduction et à la déshalogénation consécutives ou simultanées du 3,5-dinitrochlorobenzène 2,4-disubstitué de formule (II) suivante :

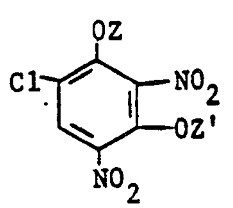

(II)

dans laquelle Z et Z′ ont les significations indiquées dans la revendication 1.

21. Procédé selon la revendication 20, caractérisé par le fait qu'il consiste,

a) dans une première étape, à réduire le composé (II) par le fer en présence d'acide acétique à une température comprise entre 50 et 100 °C pour obtenir le 3,5-diaminochlorobenzène 2,4-disubstitué de formule (IIIA) :

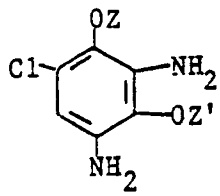

(IIIA)

dans laquelle Z et Z′ ont les significations indiquées dans la revendication 1, puis

b) dans une seconde étape à déshalogéner le composé (IIIA) dans un milieu solvant constitué par l'eau, un alcool inférieur ou un mélange hydroalcoolique en présence de palladium sur charbon, d'acétate d'ammonium et de formiate de triéthylamine à une température comprise entre 50 °C et 100 °C.

**22.** Procédé selon la revendication 20, caractérisé par le fait qu'il consiste à réduire et déshalogéner simultanément le composé (II) sous pression d'hydrogène en présence de palladium sur charbon dans un solvant choisi parmi l'eau, les alcools inférieurs ou les mélanges hydroalcooliques à une température comprise entre 50 °C et 200 °C, de préférence en présence d'acétate d'ammonium ou de triéthylamine.

**23.** Procédé de préparation d'un composé de formule (I) selon la revendication 1 dans laquelle $R_1$ et $R_2$ sont différents d'un atome d'hydrogène et ont des significations identiques, à partir d'un 3,5-dinitrochlorobenzène 2,4-disubstitué, caractérisé par le fait qu'il consiste :

a) dans une première étape, à réduire le 3,5-dinitrochlorobenzène 2,4-disubstitué de formule (II) suivante :

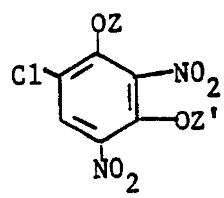

(II)

dans laquelle Z et Z′ ont les mêmes significations que dans la revendication 1 mais peuvent désigner simultanément le radical méthyle, par le fer en présence d'acide acétique à une température comprise entre 50 et 100 °C, pour obtenir le 3,5-diaminochlorobenzène 2,4-disubstitué de formule (IIIA) suivante :

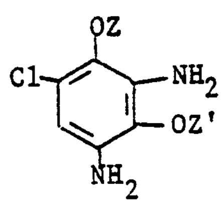

(IIIA)

Z et Z' ayant les significations indiquées ci-dessus;

b) dans une seconde étape, à procéder à la déshalogénation du composé de formule (IIIA) en présence de palladium sur charbon, d'acétate d'ammonium et de formiate de triéthylamine, à une température comprise entre 50 °C et 100 °C, dans un milieu solvant constitué par de l'eau, un alcool inférieur ou un mélange hydroalcoolique pour obtenir le 2 ,4-diamino-benzène 1 , 3-disubstitué de formule (LA) suivante

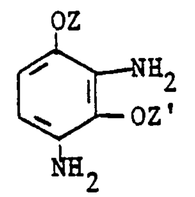

(IA)

: Z et Z' ayant les significations indiquées dans la revendication 1 et pouvant désigner simultanément un radical méthyle;

c) dans une troisième étape, à procéder à l'alkylation ou l'hydroxyalkylation du 2,4-diamino-benzène 1,3-disubstitué de formule (LA) selon les méthodes classiques utilisées pour les amines aromatiques;

les seconde et troisième étapes pouvant être inversées.

**24.** Procédé de préparation d'un composé de formule (I) selon la revendication 1, dans laquelle $R_2$ est différent d'un atome d'hydrogène et $R_1$ = H à partir d'un 3,5-dinitrochlorobenzène 2,4-disubstitué de formule (II) indiqué dans la revendication 23, caractérisé par le fait qu'il consiste :

a) dans une première étape, à procéder à une réduction ménagée du composé de formule (II) par transfert d'hydrogène en présence d'un catalyseur tel que le palladium sur charbon et de cyclohexène comme donneur d'hydrogène pour obtenir le 5-amino-3-nitrochlorobenzène 2,4-disubstitué de formule (IV) ci-après :

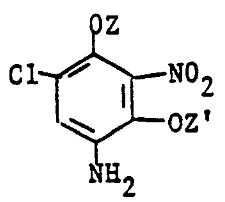

(IV)

dans laquelle Z et Z' ont les significations indiquées dans la revendication 1 mais peuvent désigner simultanément le radical méthyle;

b) dans une seconde étape à soumettre le composé de formule (IV) à une alkylation ou une hydroxyalkylation du groupement amino pour obtenir le composé de formule (V) suivante :

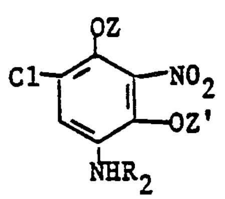

(V)

dans laquelle Z, Z' et $R_2$ ont les significations indiquées dans la revendication 1, Z et Z' pouvant désigner simultanément le radical méthyle;

c) dans une troisième étape, à procéder à la réduction du composé de formule (V) par le fer en présence d'acide acétique, à une température comprise entre 20 et 100 °C, pour obtenir le composé de formule ($III_B$ )suivante :

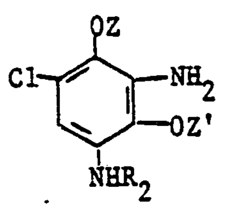

($III_B$)

dans laquelle Z, Z' et $R_2$ ont les significations indiquées ci-dessus;
d) dans une quatrième étape, à procéder à la déshalogénation du composé de formule ($III_B$) selon le procédé indiqué dans la revendication 24 pour obtenir l

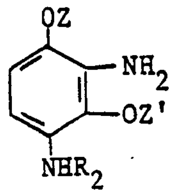

($I_B$)

e composé de formule ($I_B$ )suivante : dans laquelle Z, Z' et $R_2$ ont les significations indiquées ci-dessus.

**25.** Procédé de préparation d'un composé de formule (I) selon la revendication 1 dans laquelle $R_2$ est un atome d'hydrogène et $R_1$ est différent d'un atome d'hydrogène à partir d'un 3,5-dinitrochlorobenzène 2,4-disubstitué de formule (II) indiqué dans la revendication 24, caractérisé par le fait qu'il consiste :
a) dans une première étape à procéder à la réduction ménagée du composé de formule (II) selon la méthode indiquée dans la revendication 24 pour obtenir le 5-amino-3-nitrochlorobenzène 2,4-disubstitué de formule (IV);
b) dans une seconde étape, à acétyler le composé de formule (IV) par l'anhydride acétique à une température comprise entre 20 et 100 °C pour obtenir le composé de formule (VI) suivante :

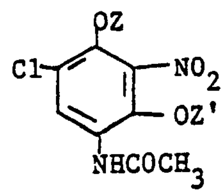

(VI)

dans laquelle Z et Z′ ont les significations indiquées dans la revendication 1 et peuvent désigner simultanément le radical méthyle;
c) dans une troisième étape, à procéder à la réduction et à la déshalogénation simultanées du composé de formule (VI) sous pression d'hydrogène en utilisant du palladium sur charbon en présence d'acétate d'ammonium ou de triéthylamine dans un solvant constitué par de l'eau, un alcool inférieur ou un mélange hydroalcoolique, à une température comprise entre 50 °C et 200 °C, pour obtenir le 4-acétamido-2-amino-benzène 1,3-disubstitué de formule (IX) suivante :

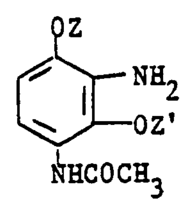

(IX)

;

d) dans une quatrième étape, à procéder à l'alkylation ou à l'hydroxyalkylation du groupement amino du composé de formule (IX) selon des méthodes classiques pour les amines aromatiques, pour obtenir le composé de formule (X) suivante :

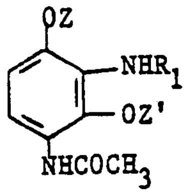

(X)

;

e) dans une cinquième étape, à désacétyler le composé de formule (X) pour obtenir le composé final de formule ($I_c$) suivante :

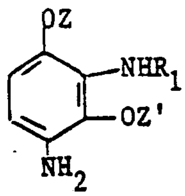

. ($I_C$)

dans laquelle $R_1$, Z et Z' ont les significations indiquées dans la revendication 1, $R_1$ étant différent d'un atome d'hydrogène et Z et Z' pouvant désigner simultanément le radical méthyle.

**26.** Procédé de préparation d'un composé de formule ($I_C$) selon la revendication 25, dans laquelle $R_2$ est un atome d'hydrogène et $R_1$ est différent d'un atome d'hydrogène, caractérisé par le fait que la troisième étape de réduction et déshalogénation simultanées du 5-acétamido-3-nitrochlorobenzène 2 ,4-disubstitué de formule (VI) est remplacée par une étape de réduction simple par le fer en présence d'acide acétique à une température comprise entre 50 °C et 100 °C conduisant à la formation du 5-acétamido-3-aminochlorobenzène 2,4-disubstitué de formule (VII) suivante :

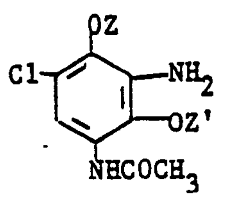

(VII)

,

dans laquelle Z et Z' ont les significations indiquées dans la revendication 1 et pouvant désigner simultanément le radical méthyle, cette étape de réduction étant suivie d'une étape d'alkylation ou d'hydroxyalkylation du composé de formule (VII) pour obtenir le composé de formule (VIII) suivante :

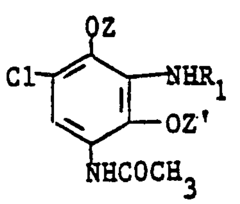

(VIII)

puis d'une étape de désacétylation du composé de formule (VIII) pour aboutir au composé de formule ($III_C$) suivante :

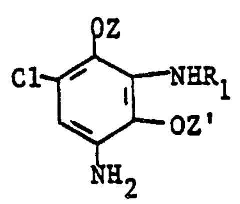

($III_C$)

qui est ensuite déshalogéné en présence de palladium sur charbon, d'acétate d'ammonium et de formiate de triéthylamine dans un solvant constitué par de l'eau, un alcool inférieur ou un mélange hydroalcoolique, à une température comprise entre 50°C et 100°C pour aboutir au composé de formule ($I_C$) indiquée dans la revendication 26 dans laquelle $R_1$, Z et Z' ont les significations indiquées dans la revendication 1, Z et Z' pouvant désigner simultanément le radical méthyle et $R_1$ étant différent d'un atome d'hydrogène.

**27.** Procédé de préparation d'une composition de teinture capillaire, caractérisé par le fait qu'il consiste à dissoudre dans un support aqueux cosmétiquement acceptable, 0,05 à 3,5% en poids, sur la base du poids total de la composition, d'au moins un composé de formule (I) :

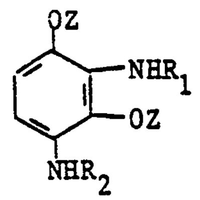
(I)

dans laquelle $R_1$ et $R_2$ représentent indépendamment l'un de l'autre un atome d'hydrogène, un radical alkyle ayant 1 à 4 atomes de carbone ou un radical mono-ou polyhydroxyalkyle ayant 2 ou 3 atomes de carbone, Z et Z' représentent indépendamment l'un de l'autre un radical alkyle ayant 1 à 4 atomes de carbone ou hydroxyalkyle ayant 2 à 4 atomes de carbone, sous réserve que lorsque $R_1$ et $R_2$ désignent simultanément un atome d'hydrogène, Z et Z' ne désignent pas simultanément le radical méthyle, ou de l'un des sels d'acide de ce composé, à titre de coupleur, et au moins un précurseur de colorant d'oxydation de type para, la concentration totale en coupleurs et en précurseurs de colorants d'oxydation de type para étant comprise entre 0,1 et 7% en poids sur la base du poids total de la composition, puis à ajouter au moins un adjuvant cosmétique choisi parmi les tensio-actifs anioniques, cationiques, non-ioniques, amphotères ou leurs mélanges, les solvants organiques, les agents épaississants, les agents antioxydants, les agents de pénétration, les agents séquestrants, les tampons et les parfums, et enfin à ajuster le pH de la composition de teinture à une valeur comprise entre 8 et 11, et de préférence entre 9 et 11, à l'aide d'un agent alcalinisant.

**28.** Procédé selon la revendication 27, caractérisé par le fait qu'on utilise à titre de coupleur de formule (I) un composé choisi parmi le 4-(β-hydroxyéthyl)amino-2-amino-1,3-diméthoxybenzène, le 4-amino-2-(β-hydroxyéthyl)amino-1,3-diméthoxybenzène, le 4-méthylamino-2-amino-1,3-diméthoxybenzène, le 2,4-di-(β-hydroxyéthyl)amino-1,3-diméthoxybenzène, le 2,4-diamino-1,3-diéthoxybenzène, le 2,4-diamino-1,3-di(γ-hydroxypropoxy)benzène ou leurs sels d'addition avec un acide.

**29.** Procédé selon la revendication 27 ou 28, caractérisé par le fait qu'on utilise un précurseur de colorant d'oxydation de type para choisi parmi les p-phénylènediamines, les p-aminophénols, les composés para hétérocycliques ou leurs mélanges.

**30.** Procédé selon la revendication 29, caractérisé par le fait qu'on utilise au moins une p-phénylènediamine répondant à la formule :

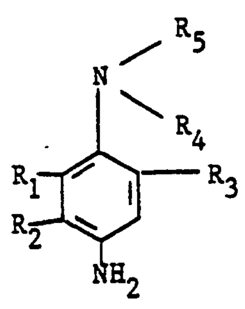
(XII)

dans laquelle $R_1$, $R_2$ et $R_3$ sont indentiques ou différents et représentent un atome d'hydrogène ou

d'halogène, un radical alkyle ayant 1 à 4 atomes de carbone, un radical alcoxy ayant 1 à 4 atomes de carbone, $R_4$ et $R_5$ sont identiques ou différents et représentent un atome d'hydrogène, un radical alkyle, hydroxyalkyle, alcoxyalkyle, carbamylalkyle, mésylaminoalkyle, acétylaminoalkyle, uréidoalkyle, carbéthoxyaminoalkyle, pipéridiminoalkyle, morpholinoalkyle, les groupes alkyle ou alcoxy représentés par $R_4$ et $R_5$ ayant de 1 à 4 atomes de carbone, ou bien $R_4$ et $R_5$ forment conjointement avec l'atome d'azote auquel ils sont liés, un hétérocycle pipéridino ou morpholino sous réserve que $R_1$ ou $R_3$ représentent un atome d'hydrogène lorsque $R_4$ et $R_5$ ne représentent pas un atome d'hydrogène, ou un sel du composé de formule (XII) ci-dessus.

**31.** Procédé selon la revendication 30, caractérisé par le fait qu'on utilise au moins une paraphénylènediamine choisie parmi la p-phénylènediamine, la p-toluylènediamine, la méthoxyparaphénylènediamine, la chloroparaphénylènediamine, la 2, 6-diméthyl-p-phénylènediamine, la 2,5-diméthylparaphénylènediamine, la 2-méthyl-5-méthoxy-paraphénylènediamine, la 2,6-diméthyl-5-méthoxy-paraphénylènediamine, la N,N-diméthylparaphénylènediamine, la 3-méthyl-4-amino-N,N-diéthylaniline, la N,N-di(β-hydroxyéthyl)-paraphénylènediamine, la 3-méthyl-4-amino-N,N-di-(β-hydroxyéthyl)aniline, la 3-chloro-4-amino-N,N-di-(β-hydroxyéthyl)aniline, la 4-amino-N,N-(éthyl, carbamylméthyl)aniline, la 3-méthyl-4-amino-N,N-(éthyl, carbamylméthyl)aniline, la 4-amino-N,N-(éthyl,β-pipéridinoéthyl)aniline, la 3-méthyl-4-amino-N,N-(éthyl,β-pipéridinoéthyl)aniline, la 4-amino-N,N-(éthyl, β-morpholinoéthyl)aniline, la 3-méthyl-4-amino-N,N-(éthyl,β-morpholinoéthyl)aniline, la 4-amino-N,N-(éthyl, β-acétylaminoéthyl)aniline, la 4-amino-N, β-méthoxyéthylaniline, la 3-méthyl-4-amino-N,N-(éthyl, β-acétylaminoéthyl) aniline, la 4-amino-N,N-(éthyl, β-mésylaminoéthyl)aniline, la 3-méthyl-4-amino-N,N-(éthyl, β-mésylaminoéthyl)aniline, la 4-amino- N,N-(éthyl, β-sulfoéthyl)aniline, la 3-méthyl-4-amino-N,N-(éthyl, β-sulfoéthyl)-aniline, la N-[(4'amino)phényl]-morpholine, la N-[(4'-amino)phényl] pipéridine, la 2,3-diméthyl-p-phénylènediamine, l'isopropyl-p-phénylènediamine, sous forme de base libre ou sous forme de sel cosmétiquement acceptable.

**32.** Procédé selon la revendication 29, caractérisé par le fait qu'on utilise au moins un p-aminophénol choisi parmi le p-aminophénol, le 2-méthyl-4-aminophénol, le 3-méthyl-4-aminophénol, le 2-chloro-4-aminophénol, le 3-chloro-4-aminophénol, le 2,6-diméthyl-4-aminophénol, le 3,5-diméthyl-4-aminophénol, le 2,3-diméthyl-4-aminophénol, le 2,5-diméthyl-4-aminophénol, le 2-hydroxyméthyl-4-aminophénol, le 2-(β-hydroxyéthyl)-4-aminophénol, le 2-méthoxy-4-aminophénol, le 3-méthoxy-4-aminophénol.

**33.** Procédé selon la revendication 29, caractérisé par le fait qu'on utilise un composé para hétérocyclique choisi parmi le 2,5-diamino-pyridine, la 2-hydroxy-5-amino-pyridine et la tétraaminopyrimidine.

**34.** Procédé selon l'une quelconque des revendications 27 à 33, caractérisé par le fait qu'on ajoute d'autres coupleurs choisis parmi les métadiphénols, les métaaminophénols, les métaphénylènediamines, les métaacylaminophénols, les métauréidophénols, les métacarbalcoxyaminophénols, l'α-naphtol, les composés β-cétoniques et les pyrazolones.

**35.** Procédé selon l'une quelconque des revendications 27 à 34, caractérisé par le fait qu'on ajoute également des précurseurs de colorants de type ortho choisis parmi les orthoaminophénols, les orthophénylènediamines et les orthodiphénols.

**36.** Procédé selon l'une quelconque des revendications 27 à 35, caractérisé par le fait qu'on ajoute en outre des colorants directs choisis parmi les colorants azoïques, anthraquinoniques et les dérivés nitrés de la série benzénique.

**37.** Procédé selon l'une quelconque des revendications 27 à 36, caractérisé par le fait qu'on ajoute 1 à 40% en poids d'un solvant organique choisi parmi les alcanols inférieurs, le glycérol, les glycols ou éthers de glycols, et leurs mélanges.

**38.** Procédé selon l'une quelconque des revendications 27 à 36, caractérisé par le fait qu'on ajoute 0,5 à 40% en poids d'au moins un agent tensio-actif anionique, cationique, non-ionique, amphotère ou leurs mélanges.

## Claims

### Claims for the following Contracting States : AT, BE, CH, DE, FR, GB, GR, IT, LI, NL, SE

1. Compounds of formula (I)

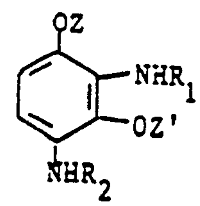

(I)

in which:
$R_1$ and $R_2$ denote independently of each other a hydrogen atom, an alkyl radical containing from 1 to 4 carbon atoms or a mono- or polyhydroxyalkyl radical containing 2 or 3 carbon atoms,
Z and z' denote independently of each other an alkyl radical containing from 1 to 4 carbon atoms or a hydroxyalkyl radical containing from 2 to 4 carbon atoms, provided that when $R_1$ and $R_2$ simultaneously denote a hydrogen atom, Z and Z' do not simultaneously denote the methyl radical, and their addition salts with an acid.

2. Compounds according to Claim 1, characterised in that they are chosen from 4-(β-hydroxyethyl)amino-2-amino-1,3-dimethoxybenzene, 4-amino-2-(β-hydroxyethyl)amino-1,3-dimethoxybenzene, 4-methylamino-2-amino-1,3-dimethoxybenzene, 2,4-di(β-hydroxyethyl)amino-1,3-dimethoxybenzene, 2,4-diamino-1,3-diethoxybenzene, 2,4-diamino-1,3-di(γ-hydroxypropoxy)benzene and their addition salts with an acid.

3. Use as a coupler of a compound of formula (I) according to Claim 1 or 2 or of one of its addition salts with an acid, in combination with precursors of oxidation dyes, for dyeing keratinous fibres, in particular human hair.

4. Hair-dyeing composition characterised in that it contains, in a cosmetically acceptable aqueous carrier, at least one compound of formula (I) according to Claim 1 or 2 or one of its acid salts, as a coupler, in combination with at least one precursor of oxidation dye of para type.

5. Dye composition according to Claim 4, characterised in that it contains 0.05 to 3.5 % by weight of compound (I) or of one of its addition salts with an acid, on the basis of the total weight of the composition.

6. Dye composition according to Claim 4, characterised in that the precursor of oxidation dye of para type is chosen from para-phenylenediamines, para-aminophenols, para heterocyclic compounds or their mixtures.

7. Dye composition according to Claim 6, characterised in that the para-phenylenediamines correspond to the formula:

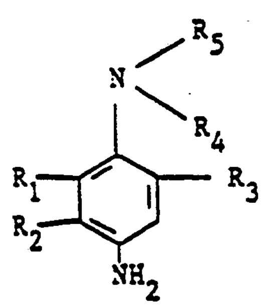

(XII)

in which $R_1$, $R_2$ and $R_3$ are identical or different and denote a hydrogen or halogen atom, an alkyl radical containing 1 to 4 carbon atoms, an alkoxy radical containing 1 to 4 carbon atoms, $R_4$ and $R_5$ are

identical or different and denote a hydrogen atom or an alkyl, hydroxyalkyl, alkoxyalkyl, carbamylalkyl, mesylaminoalkyl, acetylaminoalkyl, ureidoalkyl, carbethoxyaminoalkyl, piperidinoalkyl or morpholinoalkyl radical, the alkyl or alkoxy groups denoted by $R_4$ and $R_5$ containing from 1 to 4 carbon atoms, or else $R_4$ and $R_5$ together with the nitrogen atom to which they are bonded form a piperidino or morpholino heterocyclic ring, provided that $R_1$ or $R_3$ denote a hydrogen atom when $R_4$ and $R_5$ do not denote a hydrogen atom, or are salts of the compounds of above formula (XII).

8. Dye composition according to Claim 7, characterised in that it contains at least one para-phenylenediamine chosen from p-phenylenediamine, p-tolylenediamine, methoxy-para-phenylenediamine, chloro-para-phenylenediamine, 2,6-dimethyl-p-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-di-(β-hydroxyethyl)-para-phenylenediamine, 3-methyl-4-amino-N,N-di(β-hydroxyethyl)aniline, 3-chloro-4-amino-N,N-di(β-hydroxyethyl)aniline, 4-amino-N,N-(ethyl, carbamylmethyl)aniline, 3-methyl-4-amino-N,N-(ethyl, carbamylmethyl)aniline, 4-amino-N,N-(ethyl, β-piperidinoethyl)aniline, 3-methyl-4amino-N,N-(ethyl, β-piperidinoethyl)aniline, 4-amino-N,N-(ethyl, β-morpholinoethyl)aniline, 3-methyl-4-amino-N,N-(ethyl, -morpholinoethyl)aniline, 4-amino-N,N-(ethyl, β-acetylaminoethyl)aniline, 4-amino-N-β-methoxyethylaniline, 3-methyl-4-amino-N,N-(ethyl, β-acetylaminoethyl)aniline, 4-amino-N,N-(ethyl, β-mesylaminoethyl)aniline, 3-methyl-4-amino-N,N(ethyl, β-mesylaminoethyl)aniline, 4-amino-N,N-(ethyl, β-sulphoethyl)aniline, 3-methyl-4-amino-N,N-(ethyl, β-sulphoethyl)aniline, N-[(4'-amino)phenyl]morpholine, N-[(4'-amino)phenyl]piperidine, 2,3-dimethyl-p-phenylenediamine and isopropyl-p-phenylenediamine, in the form of free base or in the form of cosmetically acceptable salt.

9. Dye composition according to Claim 6, characterised in that it contains at least one p-aminophenol chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)4-aminophenol, 2-methoxy-4-aminophenol and 3-methoxy-4-aminophenol.

10. Dye composition according to Claim 6, characterised in that the precursor of oxidation dye of para type is a para heterocyclic compound chosen from 2,5-diaminopyridine, 2-hydroxy-5-aminopyridine and tetraaminopyrimidine.

11. Dye composition according to any one of Claims 4 to 10, characterised in that it contains other couplers chosen from meta-diphenols, meta-aminophenols, metaphenylenediamines, meta-acylaminophenols, meta-ureidophenols, meta-carbalkoxyaminophenols, α-naphthol, β-ketonic compounds and pyrazolones.

12. Dye composition according to any one of Claims 4 to 11, characterised in that it contains 0.1 to 7 % by weight of one or more precursors of oxidation dyes of para type and couplers on the basis of the total weight of the composition.

13. Dye composition according to any one of Claims 4 to 12, characterised in that it additionally contains dye precursors of ortho type chosen from ortho-aminophenols, ortho-phenylenediamines and ortho-diphenols.

14. Dye composition according to any one of Claims 4 to 13, characterised in that it additionally contains direct dyes chosen from azo and anthraquinone dyes and nitrated derivatives of the benzene series.

15. Dye composition according to any one of Claims 4 to 14, characterised in that it has a pH of between 8 and 11 and preferably between 9 and 11.

16. Dye composition according to any one of Claims 4 to 15, characterised in that it contains 1 to 40 % by weight of an organic solvent chosen from lower alkanols, glycerol, glycols or glycol ethers and their mixtures.

17. Dye composition according to any one of Claims 4 to 16, characterised in that it additionally contains 0.5 to 40 % by weight of at least one anionic, cationic, nonionic or amphoteric surface-active agent or

mixtures thereof.

**18.** Dye composition according to any one of Claims 4 to 17, characterised in that it additionally contains cosmetic adjuvants chosen from thickeners, antioxidant agents, penetrating agents, sequestering agents, buffers, perfumes, alkalifying agents and propellants.

**19.** Hair-dyeing process using development by an oxidising agent, characterised in that it consists in mixing at the time of use the dye composition according to any one of Claims 4 to 18 with an oxidising solution in a sufficient quantity and in then applying the mixture obtained to hair, in leaving it in place for 10 to 40 minutes, preferably 15 to 30 minutes, and in then rinsing the hair, washing it with shampoo, rinsing it with water and drying it.

**20.** Hair-dyeing process using development by an oxidising agent, characterised in that it consists in a first step in applying to hair a dye composition containing at least one precursor of oxidation dye of para type such as defined in any one of Claims 6 to 10 and then, in a second step, in applying a dye composition containing a compound of formula (I) or one of its addition salts with an acid, the oxidising agent being present in the composition applied in the second step or else applied to the hair itself in a third step, in leaving it in place for 10 to 40 minutes, preferably 15 to 30 minutes, and in then rinsing the hair, washing it with shampoo, rinsing it again and drying it.

**21.** Process for the preparation of a compound of formula (I) according to Claim 1, in which $R_1$ and $R_2$ denote a hydrogen atom and Z and Z' do not simultaneously denote the methyl radical, characterised in that it consists in performing the consecutive or simultaneous reduction and dehalogenation of 2,4-disubstituted 3,5-dinitrochlorobenzene of following formula (II):

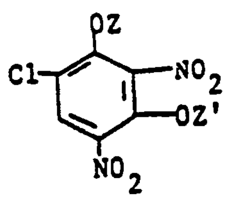

(II)

in which Z and Z' have the meanings shown in Claim 1.

**22.** Process according to Claim 21, characterised in that it consists,

a) in a first stage, in reducing the compound (II) with iron in the presence of acetic acid at a temperature of between 50 and 100°C to obtain the 2,4-disubstituted 3,5-diaminochlorobenzene of formula (IIIA):

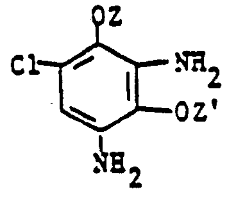

(IIIA)

in which Z and Z' have the meanings shown in Claim 1, and then

b) in a second stage, in dehalogenating the compound (IIIA) in a solvent medium consisting of water, a lower alcohol or a hydroalcoholic mixture in the presence of palladium on charcoal, of ammonium acetate and of triethylamine formate at a temperature of between 50°C and 100°C.

**23.** Process according to Claim 21, characterised in that it consists in simultaneously reducing and dehalogenating the compound (II) under hydrogen pressure in the presence of palladium on charcoal in a solvent chosen from water, lower alcohols or hydroalcoholic mixtures at a temperature of between

50°C and 200°C, preferably in the presence of ammonium acetate or of triethylamine.

**24.** Process for the preparation of a compound of formula (I) according to Claim 1, in which $R_1$ and $R_2$ are other than a hydrogen atom and have identical meanings, from a 2,4-disubstituted 3,5-dinitrochlorobenzene, characterised in that it consists:

a) in a first stage, in reducing the 2,4-disubstituted 3,5-dinitrochlorobenzene of following formula (II):

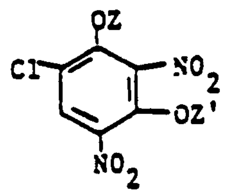

(II)

in which Z and Z' have the same meanings as in Claim 1, but may simultaneously denote the methyl radical, using iron in the presence of acetic acid at a temperature of between 50 and 100°C, to obtain the 2,4-disubstituted 3,5-diaminochlorobenzene of following formula (IIIA):

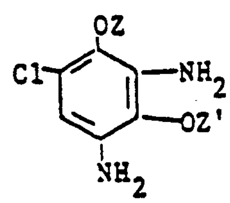

(IIIA)

Z and Z' having the meanings shown above;

b) in a second stage, in performing the dehalogenation of the compound of formula (IIIA) in the presence of palladium on charcoal, of ammonium acetate and of triethylamine formate, at a temperature of between 50°C and 100°C, in a solvent medium consisting of water, a lower alcohol or a hydroalcoholic mixture, to obtain the 1,3-disubstituted 2,4-diaminobenzene of following formula (IA):

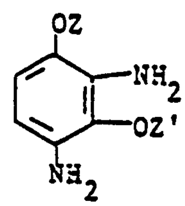

(IA)

Z and Z' having the meanings shown in Claim 1 and being capable of simultaneously denoting a methyl radical;

c) in a third stage, in performing the alkylation or hydroxyalkylation of the 1,3-disubstituted 2,4-diaminobenzene of formula (IA) according to the conventional methods employed for aromatic amines;

it being possible for the second and third stages to be inverted.

**25.** Process for the preparation of a compound of formula (I) according to Claim 1, in which $R_2$ is other than a hydrogen atom and $R_1$ = H from a 2,4-disubstituted 3,5-dinitrochlorobenzene of formula (II), shown in Claim 25, characterised in that it consists:

a) in a first stage, in performing a controlled reduction of the compound of formula (II) using hydrogen transfer in the presence of a catalyst such as palladium on charcoal and of cyclohexene as hydrogen donor, to obtain the 2,4-disubstituted 5-amino-3-nitrochlorobenzene of formula (IV) below:

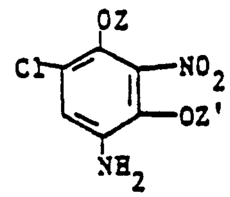

(IV)

in which Z and Z' have the meanings shown in Claim 1 but may simultaneously denote the methyl radical;
b) in a second stage, in subjecting the compound of formula (IV) to an alkylation or a hydroxyalkylation of the amino group to obtain the compound of following formula (V):

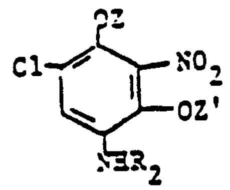

(V)

in which Z, Z' and $R_2$ have the meanings shown in Claim 1, it being possible for Z and Z' simultaneously to denote the methyl radical;
c) in a third stage, in performing the reduction of the compound of formula (V) using iron in the presence of acetic acid, at a temperature of between 20 and 100° C, to obtain the compound of following formula ($III_B$):

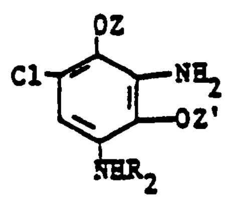

($III_B$)

in which Z, Z' and $R_2$ have the meanings shown above;
d) in a fourth stage, in performing the dehalogenation of the compound of formula ($III_B$) according to the process shown in Claim 24, to obtain the compound of following formula ($I_B$):

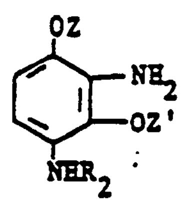

($I_B$)

in which Z, Z' and $R_2$ have the meanings shown above.

**26.** Process for the preparation of a compound of formula (I) according to Claim 1, in which $R_2$ is a hydrogen atom and $R_1$ is other than a hydrogen atom from a 2,4-disubstituted 3,5-dinitrochlorobenzene of formula (II), shown in Claim 24, characterised in that it consists:
a) in a first stage, in performing the controlled reduction of the compound of formula (II) according to the method shown in Claim 25, to obtain the 2,4-disubstituted 5-amino-3-nitrochlorobenzene of formula (IV);
b) in a second stage, in acetylating the compound of formula (IV) using acetic anhydride at a

temperature of between 20 and 100 ° C to obtain the compound of following formula (VI):

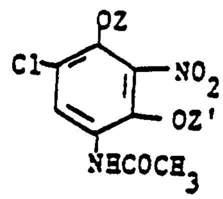

(VI)

in which Z and Z' have the meanings shown in Claim 1 and may simultaneously denote the methyl radical;

c) in a third stage, in performing the simultaneous reduction and dehalogenation of the compound of formula (IV) under hydrogen pressure by employing palladium on charcoal in the presence of ammorium acetate or of triethylamine in a solvent consisting of water, a lower alcohol or a hydroalcoholic mixture, at a temperature of between 50 ° C and 200 ° C, to obtain the 1,3-disubstituted 4-acetamido-2-aminobenzene of following formula (IX):

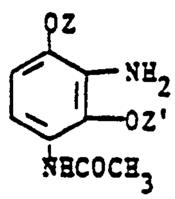

(IX);

d) in a fourth stage, in performing the alkylation or the hydroxyalkylation of the amino group of the compound of formula (IX) according to conventional methods for aromatic amines, to obtain the compound of following formula (X):

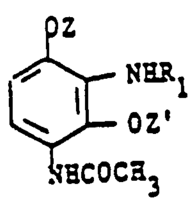

(X);

e) in a fifth stage, in deacetylating the compound of formula (X) to obtain the final compound of following formula ($I_C$):

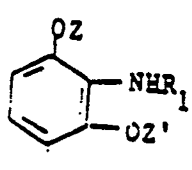

($I_C$)

in which $R_1$, Z and Z' have the meanings shown in Claim 1, $R_1$ being other than a hydrogen atom and Z and Z' being capable of simultaneously denoting the methyl radical.

**27.** Process for the preparation of a compound of formula ($I_C$) according to Claim 26, in which $R_2$ is a hydrogen atom and $R_1$ is other than a hydrogen atom, characterised in that the third stage of simultaneous reduction and dehalogenation of the 2,4-disubstituted 5-acetamido-3-nitrochlorobenzene of formula (VI) is replaced by a simple reduction stage using iron in the presence of acetic acid at a temperature of between 50 ° C and 100 ° C, resulting in the formation of the 2,4-disubstituted 5-acetamido-3-aminochlorobenzene of following formula (VII):

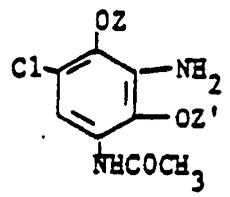

(VII),

in which Z and Z' have the meanings shown in Claim 1 and may simultaneously denote the methyl radical, this reduction stage being followed by a stage of alkylation or hydroxyalkylation of the compound of formula (VII) to obtain the compound of following formula (VIII):

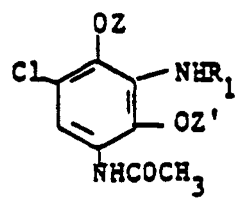

(VIII)

and then a stage of deacetylation of the compound of formula (VIII) to result in the compound of following formula ($III_C$):

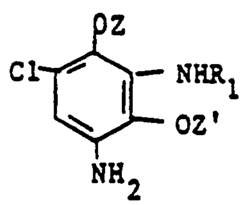

($III_C$)

which is then dehalogenated in the presence of palladium on charcoal, of ammonium acetate and of triethylamine formate in a solvent consisting of water, a lower alcohol or a hydroalcoholic mixture, at a temperature of between 50° C and 100° C to result in the compound of formula ($I_C$) shown in Claim 26, in which $R_1$, Z and Z' have the meanings shown in Claim 1, Z and Z' being capable of simultaneously denoting the methyl radical and $R_1$ being other than a hydrogen atom.

**28.** Compound of formula:

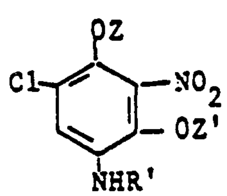

(XI)

in which R' denotes a hydrogen atom, an alkyl radical containing from 1 to 4 carbon atoms, a mono- or polyhydroxyalkyl radical containing 2 or 3 carbon atoms, acetyl or β-chloroethoxycarbonyl, and Z and Z' denote independently of each other an alkyl radical containing from 1 to 4 carbon atoms or a hydroxyalkyl radical containing from 2 to 4 carbon atoms.

**29.** Compound according to Claim 28, characterised in that it is chosen from 5-amino-2,4-dimethoxy-3-nitrochlorobenzene, 5-acetamido-2,4-dimethoxy-3-nitrochlorobenzene, 5-methylamino-2,4-dimethoxy-3-nitrochlorobenzene and 5-(β-chloroethoxycarbonyl)amino-2,4-dimethoxy-3-nitrochlorobenzene.

**Claims for the following Contracting State : ES**

1. Use as a coupler of a compound of formula (I)

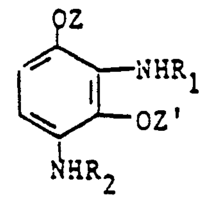

(I)

in which:
$R_1$ and $R_2$ denote independently of each other a hydrogen atom, an alkyl radical containing from 1 to 4 carbon atoms or a mono- or polyhydroxyalkyl radical containing 2 or 3 carbon atoms,
Z and Z' denote independently of each other an alkyl radical containing from 1 to 4 carbon atoms or a hydroxyalkyl radical containing from 2 to 4 carbon atoms, provided that when $R_1$ and $R_2$ simultaneously denote a hydrogen atom, Z and Z' do not simultaneously denote the methyl radical,
or of one of its addition salts with an acid, in combination with precursors of oxidation dyes, for dyeing keratinous fibres, in particular human hair.

2. Hair-dyeing composition characterised in that it contains, in a cosmetically acceptable aqueous carrier, at least one compound of formula (I) according to Claim 1 or one of its acid salts, as a coupler, in combination with at least one precursor of oxidation dye of para type.

3. Dye composition according to Claim 2, characterised in that it contains, as a coupler, at least one compound chosen from 4-(β-hydroxyethyl)amino-2-amino-1,3-dimethoxybenzene, 4-amino-2(β-hydroxyethyl)amino-1,3-dimethoxybenzene, 4-methylamino-2-amino-1,3-dimethoxybenzene, 2,4-di(β-hydroxyethyl)amino-1,3-dimethoxybenzene, 2,4-diamino-1,3-diethoxybenzene, 2,4-diamino-1,3-di(γ-hydroxypropoxy)benzene and their addition salts with an acid.

4. Dye composition according to Claim 2 or 3, characterised in that it contains 0.05 to 3.5 % by weight of compound (I) or of one of its addition salts with an acid, on the basis of the total weight of the composition.

5. Dye composition according to Claim 2, characterised in that the precursor of oxidation dye of para type is chosen from para-phenylenediamines, para-aminophenols, para heterocyclic compounds or their mixtures.

6. Dye composition according to Claim 5, characterised in that the para-phenylenediamines correspond to the formula:

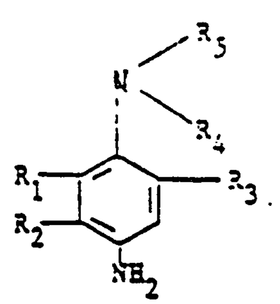

(XII)

in which $R_1$, $R_2$ and $R_3$ are identical or different and denote a hydrogen or halogen atom, an alkyl radical containing 1 to 4 carbon atoms, an alkoxy radical containing 1 to 4 carbon atoms, $R_4$ and $R_5$ are identical or different and denote a hydrogen atom or an alkyl, hydroxyalkyl, alkoxyalkyl, carbamylalkyl,

mesylaminoalkyl, acetylaminoalkyl, ureidoalkyl, carbethoxyaminoalkyl , piperidinoalkyl or morpholinoalkyl radical , the alkyl or alkoxy groups denoted by $R_4$ and $R_5$ containing from 1 to 4 carbon atoms, or else $R_4$ and $R_5$ together with the nitrogen atom to which they are bonded form a piperidino or morpholino heterocyclic ring, provided that $R_1$ or $R_3$ denote a hydrogen atom when $R_4$ and $R_5$ do not denote a hydrogen atom, or are salts of the compounds of above formula (XII).

7. Dye composition according to Claim 6, characterised in that it contains at least one para-phenylenediamine chosen from p-phenylenediamine, p-tolylenediamine, methoxy-para-phenylenediamine, chloro-paraphenylenediamine, 2,6-dimethyl-p-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-di-(β-hydroxyethyl)-para-phenylenediamine, 3-methyl-4-amino-N,N-di(β-hydroxyethyl)aniline, 3-chloro-4-amino-N,N-di(β-hydroxyethyl)aniline, 4-amino-N,N-(ethyl, carbamylmethyl)aniline, 3-methyl-4-amino-N,N-(ethyl, carbamylmethyl)aniline, 4-amino-N,N-(ethyl, β-piperidinoethyl)aniline, 3-methyl-4-amino-N,N-(ethyl, β-piperidinoethyl)aniline, 4-amino-N,N-(ethyl, β-morpholinoethyl)aniline, 3-methyl-4-amino-N,N-(ethyl, β-morpholinoethyl)aniline, 4-amino-N,N-(ethyl, β-acetylaminoethyl)aniline, 4-amino-N-β-methoxyethylaniline, 3-methyl-4-amino-N,N-(ethyl, β-acetylaminoethyl)aniline, 4-amino-N,N-(ethyl, β-mesylaminoethyl)aniline, 3-methyl-4-amino-N,N-(ethyl, β-mesylaminoethyl)aniline, 4-amino-N,N-(ethyl, β-sulphoethyl)aniline, 3-methyl-4-amino-N,N(ethyl, β-sulphoethyl)aniline, N-[(4'amino)phenyl]-morpholine, N-[(4'-amino)phenyl]piperidine, 2, 3-dimethyl-p-phenylenediamine and isopropyl-p-phenylenediamine, in the form of free base or in the form of a cosmetically acceptable salt.

8. Dye composition according to Claim 5, characterised in that it contains at least one p-aminophenol chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol and 3-methoxy-4-aminophenol.

9. Dye composition according to Claim 5, characterised in that the precursor of oxidation dye of para type is a para heterocyclic compound chosen from 2,5-diaminopyridine, 2-hydroxy-5-aminopyridine and tetraaminopyrimidine.

10. Dye composition according to any one of Claims 2 to 9, characterised in that it contains other couplers chosen from meta-diphenols, meta-aminophenols, metaphenylenediamines, meta-acylaminophenols, meta-ureidophenols, meta-carbalkoxyaminophenols, α-naphthol, β-ketonic compounds and pyrazolones.

11. Dye composition according to any one of Claims 2 to 10, characterised in that it contains 0.1 to 7 % by weight of one or more precursors of oxidation dyes of para type and couplers on the basis of the total weight of the composition.

12. Dye composition according to any one of Claims 2 to 11, characterised in that it additionally contains dye precursors of ortho type chosen from ortho-aminophenols, ortho-phenylenediamines and ortho-diphenols.

13. Dye composition according to any one of Claims 2 to 12, characterised in that it additionally contains direct dyes chosen from azo and anthraquinone dyes and nitrated derivatives of the benzene series.

14. Dye composition according to any one of Claims 2 to 13, characterised in that it has a pH of between 8 and 11 and preferably between 9 and 11.

15. Dye composition according to any one of Claims 2 to 14, characterised in that it contains 1 to 40 % by weight of an organic solvent chosen from lower alkanols, glycerol, glycols or glycol ethers and their mixtures.

16. Dye composition according to any one of Claims 2 to 15, characterised in that it additionally contains 0.5 to 40 % by weight of at least one anionic, cationic, nonionic or amphoteric surface-active agent or mixtures thereof.

17. Dye composition according to any one of Claims 2 to 16, characterised in that it additionally contains cosmetic adjuvants chosen from thickeners, antioxidant agents, penetrating agents, sequestering agents, buffers, perfumes, alkalifying agents and propellants.

18. Hair-dyeing process using development by an oxidising agent, characterised in that it consists in mixing at the time of use the dye composition according to any one of Claims 2 to 17 with an oxidising solution in a sufficient quantity and in then applying the mixture obtained to hair, in leaving it in place for 10 to 40 minutes, preferably 15 to 30 minutes, and in then rinsing the hair, washing it with shampoo, rinsing it with water and drying it.

19. Hair-dyeing process using development by an oxidising agent, characterised in that it consists in a first step in applying to hair a dye composition containing at least one precursor of oxidation dye of para type such as defined in any one of Claims 5 to 9 and then, in a second step, in applying a dye composition containing a compound of formula (I) or one of its addition salts with an acid, the oxidising agent being present in the composition applied in the second step or else applied to the hair itself in a third step, in leaving it in place for 10 to 40 minutes, preferably 15 to 30 minutes, and in then rinsing the hair, washing it with shampoo, rinsing it again and drying it.

20. Process for the preparation of a compound of formula (I) according to Claim 1, in which $R_1$ and $R_2$ denote a hydrogen atom and Z and Z' do not simultaneously denote the methyl radical, characterised in that it consists in performing the consecutive or simultaneous reduction and dehalogenation of 2,4-disubstituted 3,5-dinitrochlorobenzene of following formula (II):

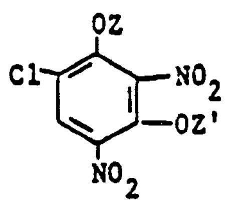

(II)

in which Z and Z' have the meanings shown in Claim 1.

21. Process according to Claim 20, characterised in that it consists,

a) in a first stage, in reducing the compound (II) with iron in the presence of acetic acid at a temperature of between 50 and 100°C to obtain the 2,4-disubstituted 3,5-diaminochlorobenzene of formula (IIIA):

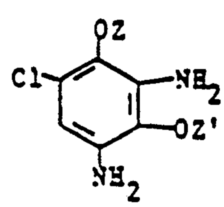

(IIIA)

in which Z and Z' have the meanings shown in Claim 1, and then

b) in a second stage, in dehalogenating the compound (IIIA) in a solvent medium consisting of water, a lower alcohol or a hydroalcoholic mixture in the presence of palladium on charcoal, of ammonium acetate and of triethylamine formate at a temperature of between 50°C and 100°C.

22. Process according to Claim 20, characterised in that it consists in simultaneously reducing and dehalogenating the compound (II) under hydrogen pressure in the presence of palladium on charcoal in a solvent chosen from water, lower alcohols or hydroalcoholic mixtures at a temperature of between 50°C and 200°C, preferably in the presence of ammonium acetate or of triethylamine.

23. Process for the preparation of a compound of formula (I) according to Claim 1, in which $R_1$ and $R_2$ are

other than a hydrogen atom and have identical meanings, from a 2,4-disubstituted 3,5-dinitrochlorobenzene, characterised in that it consists:

a) in a first stage, in reducing the 2,4-disubstituted 3,5-dinitrochlorobenzene of following formula (II):

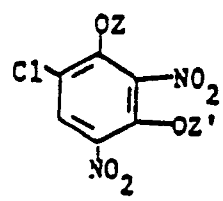

(II)

in which Z and Z' have the same meanings as in Claim 1, but may simultaneously denote the methyl radical, using iron in the presence of acetic acid at a temperature of between 50 and 100 °C, to obtain the 2,4-disubstituted 3,5-diaminochlorobenzene of following formula (IIIA):

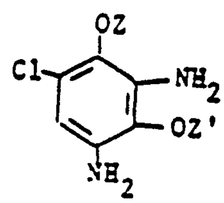

(IIIA)

Z and Z' having the meanings shown above;

b) in a second stage, in performing the dehalogenation of the compound of formula (IIIA) in the presence of palladium on charcoal, of ammonium acetate and of triethylamine formate, at a temperature of between 50 °C and 100 °C, in a solvent medium consisting of water, a lower alcohol or a hydroalcoholic mixture, to obtain the 1,3-disubstituted 2,4-diaminobenzene of following formula (IA):

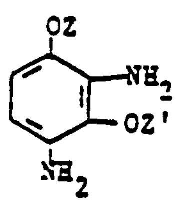

(IA)

Z and Z' having the meanings shown in Claim 1 and being capable of simultaneously denoting a methyl radical;

c) in a third stage, in performing the alkylation or hydroxyalkylation of the 1,3-disubstituted 2,4-diaminobenzene of formula (IA) according to the conventional methods employed for aromatic amines;

it being possible for the second and third stages to be inverted.

**24.** Process for the preparation of a compound of formula (I) according to Claim 1, in which $R_2$ is other than a hydrogen atom and $R_1$ = H from a 2,4-disubstituted 3,5-dinitrochlorobenzene of formula (II), shown in Claim 23, characterised in that it consists:

a) in a first stage, in performing a controlled reduction of the compound of formula (II) using hydrogen transfer in the presence of a catalyst such as palladium on charcoal and of cyclohexene as hydrogen donor, to obtain the 2,4-disubstituted 5-amino-3-nitrochlorobenzene of formula (IV) below:

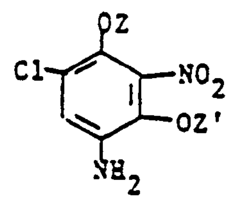

(IV)

in which Z and Z' have the meanings shown in Claim 1 but may simultaneously denote the methyl radical;

b) in a second stage, in subjecting the compound of formula (IV) to an alkylation or a hydroxyalkylation of the amino group to obtain the compound of following formula (V):

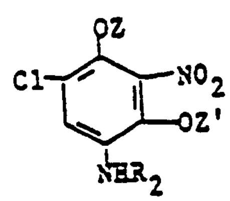

(V)

in which Z, Z' and $R_2$ have the meanings shown in Claim 1, it being possible for Z and Z' simultaneously to denote the methyl radical;

c) in a third stage, in performing the reduction of the compound of formula (V) using iron in the presence of acetic acid, at a temperature of between 20 and 100°C, to obtain the compound of following formula ($III_B$):

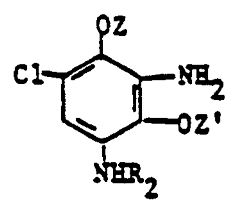

($III_B$)

in which Z, Z' and $R_2$ have the meanings shown above;

d) in a fourth stage, in performing the dehalogenation of the compound of formula ($III_B$) according to the process shown in Claim 24, to obtain the compound of following formula ($I_B$):

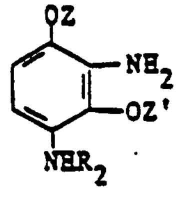

($I_B$)

in which Z, Z' and $R_2$ have the meanings shown above.

**25.** Process for the preparation of a compound of formula (I) according to Claim 1, in which $R_2$ is a hydrogen atom and $R_1$ is other than a hydrogen atom from a 2,4-disubstituted 3,5-dinitrochlorobenzene of formula (II), shown in Claim 23, characterised in that it consists:

a) in a first stage, in performing the controlled reduction of the compound of formula (II) according to the method shown in Claim 24, to obtain the 2,4-disubstituted 5-amino-3-nitrochlorobenzene of formula (IV);

b) in a second stage, in acetylating the compound of formula (IV) using acetic anhydride at a temperature of between 20 and 100 °C to obtain the compound of following formula (VI):

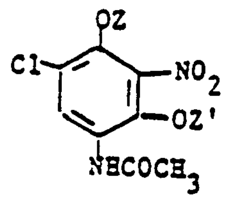

(VI)

in which Z and Z' have the meanings shown in Claim 1 and may simultaneously denote the methyl radical;

c) in a third stage, in performing the simultaneous reduction and dehalogenation of the compound of formula (IV) under hydrogen pressure by employing palladium on charcoal in the presence of ammonium acetate or of triethylamine in a solvent consisting of water, a lower alcohol or a hydroalcoholic mixture, at a temperature of between 50 °C and 200 °C, to obtain the 1,3-disubstituted 4-acetamido-2-aminobenzene of following formula (IX):

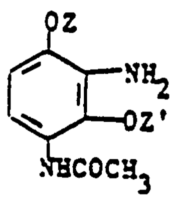

(IX);

d) in a fourth stage, in performing the alkylation or the hydroxyalkylation of the amino group of the compound of formula (IX) according to conventional methods for aromatic amines, to obtain the compound of following formula (X):

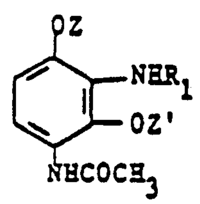

(X);

e) in a fifth stage, in deacetylating the compound of formula (X) to obtain the final compound of following formula ($I_C$):

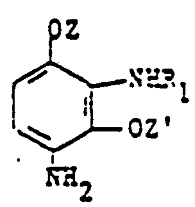

($I_C$)

in which $R_1$, Z and Z' have the meanings shown in Claim 1, $R_1$ being other than a hydrogen atom and Z and Z' being capable of simultaneously denoting the methyl radical.

**26.** Process for the preparation of a compound of formula ($I_C$) according to Claim 25, in which $R_2$ is a hydrogen atom and $R_1$ is other than a hydrogen atom, characterised in that the third stage of simultaneous reduction and dehalogenation of the 2,4-disubstituted 5-acetamido-3-nitrochlorobenzene

of formula (VI) is replaced by a simple reduction stage using iron in the presence of acetic acid at a temperature of between 50° C and 100° C, resulting in the formation of the 2,4-disubstituted 5-acetamido-3-aminochlorobenzene of following formula (VII):

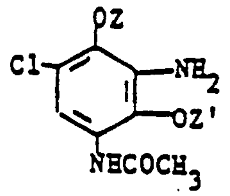

(VII),

in which Z and Z' have the meanings shown in Claim 1 and may simultaneously denote the methyl radical, this reduction stage being followed by a stage of alkylation or hydroxyalkylation of the compound of formula (VII) to obtain the compound of following formula (VIII):

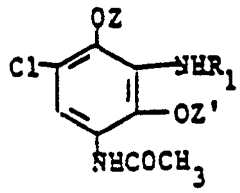

(VIII)

and then a stage of deacetylation of the compound of formula (VIII) to result in the compound of following formula ($III_C$):

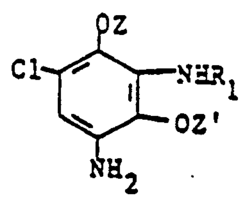

($III_C$)

which is then dehalogenated in the presence of palladium on charcoal, of ammonium acetate and of triethylamine formate in a solvent consisting of water, a lower alcohol or a hydroalcoholic mixture, at a temperature of between 50° C and 100° C to result in the compound of formula ($I_C$) shown in Claim 26, in which $R_1$, Z and Z' have the meanings shown in Claim 1, Z and Z' being capable of simultaneously denoting the methyl radical and $R_1$ being other than a hydrogen atom.

**27.** Process for the preparation of a hair-dyeing composition, characterised in that it consists in dissolving in a cosmetically acceptable aqueous carrier 0.05 to 3.5 % by weight, based on the total weight of the composition, of at least one compound of formula (I):

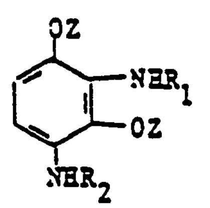

(I)

in which $R_1$ and $R_2$ denote independently of each other a hydrogen atom, an alkyl radical containing 1

to 4 carbon atoms or a mono- or polyhydroxyalkyl raical containing 2 or 3 carbon atoms, Z and Z' denoting independently of each other an alkyl radical containing 1 to 4 carbon atoms or hydroxyalkyl radical containing 2 to 4 carbon atoms, provided that when $R_1$ and $R_2$ simultaneously denote a hydrogen atom, Z and Z' do not simultaneously denote the methyl radical, or of one of the acid salts of this compound, as a coupler, and at least one precursor of oxidation dye of para type, the total concentration of couplers and of precursors of oxidation dyes of para type being between 0.1 and 7 % by weight based on the total weight of the composition, and in then adding at least one cosmetic adjuvant chosen from anionic, cationic, nonionic and amphoteric surfactants or mixtures thereof, organic solvents, thickening agents, antioxidant agents, penetrating agents, sequestering agents, buffers and perfumes and in finally adjusting the pH of the dye composition to a value of between 8 and 11, and preferably between 9 and 11, with the aid of an alkalifying agent.

**28.** Process according to Claim 27, characterised in that the compound employed as a coupler of formula (I) is chosen from 4-(β-hydroxyethyl)amino-2-amino-1,3-dimethoxybenzene, 4-amino-2-(β-hydroxyethyl)-amino-1,3-dimethoxybenzene, 4-methylamino-2-amino-1,3-dimethoxybenzene, 2,4-di(β-hydroxyethyl)-amino-1,3-dimethoxybenzene, 2,4-diamino-1,3-diethoxybenzene, 2,4-diamino-1,3-di(γ-hydroxypropoxy)-benzene or their addition salts with an acid.

**29.** Process according to Claim 27 or 28, characterised in that a precursor of oxidation dye of para type is employed, chosen from p-phenylenediamines, p-aminophenols, para heterocyclic compounds or mixtures thereof.

**30.** Process according to Claim 29, characterised in that at least one p-phenylenediamine is employed, corresponding to the formula:

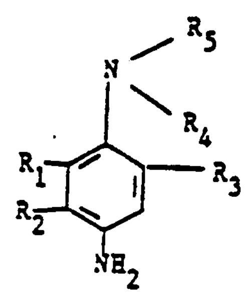

(XII)

in which $R_1$, $R_2$ and $R_3$ are identical or different and denote a hydrogen or halogen atom, an alkyl radical containing 1 to 4 carbon atoms, an alkoxy radical containing 1 to 4 carbon atoms, $R_4$ and $R_5$ are identical or different and denote a hydrogen atom or an alkyl, hydroxyalkyl, alkoxyalkyl, carbamylalkyl, mesylaminoalkyl, acetylaminoalkyl, ureidoalkyl, carbethoxyaminoalkyl, piperidiminoalkyl or morpholinoalkyl radical, the alkyl or alkoxy groups denoted by $R_4$ and $R_5$ containing from 1 to 4 carbon atoms, or else $R_4$ and $R_5$ together with the nitrogen atom to which they are bonded form a piperidino or morpholino heterocyclic ring, provided that $R_1$ or $R_3$ denote a hydrogen atom when $R_4$ and $R_5$ do not denote a hydrogen atom, or a salt of the compound of above formula (XII).

**31.** Process according to Claim 30, characterised in that at least one para-phenylenediamine is employed, chosen from p-phenylenediamine, p-tolylenediamine, methoxy-para-phenylenediamine, chloro-para-phenylenediamine, 2,6-dimethyl-p-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline,N,N-di(β-hydroxyethyl)paraphenylenediamine, 3-methyl-4-amino-N,N di(B-hydroxyethyl)aniline, 3-chloro-4-amino-N,N-di(β-hydroxyethyl)aniline, 4-amino-N,N-(ethyl, N,N-di(β-hydroxyethyl)aniline, 4-amino-N,N-(ethyl, carbamylmethyl)aniline, 3-methyl-4-amino-N,N-(ethyl, carbamylmethyl)aniline, 4-amino-N,N-(ethyl, β-piperidinoethyl)aniline, 3-methyl-4-amino-N,N-(ethyl, β-piperidinoethyl)aniline, 4-amino-N,N-(ethyl, β-morpholinoethyl)aniline, 3-methyl-4-amino-N,N-(ethyl, β-morpholinoethyl)aniline, 4-amino-N,N-(ethyl, β-acetylaminoethyl)aniline, 4-amino-N-β-methoxyethylaniline, 3-methyl-4-amino-N,N-(ethyl, β -acetylaminoethyl)aniline, 4-amino-N,N(ethyl, β-mesylaminoethyl)aniline, 3-methyl-4-amino-N,N(ethyl, β-mesylaminoethyl)aniline, 4-amino-N,N-(ethyl, β-sulphoethyl)aniline, 3-methyl-4-amino-N,N-(ethyl, β-sulphoethyl)aniline, N[(4'amino)phenyl]morpholine,

N[(4'-amino)phenyl]piperidine, 2 ,3-dimethyl-p-phenylenediamine and isopropyl-p-phenylenediamine, in the form of free base or in the form of a cosmetically acceptable salt.

**32.** Process according to Claim 29, characterised in that at least one p-aminophenol is employed, chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2,5-dimethyl-4-aminophenol, 2-hydroxymethyl-4-aminophenol, 2-(β-hydroxyethyl)-4-aminophenol, 2-methoxy-4-aminophenol and 3-methoxy-4-aminophenol.

**33.** Process according to Claim 29, characterised in that a para heterocyclic compound is employed, chosen from 2,5-diaminopyridine, 2-hydroxy-5-aminopyridine and tetraaminopyrimidine.

**34.** Process according to any one of Claims 27 to 33, characterised in that other couplers are added, chosen from meta-diphenols, meta-aminophenols, meta-phenylenediamines, meta-acylaminophenols, meta-ureidophenols, meta-carbalkoxyaminophenols, α-naphthol, β-ketonic compounds and pyrazolones.

**35.** Process according to any one of Claims 27 to 34, characterised in that dye precursors of ortho type are also added, chosen from ortho-aminophenols, ortho-phenylenediamines and ortho-diphenols.

**36.** Process according to any one of Claims 27 to 35, characterised in that direct dyes are additionally added, chosen from azo and anthraquinone dyes and nitrated derivatives of the benzene series.

**37.** Process according to any one of Claims 27 to 36, characterised in that 1 to 40 % by weight of an organic solvent is added, chosen from lower alkanols, glycerol, glycols or glycol ethers and mixtures thereof.

**38.** Process according to any one of Claims 27 to 36, characterised in that 0.5 to 40 % by weight of at least one anionic, cationic, nonionic or amphoteric surface-active agent or mixtures thereof is added.

**Patentansprüche**
**Patentsprüche für folgende Vertragsstaaten: AT, BE, CH, DE, FR, GB, GR, IT, LI, NL, SE**

**1.** Verbindungen der allgemeinen- Formel (I)

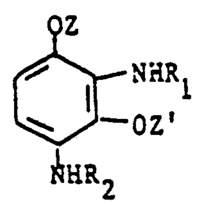

(I)

worin
$R_1$ und $R_2$ unabhängig voneinander ein Wasserstoffatom, einen Alkylrest mit 1 bis 4 Kohlenstoffatomen oder einen Mono- oder Polyhydroxyalkylrest mit 2 oder 3 Kohlenstoffatomen bedeuten,
Z und Z' unabhängig voneinander einen Alkylrest mit 1 bis 4 Kohlenstoffatomen oder einen Hydroxyalkylrest mit 2 bis 4 Kohlenstoffatomen bedeuten, mit der Maßgabe, daß, wenn $R_1$ und $R_2$ gleichzeitig ein Wasserstoffatom bedeuten, Z und Z' nicht gleichzeitig einen Methylrest bedeuten,
und die Säureadditionssalze davon.

**2.** Verbindungen nach Anspruch 1, dadurch gekennzeichnet, daß sie ausgewählt sind unter
4- (ß-Hydroxyethyl)-amino-2-amino-1,3-dimethoxybenzol, 4-Amino-2-(ß-hydroxyethyl)-amino-1,3-dimethoxybenzol, 4-Methylamino-2-amino-1,3-dimethoxybenzol, 2, 4-Di-(ß-hydroxyethyl)-amino-1,3-dimethoxybenzol, 2, 4-Diamino-1,3-diethoxybenzol, 2,4-Diamino-1,3-di-(γ-hydroxypropoxy)-benzol und den Säureadditionssalzen davon.

**3.** Verwendung einer Verbindung der allgemeinen Formel (I) gemäß Anspruch 1 oder 2 oder eines

Säureadditionssalzes davon als Kuppler in Kombination mit Oxidationsfarbstoffprekursoren zur Färbung von Keratinfasern, insbesondere von menschlichem Haar.

4. Haarfärbemittel, dadurch gekennzeichnet, daß es in einem wäßrigen, kosmetisch verträglichen Träger mindestens eine Verbindung der allgemeinen Formel (I) gemäß Anspruch 1 oder 2 oder eines der Säureadditionssalze davon als Kuppler in Kombination mit mindestens einem Oxidationsfarbstoff-Prekursor vom Paratyp enthält.

5. Färbemittel nach Anspruch 4, dadurch gekennzeichnet, daß es von der Verbindung (I) oder einem Säureadditionssalz davon 0,05 - 3,5 Gew.-%, bezogen auf das Gesamtgewicht des Mittels, enthält.

6. Färbemittel nach Anspruch 4, dadurch gekennzeichnet, daß der Oxidationsfarbstoff-Prekursor vom Paratyp ausgewählt ist unter p-Phenylendiaminen, p-Aminophenolen, heterocyclischen Paraverbindungen oder den Gemischen davon.

7. Färbemittel nach Anspruch 6, dadurch gekennzeichnet, daß die p-Phenylendiamine folgende allgemeine Formel besitzen:

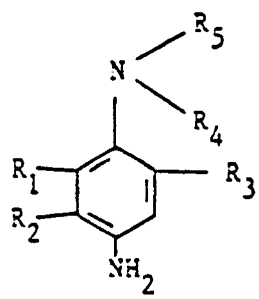

(XII)

worin $R_1$, $R_2$ und $R_3$ gleich oder verschieden sind und ein Wasserstoff- oder Halogenatom, einen Alkylrest mit 1 bis 4 Kohlenstoffatomen oder einen Alkoxyrest mit 1 bis 4 Kohlenstoffatomen bedeuten, $R_4$ und $R_5$ gleich oder verschieden sind und ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbethoxyaminoalkyl-, Piperidinoalkyl-oder Morpholinoalkylrest bedeuten, wobei die Alkyl-oder Alkoxygruppen bedeutenden Reste $R_4$ und $R_5$ 1 bis 4 Kohlenstoffatome besitzen; oder
$R_4$ und $R_5$ zusammen mit dem Stickstoffatom, an dem sie gebunden sind, einen Piperidino- oder Morpholino-heterozyklus bilden, mit der Maßgabe, daß $R_1$ oder $R_3$ ein Wasserstoffatom bedeutet, wenn $R_4$ und $R_5$ nicht für ein Wasserstoffatom stehen,
oder die Salze der Verbindungen gemäß allgemeiner Formel (XII).

8. Färbemittel nach Anspruch 7, dadurch gekennzeichnet, daß es mindestens ein p-Phenylendiamin enthält, ausgewählt unter p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di-(ß-hydroxyethyl)-p-phenylendiamin, 3-Methyl-4-amino-N,N-di-(ß-hydroxyethyl)-anilin, 3-Chlor-4-amino-N,N-di-(ß-hydroxyethyl)-anilin, 4-Amino-N,N-(ethyl, carbamylmethyl)-anilin, 3-Methyl-4-amino-N,N-(ethyl, carbamylmethyl)-anilin, 4-Amino-N,N-(ethyl, ß-piperidinoethyl)-anilin, 3-Methyl-4-amino-N,N-(ethyl, ß-piperidinoethyl)-anilin, 4-Amino-N,N-(ethyl, ß-morpholinoethyl)-anilin, 3-Methyl-4-amino-N,N-(ethyl, ß-morpholinoethyl)-anilin, 4-Amino-N,N-(ethyl, ß-acetylaminoethyl)-anilin, 4-Amino-N, ß-methoxyethylanilin, 3-Methyl-4-amino-N,N-(ethyl, ß-acetylaminoethyl)-anilin, 4-Amino-N,N-(ethyl, ß-mesylaminoethyl)-anilin, 3-Methyl-4-amino-N,N-(ethyl, ß-mesylaminoethyl)-anilin, 4-Amino-N,N-(ethyl, ß-sulfoethyl)-anilin, 3-Methyl-4-amino-N,N-(ethyl, ß-sulfoethyl)-anilin, N-[(4'-Amino)-phenyl]-morpholin, N-[(4'-Amino)-phenyl]-piperidin, 2,3-Dimethyl-p-phenylendiamin und Isopropyl-p-phenylendiamin, in Form der freien Base oder als kosmetisch verträgliches Salz.

9. Färbemittel nach Anspruch 6, dadurch gekennzeichnet, daß es mindestens ein p-Aminophenol enthält, ausgewählt unter p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(ß-Hydroxyethyl)-4-aminophenol, 2-Methoxy-4-aminophenol und 3-Methoxy-4-aminophenol.

**10.** Färbemittel nach Anspruch 6, dadurch gekennzeichnet, daß der Oxidationsfarbstoff-Prekursor vom Paratyp eine heterocyclische Paraverbindung ist, ausgewählt unter 2,5-Diaminopyridin, 2-Hydroxy-5-aminopyridin und Tetraaminopyrimidin.

**11.** Färbemittel nach einem der Ansprüche 4 bis 10, dadurch gekennzeichnet, daß es weitere Kuppler enthält, ausgewählt unter Metadiphenolen, Metaaminophenolen, Metaphenylendiaminen, Metaacylaminophenolen, Metaureidophenolen, Metacarbalkoxyaminophenolen, α-Naphthol, ß-Ketoverbindungen und Pyrazolonen.

**12.** Färbemittel nach einem der Ansprüche 4 bis 11, dadurch gekennzeichnet, daß es 0,1 bis 7 Gew.-% eines oder mehrerer Oxidationsfarbstoff-Prekursoren vom Paratyp und Kuppler, bezogen auf das Gesamtgewicht des Mittels, enthält.

**13.** Färbemittel nach einem der Ansprüche 4 bis 12, dadurch gekennzeichnet, daß es außerdem Farbstoff-Prekursoren vom Ortho-Typ enthält, ausgewählt unter o-Aminophenolen, o-Phenylendiaminen und o-Diphenolen.

**14.** Färbemittel nach einem der Ansprüche 4 bis 13, dadurch gekennzeichnet, daß es außerdem Direktfarbstoffe enthält, ausgewählt unter Azo-, Anthrachinonfarbstoffen und Nitroderivaten der Benzolreihe.

**15.** Färbemittel nach einem der Ansprüche 4 bis 14, dadurch gekennzeichnet, daß es einen pH-Wert von 8 - 11 und vorzugsweise von 9 - 11 besitzt.

**16.** Färbemittel nach einem der Ansprüche 4 bis 15, dadurch gekennzeichnet, daß es 1 - 40 Gew.-% eines organischen Lösungsmittels enthält, ausgewählt unter Niedrigalkanolen, Glycerin, Glykolen oder Glykolethern und Gemischen davon.

**17.** Färbemittel nach einem der Ansprüche 4 bis 16, dadurch gekennzeichnet, daß es außerdem 0,5 - 40 Gew.-% mindestens eines oberflächenaktiven anionischen, kationischen, nicht-ionischen, amphoteren Mittels oder ein Gemisch davon enthält.

**18.** Färbemittel nach einem der Ansprüche 4 bis 17, dadurch gekennzeichnet, daß es außerdem kosmetische Adjuvantien enthält, ausgewählt unter Verdickungsmitteln, Antioxidantien, Penetrationsmitteln, Sequestriermitteln, Puffern, Parfüms, Alkalisierungsmitteln und Treibmitteln.

**19.** Verfahren zur Haarfärbung unter Entwicklung mit einem Oxidans, dadurch gekennzeichnet, daß man im Augenblick der Anwendung das Färbemittel nach einem der Ansprüche 4 bis 18 mit einer Oxidationslösung in ausreichender Menge mischt, anschließend das erhaltene Gemisch auf das Haar aufträgt, es dort 10 - 40 min, vorzugsweise 15 - 30 min beläßt, anschließend das Haar spült, es mit einem Shampoo wäscht, mit Wasser spült und trocknet.

**20.** Verfahren zur Haarfärbung unter Entwicklung mit einem Oxidans, dadurch gekennzeichnet, daß man zuerst auf das Haar ein Färbemittel aufträgt, welches mindestens einen Oxidationsfarbstoff-Prekursor vom Paratyp entsprechend der Definition gemäß einem der Ansprüche 6 bis 10 aufträgt, dann in einem zweiten Schritt ein Färbemittel aufträgt, welches eine Verbindung der allgemeinen Formel (I) oder ein Säureadditionssalz davon enthält, wobei das Oxidationsmittel in dem im zweiten Schritt aufgetragenen Mittel enthalten ist, oder auf das Haar selbst in einem dritten Schritt aufgetragen wird, 10 - 40 min, vorzugsweise 15 - 30 min einwirken läßt, anschließend das Haar spült, es mit einem Shampoo wäscht, erneut spült und trocknet.

**21.** Verfahren zur Herstellung einer Verbindung der allgemeinen Formel (I) gemäß Anspruch 1, worin $R_1$ und $R_2$ ein Wasserstoffatom bedeuten und Z und Z' nicht gleichzeitig einen Methylrest bedeuten, dadurch gekennzeichnet, daß man nacheinander oder gleichzeitig eine Reduktion und eine Dehalogenierung von 2,4-disubstiuiertem 3,5-Dinitrochlorbenzol der allgemeinen Formel (II)

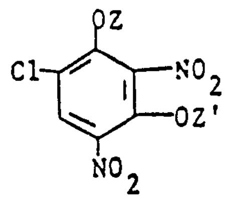

(II)

durchführt, worin Z und Z' die in Anspruch 1 angegebenen Bedeutungen besitzen.

**22.** Verfahren nach Anspruch 21, dadurch gekennzeichnet, daß man

a) in einem ersten Schritt die Verbindung (II) mit Eisen in Gegenwart von Essigsäure bei einer Temperatur im Bereich von 50 - 100 °C reduziert, wobei man 2,4-disubstituiertes 3,5-Diaminochlorbenzol der allgemeinen Formel (IIIA)

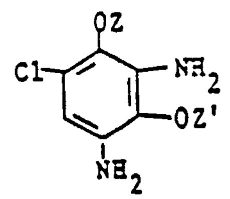

(IIIA)

erhält, worin Z und Z' die in Anspruch 1 angegebenen Bedeutungen besitzen, und anschließend

b) in einem zweiten Schritt die Verbindung (IIIA) in einem Lösungsmittelmilieu, bestehend aus Wasser, einem Niedrigalkohol oder einem Wasser/Alkohol-Gemisch in Gegenwart von Palladium-auf-Kohle, Ammoniumacetat und Triethylaminformiat bei einer Temperatur im Bereich von 50 - 100 °C enthalogeniert.

**23.** Verfahren nach Anspruch 21, dadurch gekennzeichnet, daß man die Verbindung (II) unter Wasserstoffdruck in Gegenwart von Palladium-auf-Kohle in einem Lösungsmittel, ausgewählt unter Wasser, Niedrigalkoholen oder Wasser/Alkohol-Gemischen bei einer Temperatur im Bereich von 50 - 200 °C, vorzugsweise in Gegenwart von Ammoniumacetat und Triethylamin gleichzeitig reduziert und enthalogeniert.

**24.** Verfahren zur Herstellung einer Verbindung der allgemeinen Formel (I) gemäß Anspruch 1, worin $R_1$ und $R_2$ kein Wasserstoffatom bedeuten und die gleichen Bedeutungen besitzen, mit Hilfe eines 2,4-disubstituierten 3,5-Dinitrochlorbenzols, dadurch gekennzeichnet, daß man

a) in einem ersten Schritt das 2,4-disubstituierte 3,5-Dinitrochlorbenzol der allgemeinen Formel (II)

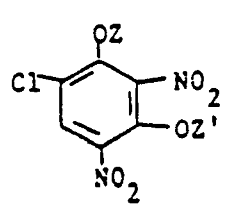

(II)

worin Z und Z' die in Anspruch 1 angegebenen Bedeutungen besitzen aber gleichzeitig einen Methylrest bedeuten können, mit Eisen in Gegenwart von Essigsäure bei einer Temperatur im Bereich von 50 - 100 °C reduziert, wobei man 2,4-disubstituiertes 3,5-Diaminochlorbenzol der allgemeinen Formel (IIIA)

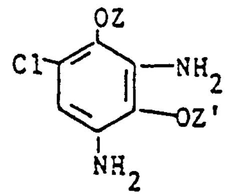

(IIIA)

erhält, worin Z und Z' die oben angegebenen Bedeutungen besitzen;

b) in einem zweiten Schritt die Verbindung der allgemeinen Formel (IIIA) in Gegenwart von Palladium-auf-Kohle, Ammoniumacetat und Triethylaminformiat bei einer Temperatur im Bereich von 50 - 100 °C in einem Lösungsmittelmilieu, bestehend aus Wasser, einem Niedrigalkohol oder einem Wasser/Alkohol-Gemisch enthalogeniert, wobei man das 1,3-disubstituierte 2,4-Diaminobenzol der Formel (IA)

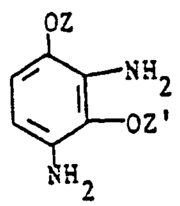

(IA)

erhält, worin Z und Z' die in Anspruch 1 angegebenen Bedeutungen besitzen und gleichzeitig einen Methylrest bedeuten können; und

c) in einem dritten Schritt das 1,3-disubstituierte 2,4-Diaminobenzol der allgemeinen Formel (IA) mit Hilfe von herkömmlichen, bei aromatischen Aminen verwendeten Methoden,alkyliert oder hydroxyalkyliert;

wobei der zweite und dritte Schritt vertauscht sein können.

**25.** Verfahren zur Herstellung einer Verbindung der allgemeinen Formel (I) gemäß Anspruch 1, worin $R_2$ kein Wasserstoffatom bedeutet und $R_1$ gleich H ist, mit Hilfe eines 2,4-disubstituierten 3,5-Dinitrochlorbenzols der allgemeinen Formel (II) gemäß Anspruch 24, dadurch gekennzeichnet, daß man:

a) in einem ersten Schritt eine vorsichtige Reduktion der Verbindung der Formel (II) durch Wasserstoffübertragung in Gegenwart eines Katalysators, wie Palladium-auf-Kohle, und Cyclohexen als Wasserstoffdonor durchführt, wobei man 2,4-disubstituiertes 5-Amino-3-nitrochlorbenzol der allgemeinen Formel (IV)

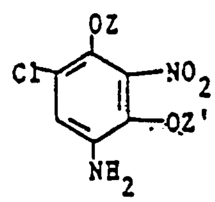

(IV)

erhält, worin Z und Z' die in Anspruch 1 angegebenen Bedeutungen besitzen aber gleichzeitig einen Methylrest bedeuten können;

b) in einem zweiten Schritt die Verbindung der Formel (IV) einer Alkylierung oder einer Hydroxyalkylierung der Aminogruppe unterzieht, wobei man eine Verbindung der allgemeinen Formel (V)

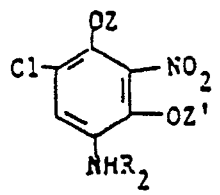

(V)

erhält, worin Z, Z' und $R_2$ die in Anspruch 1 angegebenen Bedeutungen besitzen, wobei Z und Z' gleichzeitig einen Methylrest bedeuten können;

c) in einem dritten Schritt die Verbindung der allgemeinen Formel (V) mit Eisen in Gegenwart von Essigsäure bei einer Temperatur im Bereich von 20 - 100 °C reduziert, wobei man die Verbindung der Formel ($III_B$)

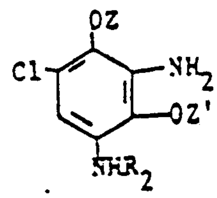

($III_3$)

erhält, worin Z, Z' und $R_2$ die oben angegebenen Bedeutungen besitzen; und

d) in einem vierten Schritt die Verbindung der allgemeinen Formel ($III_B$) mit Hilfe des in Anspruch 24 angegebenen Verfahrens enthalogeniert, wobei man die Verbindung der allgemeinen Formel ($I_B$)

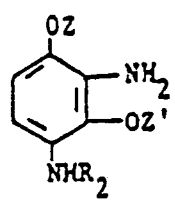

($I_B$)

erhält, worin Z, Z' und $R_2$ die oben angegebenen Bedeutungen besitzen.

**26.** Verfahren zur Herstellung einer Verbindung der allgemeinen Formel (I) gemäß Anspruch 1, worin $R_2$ ein Wasserstoffatom bedeutet und $R_1$ kein Wasserstoffatom bedeutet, mit Hilfe eines 2,4-disubstituierten 3,5-Dinitrochlorbenzols der allgemeinen Formel (II) gemäß Anspruch 24, dadurch gekennzeichnet, daß man:

a) in einem ersten Schritt eine Verbindung der allgemeinen Formel (II) gemäß dem in Anspruch 25 angegebenen Verfahren vorsichtig reduziert, wobei man 2,4-disubstituiertes 5-Amino-3-nitrochlorbenzol der allgemeinen Formel (IV) erhält;

b) in einem zweiten Schritt die Verbindung der Formel (IV) mit Essigsäureanhydrid bei einer Temperatur im Bereich von 20 - 100 °C acetyliert, wobei man eine Verbindung der allgemeinen Formel (VI)

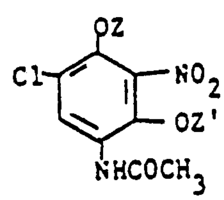

(VI)

erhält, worin Z und Z' die in Anspruch 1 angegebenen Bedeutungen besitzen und gleichzeitig einen Methylrest bedeuten können;

c) in einem dritten Schritt die Verbindung der allgemeinen Formel (VI) unter Wasserstoffdruck bei Verwendung von Palladium-auf-Kohle in Gegenwart von Ammoniumacetat oder Triethylamin in einem Lösungsmittel, bestehend aus Wasser, einem Niedrigalkohol oder einem Wasser/Alkohol-Gemisch bei einer Temperatur im Bereich von 50 - 200 °C gleichzeitig reduziert und enthalogeniert, wobei man 1,3-disubstituiertes 4-Acetamido-2-aminobenzol der allgemeinen Formel (IX) erhält:

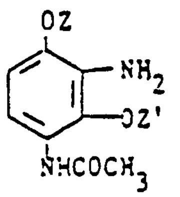

(IX)

d) in einem vierten Schritt die Aminogruppe der Verbindung der allgemeinen Formel (IX) mit Hilfe herkömmlicher Methoden für aromatische Amine alkyliert oder hydroxyalkyliert, wobei man eine Verbindung der allgemeinen Formel (X) erhält:

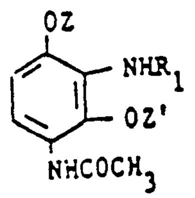

(X)

und

e) in einem fünften Schritt die Verbindung (X) deacetyliert, wobei man das Endprodukt der allgemeinen Formel ($I_C$):

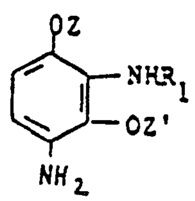

($I_C$)

erhält, worin $R_1$, Z und Z' die in Anspruch 1 angegebenen Bedeutungen besitzen, wobei $R_1$ von Wasserstoff verschieden ist und Z und Z' gleichzeitig einen Methylrest bedeuten können.

**27.** Verfahren zur Herstellung einer Verbindung der allgemeinen Formel ($I_C$) nach Anspruch 26, worin $R_2$ ein Wasserstoffatom bedeutet und $R_1$ von Wasserstoff verschieden ist, dadurch gekennzeichnet, daß der dritte Schritt der gleichzeitigen Reduktion und Enthalogenierung des 2,4-disubstituierten 5-Acetamido-3-nitrochlorbenzols der allgemeinen Formel (VI) ersetzt ist durch eine einfache Reduktion mit Eisen in Gegenwart von Essigsäure bei einer Temperatur im Bereich von 50 - 100 °C, wobei 2,4-disubstituiertes 5-Acetamido-3-aminochlorbenzol der allgemeinen Formel (VII):

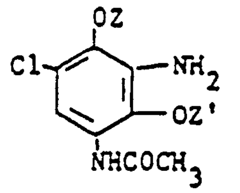

(VII)

gebildet wird, worin Z und Z' die in Anspruch 1 angegebenen Bedeutungen besitzen und gleichzeitig einen Methylrest bedeuten können, wobei dieser Reduktionsschritt gefolgt wird von einer Alkylierung oder Hydroxyalkylierung der Verbindung der allgemeinen Formel (VII), wobei man eine Verbindung der allgemeinen Formel (VIII):

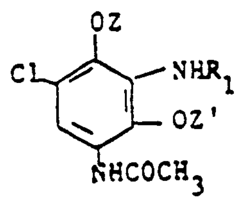

(VIII)

erhält, und anschließend von einer Deacetylierung der Verbindung der allgemeinen Formel (VIII), wobei man eine Verbindung der allgemeinen Formel ($III_C$):

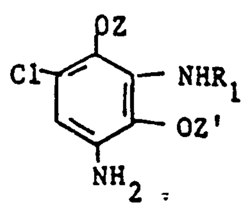

($III_C$)

erhält, die anschließend in Gegenwart von Palladium-auf-Kohle, Ammoniumacetat und Triethylaminformiat in einem Lösungsmittel, bestehend aus Wasser, einem Niedrigalkohol oder einem Wasser/Alkohol-Gemisch, bei einer Temperatur im Bereich von 50 - 100° C enthalogeniert wird, wobei man zu einer Verbindung der allgemeinen Formel ($I_C$) gemäß Anspruch 26 gelangt, worin $R_1$, Z und Z' die in Anspruch 1 angegebenen Bedeutungen besitzen, Z und Z' gleichzeitig einen Methylrest bedeuten können und $R_1$ von einem Wasserstoffatom verschieden ist.

**28.** Verbindung der allgemeinen Formel:

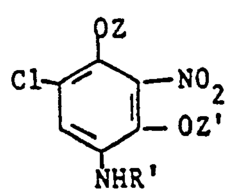

(XI)

worin R' ein Wasserstoffatom, einen Alkylrest mit 1 bis 4 Kohlenstoffatomen, einen Mono- oder Polyhydroxyalkylrest mit 2 oder 3 Kohlenstoffatomen, Acetyl oder ß-Chlorethoxycarbonyl bedeutet und Z und Z' unabhängig voneinander einen Alkylrest mit 1 bis 4 Kohlenstoffatomen oder einen Hydroxyalkylrest mit 2 bis 4 Kohlenstoffatomen bedeuten.

**29.** Verbindung nach Anspruch 28, dadurch gekennzeichnet, daß sie ausgewählt ist unter 5-Amino-2,4-dimethoxy-3-nitrochlorbenzol, 5-Acetamido-2,4-dimethoxy-3-nitrochlorbenzol, 5-Methylamino-2,4-dimethoxy-3-nitrochlorbenzol und 5-(ß-Chlorethoxycarbonyl)-amino-2,4-dimethoxy-3-nitrochlorbenzol.

**Patentantsprüche für folgenden Vertragsstaat: ES**

1. Verwendung als Kuppler einer Verbindung der allgemeinen Formel (I)

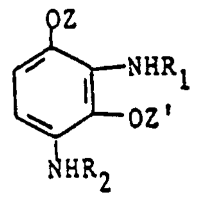

(I)

worin
$R_1$ und $R_2$ jeweils unabhängig vôneinander ein Wasserstoffatom, einen Alkylrest mit 1 bis 4 Kohlenstoffatomen oder einen Mono- oder Polyhydroxyalkylrest mit 2 oder 3 Kohlenstoffatomen bedeuten, und Z und Z' jeweils unabhängig voneinander einen Alkylrest mit 1 bis 4 Kohlenstoffatomen oder einen Hydroxyalkylrest mit 2 bis 4 Kohlenstoffatomen bedeuten, mit der Maßgabe, daß wenn $R_1$ und $R_2$ gleichzeitig ein Wasserstoffatom bedeuten, Z und Z' nicht gleichzeitig einen Methylrest bedeuten,
oder eines Säureadditionssalzes davon in Kombination mit Oxidationsfarbstoff-Prekursoren zur Färbung von Keratinfasern, insbesondere von menschlichem Haar.

2. Haarfärbemittel, dadurch gekennzeichnet, daß es in einem wäßrigen, kosmetisch verträglichen Träger mindestens eine Verbindung der allgemeinen Formel (I) gemäß Anspruch 1 oder eines der Säuresalze davon als Kuppler in Kombination mit mindestens einem Oxidationsfarbstoff-Prekursor vom Paratyp enthält.

3. Färbemittel nach Anspruch 2, dadurch gekennzeichnet, daß es als Kuppler mindestens eine Verbindung enthält, ausgewählt unter
4-(ß-Hydroxyethyl)-amino-2-amino-1,3-dimethoxybenzol,
4-Amino-2-(ß-hydroxyethyl)-amino-1,3-dimethoxybenzol,
4-Methylamino-2-amino-1,3-dimethoxybenzol,
2,4-Di-(ß-hydroxyethyl)-amino-1,3-dimethoxybenzol,
2,4-Diamino-1,3-diethoxybenzol,
2,4-Diamino-1,3-di-(γ-hydroxypropoxy)-benzol
und den Säureadditionssalzen davon.

4. Färbemittel nach Anspruch 2 oder 3, dadurch gekennzeichnet, daß es von der Verbindung (I) oder einem Säureadditionssalz davon 0,05 - 3,5 Gew.-%, bezogen auf das Gesamtgewicht des Mittels, enthält.

5. Färbemittel nach Anspruch 2, dadurch gekennzeichnet, daß der Oxidationsfarbstoff-Prekursor vom Paratyp ausgewählt ist unter p-Phenylendiaminen, p-Aminophenolen, heterocyclischen Paraverbindungen oder den Gemischen davon.

6. Färbemittel nach Anspruch 5, dadurch gekennzeichnet, daß die p-Phenylendiamine folgende allgemeine Formel besitzen:

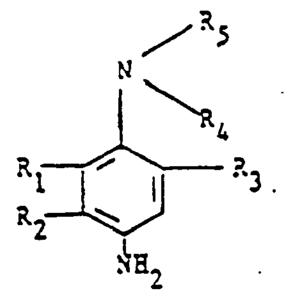

(XII)

worin $R_1$, $R_2$ und $R_3$ gleich oder verschieden sind und ein Wasserstoff- oder Halogenatom, einen Alkylrest mit 1 bis 4 Kohlenstoffatomen oder einen Alkoxyrest mit 1 bis 4 Kohlenstoffatomen bedeuten, $R_4$ und $R_5$ gleich oder verschieden sind und ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbethoxyaminoalkyl-, Piperidinoalkyl-oder Morpholinoalkylrest bedeuten, wobei die Alkyl-oder Alkoxygruppen bedeutenden Reste $R_4$ und $R_5$ 1 bis 4 Kohlenstoffatome besitzen, oder
$R_4$ und $R_5$ zusammen mit dem Stickstoffatom, an dem sie gebunden sind, einen Piperidino- oder Morpholino-heterozyklus bilden, mit der Maßgabe, daß $R_1$ oder $R_3$ ein Wasserstoffatom bedeutet, wenn $R_4$ und $R_5$ nicht für ein Wasserstoffatom stehen,
oder die Salze der Verbindungen gemäß allgemeiner Formel (XII).

7. Färbemittel nach Anspruch 6, dadurch gekennzeichnet, daß es mindestens ein p-Phenylendiamin enthält, ausgewählt unter p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di-(ß-hydroxyethyl)-p-phenylendiamin, 3-Methyl-4-amino-N,N-di-(ß-hydroxyethyl)-anilin, 3-Chlor-4-amino-N,N-di-(ß-hydroxyethyl)-anilin, 4-Amino-N,N-(ethyl, carbamylmethyl)-anilin, 3-Methyl-4-amino-N,N-(ethyl, carbamylmethyl)-anilin, 4-Amino-N,N-(ethyl, ß-piperidinoethyl)-anilin, 3-Methyl-4-amino-N,N-(ethyl, ß-piperidinoethyl)-anilin, 4-Amino-N,N-(ethyl, ß-morpholinoethyl)-anilin, 3-Methyl-4-amino-N,N-(ethyl, ß-morpholinoethyl)-anilin, 4-Amino-N,N-(ethyl, ß-acetylaminoethyl)-anilin, 4-Amino-N, ß-methoxyethylanilin, 3-Methyl-4-amino-N,N-(ethyl, ß-acetylaminoethyl)-anilin, 4-Amino-N,N-(ethyl, ß-mesylaminoethyl)-anilin, 3-Methyl-4-amino-N,N-(ethyl, ß-mesylaminoethyl)-anilin, 4-Amino-N,N-(ethyl, ß-sulfoethyl)-anilin, 3-Methyl-4-amino-N,N-(ethyl, ß-sulfoethyl)-anilin, N-[(4'-Amino)-phenyl]-morpholin, N-[(4'-Amino)-phenyl]-piperidin, 2,3-Dimethyl-p-phenylendiamin und Isopropyl-p-phenylendiamin in Form der freien Base oder als kosmetisch verträgliches Salz.

8. Färbemittel nach Anspruch 5, dadurch gekennzeichnet, daß es mindestens ein p-Aminophenol enthält, ausgewählt unter p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(ß-Hydroxyethyl)-4-aminophenol, 2-Methoxy-4-aminophenol und 3-Methoxy-4-aminophenol.

9. Färbemittel nach Anspruch 5, dadurch gekennzeichnet, daß der Oxidationsfarbstoff-Prekursor vom Paratyp eine heterocyclische Paraverbindung ist, ausgewählt unter 2,5-Diaminopyridin, 2-Hydroxy-5-aminopyridin und Tetraaminopyrimidin.

10. Färbemittel nach einem der Ansprüche 2 bis 9, dadurch gekennzeichnet, daß es weitere Kuppler enthält, ausgewählt unter Metadiphenolen, Metaaminophenolen, Metaphenylendiaminen, Metaacylaminophenolen, Metaureidophenolen, Metacarbalkoxyaminophenolen, α-Naphthol, ß-Ketoverbindungen und Pyrazolonen.

11. Färbemittel nach einem der Ansprüche 2 bis 10, dadurch gekennzeichnet, daß es 0,1 bis 7 Gew.-% eines oder mehrerer Oxidationsfarbstoff-Prekursoren vom Paratyp und Kuppler, bezogen auf das Gesamtgewicht des Mittels, enthält.

12. Färbemittel nach einem der Ansprüche 2 bis 11, dadurch gekennzeichnet, daß es außerdem Farbstoff-Prekursoren vom Ortho-Typ enthält, ausgewählt unter o-Aminophenolen, o-Phenylendiaminen und o-Diphenolen.

13. Färbemittel nach einem der Ansprüche 2 bis 12, dadurch gekennzeichnet, daß es außerdem Direktfarbstoffe enthält, ausgewählt unter Azo-,Anthrachinonfarbstoffen und Nitroderivaten der Benzolreihe.

14. Färbemittel nach einem der Ansprüche 2 bis 13, dadurch gekennzeichnet, daß es einen pH-Wert von 8 - 11 und vorzugsweise von 9 - 11 besitzt.

15. Färbemittel nach einem der Ansprüche 2 bis 14, dadurch gekennzeichnet, daß es 1 - 40 Gew.-% eines organischen Lösungsmittels enthält, ausgewählt unter Niedrigalkanolen, Glycerin, Glykolen oder Glykolethern und Gemischen davon.

16. Färbemittel nach einem der Ansprüche 2 bis 15, dadurch gekennzeichnet, daß es außerdem 0,5 - 40 Gew.-% mindestens eines oberflächenaktiven, anionischen, kationischen, nicht-ionischen, amphoteren Mittels oder ein Gemisch davon enthält.

17. Färbemittel nach einem der Ansprüche 2 bis 16, dadurch gekennzeichnet, daß es außerdem kosmetische Adjuvantien enthält, ausgewählt unter Verdickungsmitteln, Antioxidantien, Penetrationsmitteln, Sequestriermitteln, Puffern, Parfüms, Alkalisierungsmitteln und Treibmitteln.

18. Verfahren zur Haarfärbung unter Entwicklung mit einem Oxidans, dadurch gekennzeichnet, daß man im Augenblick der Anwendung das Färbemittel nach einem der Ansprüche 2 bis 17 mit einer Oxidationslösung in ausreichender Menge mischt, anschließend das erhaltene Gemisch auf das Haar aufträgt, es dort 10 - 40 min, vorzugsweise 15 - 30 min beläßt, anschließend das Haar spült, es mit einem Shampoo wäscht, mit Wasser spült und trocknet.

19. Verfahren zur Haarfärbung unter Entwicklung mit einem Oxidans, dadurch gekennzeichnet, daß man zuerst auf das Haar ein Färbemittel aufträgt, welches mindestens einen Oxidationsfarbstoff-Prekursor vom Paratyp entsprechend der Definition gemäß einem der Ansprüche 5 bis 9 aufträgt, dann in einem zweiten Schritt ein Färbemittel aufträgt, welches eine Verbindung der allgemeinen Formel (I) oder ein Säureadditionssalz davon enthält, wobei das Oxidationsmittel in dem im zweiten Schritt aufgetragenen Mittel enthalten ist, oder auf das Haar selbst in einem dritten Schritt aufgetragen wird, 10 - 40 min, vorzugsweise 15 - 30 min einwirken läßt, anschließend das Haar spült, es mit einem Shampoo wäscht, erneut spült und trocknet.

20. Verfahren zur Herstellung einer Verbindung der allgemeinen Formel (I) gemäß Anspruch 1, worin $R_1$ und $R_2$ ein Wasserstoffatom bedeuten und Z und Z' nicht gleichzeitig einen Methylrest bedeuten, dadurch gekennzeichnet, daß man nacheinander oder gleichzeitig eine Reduktion und eine Dehalogenierung von 2,4-disubstiuierten 3,5-Dinitrochlorbenzol der allgemeinen Formel (II)

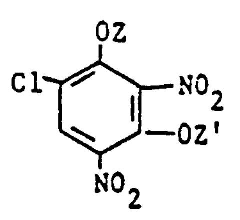

(II)

durchführt, worin Z und Z' die in Anspruch 1 angegebenen Bedeutungen besitzen.

21. Verfahren nach Anspruch 20, dadurch gekennzeichnet, daß man
a) in einem ersten Schritt die Verbindung (II) mit Eisen in Gegenwart von Essigsäure bei einer Temperatur im Bereich von 50 - 100°C reduziert, wobei man 2,4-disubstituiertes 3,5-Diaminochlorbenzol der allgemeinen Formel (IIIA)

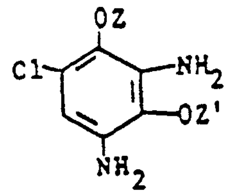

(IIIA)

erhält, worin Z und Z' die in Anspruch 1 angegebenen Bedeutungen besitzen, und anschließend
b) in einem zweiten Schritt die Verbindung (IIIA) in einem Lösungsmittelmilieu, bestehend aus Wasser, einem Niedrigalkohol oder einem Wasser/Alkohol-Gemisch in Gegenwart von Palladium-auf-Kohle, Ammoniumacetat und Triethylaminformiat bei einer Temperatur im Bereich von 50 - 100 °C enthalogeniert.

**22.** Verfahren nach Anspruch 20 , dadurch gekennzeichnet, daß man die Verbindung (II) unter Wasserstoffdruck in Gegenwart von Palladium-auf-Kohle in einem Lösungsmittel, ausgewählt unter Wasser, Niedrigalkoholen oder Wasser/Alkohol-Gemischen bei einer Temperatur im Bereich von 50 - 200 °C, vorzugsweise in Gegenwart von Ammoniumacetat und Triethylamin gleichzeitig reduziert und enthalogeniert.

**23.** Verfahren zur Herstellung einer Verbindung der allgemeinen Formel (I) gemäß Anspruch 1, worin $R_1$ und $R_2$ kein Wasserstoffatom bedeuten und die gleichen Bedeutungen besitzen, mit Hilf eines 2,4-disubstituierten 3,5-Dinitrochlorbenzols, dadurch gekennzeichnet, daß man
a) in einem ersten Schritt das 2,4-disubstituierte 3,5-Dinitrochlorbenzol der allgemeinen Formel (II)

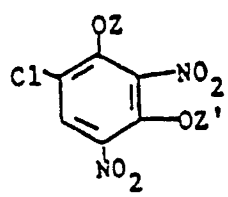

(II)

worin Z und Z' die in Anspruch 1 angegebenen Bedeutungen besitzen aber gleichzeitig einen Methylrest bedeuten können, mit Eisen in Gegenwart von Essigsäure bei einer Temperatur im Bereich von 50 - 100 °C reduziert, wobei man 2,4-disubstituiertes 3,5-Diaminochlorbenzol der allgemeinen Formel (IIIA)

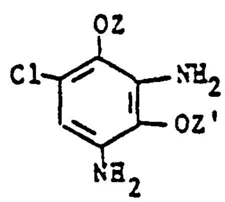

(IIIA)

erhält, worin Z und Z' die oben angegebenen Bedeutungen besitzen;
b) in einem zweiten Schritt die Verbindung der allgemeinen Formel (IIIA) in Gegenwart von Palladium-auf-Kohle, Ammoniumacetat und Triethylaminformiat bei einer Temperatur im Bereich von 50 - 100 °C in einem Lösungsmittelmilieu, bestehend aus Wasser, einem Niedrigalkohol oder einem Wasser/Alkohol-Gemisch enthalogeniert, wobei man das 1,3-disubstituierte 2,4-Diaminobenzol der: Formel (IA)

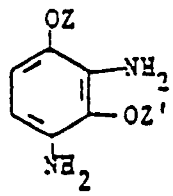

(IA)

erhält, worin Z und Z' die in Anspruch 1 angegebenen Bedeutungen besitzen und gleichzeitig einen Methylrest bedeuten können; und

c) in einem dritten Schritt das 1,3-disubstituierte 2,4-Diaminobenzol der allgemeinen Formel (IA) mit Hilfe von herkömmlichen, bei aromatischen Aminen verwendeten Methoden alkyliert oder hydroxyalkyliert;

wobei der zweite und dritte Schritt vertauscht sein können.

**24.** Verfahren zur Herstellung einer Verbindung der allgemeinen Formel (I) gemäß Anspruch 1, worin $R_1$ kein Wasserstoffatom bedeutet und $R_1$ gleich H ist, mit Hilfe eines 2,4-disubstituierten 3,5-Dinitrochlorbenzols der allgemeinen Formel (II) gemäß Anspruch 23, dadurch gekennzeichnet, daß man:

a) in einem ersten Schritt eine vorsichtige Reduktion der Verbindung der Formel (II) durch Wasserstoffübertragung in Gegenwart eines Katalysators, wie Palladium-auf-Kohle, und Cyclohexen als Wasserstoffdonor durchführt, wobei man 2,4-disubstituiertes 5-Amino-3-nitrochlorbenzol der allgemeinen Formel (IV)

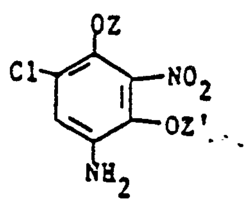

(IV)

erhält, worin Z und Z' die in Anspruch 1 angegebenen Bedeutungen besitzen aber gleichzeitig einen Methylrest bedeuten können;

b) in einem zweiten Schritt die Verbindung der Formel (IV) einer Alkylierung oder einer Hydroxyalkylierung der Aminogruppe unterzieht, wobei man eine Verbindung der allgemeinen Formel (V)

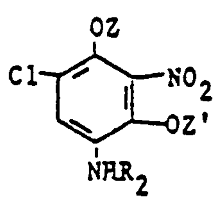

(V)

erhält, worin Z, Z' und $R_2$ die in Anspruch 1 angegebenen Bedeutungen besitzen, wobei Z und Z' gleichzeitig einen Methylrest bedeuten können;

c) in einem dritten Schritt die Verbindung der allgemeinen Formel (V) mit Eisen in Gegenwart von Essigsäure bei einer Temperatur im Bereich von 20 - 100 °C reduziert, wobei man die Verbindung der Formel ($III_B$)

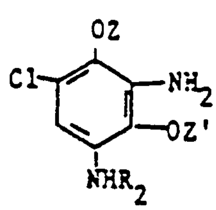

($III_3$)

erhält, worin Z, Z' und $R_2$ die oben angegebenen Bedeutungen besitzen; und

d) in einem vierten Schritt die Verbindung der allgemeinen Formel ($III_B$) mit Hilfe des in Anspruch 24 angegebenen Verfahrens enthalogeniert, wobei man die Verbindung der allgemeinen Formel ($I_B$)

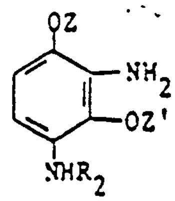

($I_B$)

erhält, worin Z, Z' und $R_2$ die oben angegebenen Bedeutungen besitzen.

**25.** Verfahren zur Herstellung einer Verbindung der allgemeinen Formel (I) gemäß Anspruch 1, worin $R_2$ ein Wasserstoffatom bedeutet und $R_1$ kein Wasserstoffatom bedeutet, mit Hilfe eines 2,4-disubstituierten 3,5-Dinitrochlorbenzols der allgemeinen Formel (II) gemäß Anspruch 23, dadurch gekennzeichnet, daß man:

a) in einem ersten Schritt eine Verbindung der allgemeinen Formel (II) gemäß dem in Anspruch 24 angegebenen Verfahren vorsichtig reduziert, wobei man 2,4-disubstituiertes 5-Amino-3-nitrochlorbenzol der allgemeinen Formel (IV) erhält;

b) in einem zweiten Schritt die Verbindung der Formel (IV) mit Essigsäureanhydrid bei einer Temperatur im Bereich von 20 - 100 °C acetyliert, wobei man eine Verbindung der allgemeinen Formel (VI)

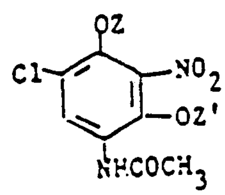

(VI)

erhält, worin Z und Z' die in Anspruch 1 angegebenen Bedeutungen besitzen und gleichzeitig einen Methylrest bedeuten können;

c) in einem dritten Schritt die Verbindung der allgemeinen Formel (VI) unter Wasserstoffdruck bei Verwendung von Palladium-auf-Kohle in Gegenwart von Ammoniumacetat oder Triethylamin in einem Lösungsmittel, bestehend aus Wasser, einem Niedrigalkohol oder einem Wasser/Alkohol-Gemisch bei einer Temperatur im Bereich von 50 - 200 °C gleichzeitig reduziert und enthalogeniert, wobei man 1,3-disubstituiertes 4-Acetamido-2-aminobenzol der allgemeinen Formel (IX) erhält:

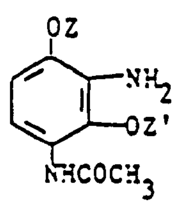

(IX)

d) in einem vierten Schritt die Aminogruppe der Verbindung der allgemeinen Formel (IX) mit Hilfe herkömmlicher Methoden für aromatische Amine alkyliert oder hydroxyalkyliert, wobei man eine Verbindung der allgemeinen Formel (X) erhält:

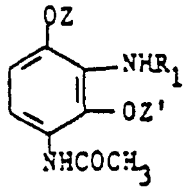 (X)

und

e) in einem fünften Schritt die Verbindung (X) deacetyliert, wobei man das Endprodukt der allgemeinen Formel ($I_C$):

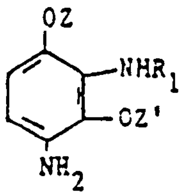 ($I_C$)

erhält, worin $R_1$, Z und Z' die in Anspruch 1 angegebenen Bedeutungen besitzen, wobei $R_1$ von Wasserstoff verschieden ist und Z und Z' gleichzeitig einen Methylrest bedeuten können.

**26.** Verfahren zur Herstellung einer Verbindung der allgemeinen Formel ($I_c$) nach Anspruch 25, worin $R_2$ ein Wasserstoffatom bedeutet und $R_1$ von Wasserstoff verschieden ist, dadurch gekennzeichnet, daß der dritte Schritt der gleichzeitigen Reduktion und Enthalogenierung des 2,4-disubstituierten 5-Acetamido-3-nitrochlorbenzols der allgemeinen Formel (VI) ersetzt ist durch eine einfache Reduktion mit Eisen in Gegenwart von Essigsäure bei einer Temperatur im Bereich von 50 - 100° C, wobei 2,4-disubstituiertes 5-Acetamido-3-aminochlorbenzol der allgemeinen Formel (VII):

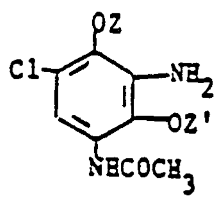 (VII)

gebildet wird, worin Z und Z' die in Anspruch 1 angegebenen Bedeutungen besitzen und gleichzeitig einen Methylrest bedeuten können, wobei dieser Reduktionsschritt gefolgt wird von einer Alkylierung oder Hydroxyalkylierung der Verbindung der allgemeinen Formel (VII), wobei man eine Verbindung der allgemeinen Formel (VIII):

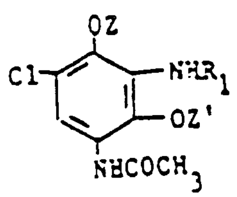 (VIII)

erhält, und anschließend von einer Deacetylierung der Verbindung der allgemeinen Formel (VIII), wobei man eine Verbindung der allgemeinen Formel ($III_C$):

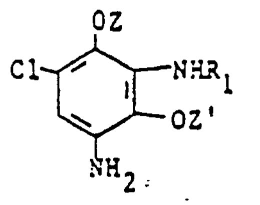

(III$_C$)

erhält, die anschließend in Gegenwart von Palladium-auf-Kohle, Ammoniumacetat und Triethylaminformiat in einem Lösungsmittel, bestehend aus Wasser, einem Niedrigalkohol oder einem Wasser/Alkohol-Gemisch, bei einer Temperatur im Bereich von 50 - 100 °C enthalogeniert wird, wobei man zu einer Verbindung der allgemeinen Formel (I$_C$) gemäß Anspruch 25 gelangt, worin $R_1$, Z und Z' die in Anspruch 1 angegebenen Bedeutungen besitzen, Z und Z' gleichzeitig einen Methylrest bedeuten können und $R_1$ von einem Wasserstoffatom verschieden ist.

**27.** Verfahren zur Herstellung eines Haarfärbemittels, dadurch gekennzeichnet, daß man in einem wäßrigen, kosmetisch verträglichen Träger 0,05 bis 3,5 Gew.-%, bezogen auf das Gesamtgewicht des Mittels, mindestens einer Verbindung der allgemeinen Formel (I):

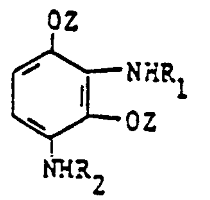

(I)

worin $R_1$ und $R_2$ jeweils unabhängig voneinander ein Wasserstoffatom, einen Alkylrest mit 1 bis 4 Kohlenstoffatomen oder einen Mono- oder Polyhydroxyalkylrest mit 2 oder 3 Kohlenstoffatomen bedeuten, Z und Z' jeweils unabhängig voneinander einen Alkylrest mit 1 bis 4 Kohlenstoffatomen oder einen Hydroxyalkylrest mit 2 bis 4 Kohlenstoffatomen bedeuten, mit der Maßgabe, daß, wenn $R_1$ und $R_2$ gleichzeitig ein Wasserstoffatom bedeuten, Z und Z' nicht gleichzeitig einen Methylrest bedeuten, oder eines Säureadditionssalzes dieser Verbindung, als Kuppler, und mindestens einen Oxidationsfarbstoff-Prekursor vom Paratyp löst, wobei die Gesamtkonzentration an Kupplern und Oxidationsfarbstoff-Prekursoren vom Paratyp im Bereich von 0,1 - 7 Gew.-%, bezogen auf das Gesamtgewicht des Mittels liegt,
anschließend mindestens ein kosmetisches Adjuvans hinzugibt, ausgewählt unter anionischen, kationischen, nichtionischen, amphoteren oberflächenaktiven Mitteln oder Gemischen davon, organischen Lösungsmitteln, Verdickungsmitteln, Antioxidantien, Penetrationsmitteln, Sequestriermitteln, Puffern und Parfüms, und
schließlich den pH des Färbemittels auf einem Wert im Bereich von 8 bis 11 und vorzugsweise im Bereich von 9 bis 11, mit Hilfe eines Alkalisierungsmittels einstellt.

**28.** Verfahren nach Anspruch 27, dadurch gekennzeichnet, daß man als Kuppler der allgemeinen Formel (I) eine Verbindung verwendet, ausgewählt unter
4-(ß-Hydroxyethyl)-amino-2-amino-1,3-dimethoxybenzol,
4-Amino-2-(ß-hydroxyethyl)-amino-1,3-dimethoxybenzol,
4-Methylamino-2-amino-1,3-dimethoxybenzol,
2,4-Di-(ß-hydroxyethyl)-amino-1,3-dimethoxybenzol,
2,4-Diamino-1,3-diethoxybenzol,
2,4-Diamino-1,3-di-(γ-hydroxypropoxy)-benzol
und den Säureadditionssalzen davon.

**29.** Verfahren nach Anspruch 27 oder 28, dadurch gekennzeichnet, daß man als Oxidationsfarbstoff-Prekursor vom Paratyp eine Verbindung verwendet, ausgewählt unter p-Phenylendiaminen, p-Aminophenolen, heterocyclischen Paraverbindungen oder Gemischen davon.

30. Verfahren nach Anspruch 29, dadurch gekennzeichnet, daß man mindestens ein p-Phenylendiamin verwendet, gemäß der allgemeinen Formel:

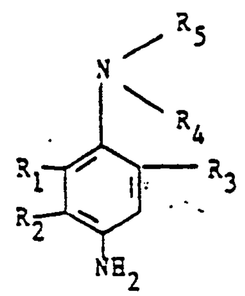

(XII)

worin $R_1$, $R_2$ und $R_3$ gleich oder verschieden sind und ein Wasserstoff- oder Halogenatom, einen Alkylrest mit 1 bis 4 Kohlenstoffatomen oder einen Alkoxyrest mit 1 bis 4 Kohlenstoffatomen bedeuten, $R_4$ und $R_5$ gleich oder verschieden sind und ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbethoxyaminoalkyl-, Piperidinoalkyloder Morpholinoalkylrest bedeuten, wobei die Alkyl-oder Alkoxygruppen bedeutenden Reste $R_4$ und $R_5$ 1 bis 4 Kohlenstoffatome besitzen, oder
$R_4$ und $R_5$ zusammen mit dem Stickstoffatom, an dem sie gebunden sind, einen Piperidino- oder Morpholino-heterozyklus bilden, mit der Maßgabe, daß $R_1$ oder $R_3$ ein Wasserstoffatom bedeutet, wenn $R_4$ und $R_5$ nicht für ein Wasserstoffatom stehen,
oder ein Salz der Verbindungen gemäß allgemeiner Formel (XII).

31. Verfahren nach Anspruch 30, dadurch gekennzeichnet, daß man mindestens ein p-Phenylendiamin verwendet, ausgewählt unter p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di-(ß-hydroxyethyl)-p-phenylendiamin, 3-Methyl-4-amino-N,N-di-(ß-hydroxyethyl)-anilin, 3-Chlor-4-amino-N,N-di-(ß-hydroxy-ethyl)-anilin, 4-Amino-N,N-(ethyl, carbamylmethyl)-anilin, 3-Methyl-4-amino-N,N-(ethyl, carbamylmethyl)-anilin, 4-Amino-N,N-(ethyl, ß-piperidinoethyl)-anilin, 3-Methyl-4-amino-N,N-(ethyl, ß-piperidinoethyl)-anilin, 4-Amino-N,N-(ethyl, ß-morpholinoethyl)-anilin, 3-Methyl-4-amino-N,N-(ethyl, ß-morpholinoethyl)-anilin, 4-Amino-N,N-(ethyl, ß-acetylaminoethyl)-anilin, 4-Amino-N-ß-methoxyethylanilin, 3-Methyl-4-amino-N,N-(ethyl, ß-acetylaminoethyl)-anilin, 4-Amino-N,N-(ethyl, ß-mesylaminoethyl)-anilin, 3-Methyl-4-amino-N,N-(ethyl, ß-mesylaminoethyl)-anilin, 4-Amino-N,N-(ethyl, ß-sulfoethyl)-anilin, 3-Methyl-4-amino-N,N-(ethyl, ß-sulfoethyl)-anilin, N-[(4'-Amino)-phenyl]-morpholin, N-[(4'-Amino)-phenyl]-piperidin,2,3-Dimethyl-p-phenylendiamin und Isopropyl-p-phenylendiamin in Form der freien Base oder als kosmetisch verträgliches Salz.

32. Verfahren nach Anspruch 29, dadurch gekennzeichnet, daß man mindestens ein p-Aminophenol verwendet, ausgewählt unter p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2,5-Dimethyl-4-aminophenol, 2-Hydroxymethyl-4-aminophenol, 2-(ß-Hydroxyethyl)-4-aminophenol, 2-Methoxy-4-aminophenol und 3-Methoxy-4-aminophenol,

33. Verfahren nach Anspruch 29, dadurch gekennzeichnet, daß man eine heterocyclische Paraverbindung verwendet, ausgewählt unter 2,5-Diaminopyridin, 2-Hydroxy-5-aminopyridin und Tetraaminopyrimidin.

34. Verfahren nach einem der Ansprüche 27 bis 33, dadurch gekennzeichnet, daß man weitere Kuppler hinzugibt, ausgewählt unter Metadiphenolen, Metaaminophenolen, Metaphenylendiaminen, Metaacylaminophenolen, Metaureidophenolen, Metacarbalkoxyaminophenolen, α-Naphthol, ß-Ketoverbindungen und Pyrazolonen.

35. Verfahren nach einem der Ansprüche 27 bis 34, dadurch gekennzeichnet, daß man ebenfalls Farbstoff-Prekursoren vom Ortho-Typ hinzugibt, ausgewählt unter o-Aminophenolen, o-Phenylendiaminen und o-Diphenolen.

36. Verfahren nach einem der Ansprüche 27 bis 35, dadurch gekennzeichnet, daß man außerdem Direktfarbstoffe hinzugibt, ausgewählt unter Azofarbstoffen, Anthrachinonfarbstoffen und Nitroderivaten der Benzolreihe.

37. Verfahren nach einem der Ansprüche 27 bis 36, dadurch gekennzeichnet, daß man 1 bis 40 Gew.-% eines organischen Lösungsmittels hinzugibt, ausgewählt unter Niedrigalkanolen, Glycerin, Glykolen oder Glykolethern und Gemischen davon.

38. Verfahren nach einem der Ansprüche 27 bis 36, dadurch gekennzeichnet, daß man 0,5 bis 40 Gew.-% mindestens eines anionischen, kationischen, nicht-ionischen oder amphoteren oberflächenaktiven Mittels oder Gemische davon hinzugibt.